(19) Europäisches Patentamt
European Patent Office
Office européen des brevets

(11) EP 1 330 233 B1

(12) **EUROPEAN PATENT SPECIFICATION**

(45) Date of publication and mention of the grant of the patent:
**02.02.2005 Bulletin 2005/05**

(21) Application number: **01979231.6**

(22) Date of filing: **28.09.2001**

(51) Int Cl.[7]: **A61K 7/42**, C07C 311/03, C07C 311/04

(86) International application number:
**PCT/US2001/027738**

(87) International publication number:
**WO 2002/032389 (25.04.2002 Gazette 2002/17)**

(54) **METHOD OF TREATING STROKE**

METHODE ZUR BEHANDLUNG VON SCHLAGANFALL

PROCEDE RELATIF AU TRAITEMENT DE L'ACCIDENT VASCULAIRE CEREBRAL

(84) Designated Contracting States:
**AT BE CH CY DE DK ES FI FR GB GR IE IT LI LU MC NL PT SE TR**

(30) Priority: **13.10.2000 US 240422 P**

(43) Date of publication of application:
**30.07.2003 Bulletin 2003/31**

(73) Proprietor: **ELI LILLY AND COMPANY**
**Indianapolis, Indiana 46285 (US)**

(72) Inventors:
- **CLEMENS, James, Allen**
**Indianapolis, IN 46220 (US)**
- **LODGE, David, Eli Lilly and Company Limited**
**Basingstoke Hants, Hampshire RG21 2XA (GB)**

(74) Representative: **Kingsbury, Oliver William**
**Eli Lilly and Company Limited,**
**Lilly Research Centre, Erl Wood Manor,**
**Sunninghill Road**
**Windlesham, Surrey GU20 6PH (GB)**

(56) References cited:
**WO-A-00/06157**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

Approximately 600,000 people in the United States suffer from stroke each year. At present, stroke is the No. 3 killer of people in the United States with approximately 160,000 victims dying each year. A stroke, also referred to as a "brain attack", is a form of cerebrovascular disease that occurs when the blood supply to the brain is disrupted and brain cells are starved of oxygen resulting in cell death in the immediate area. This area of dead cells is referred to as an infarct. As brain cells die, they release chemical messengers setting off a chain reaction called secondary injury which can also harm or kill additional brain cells in the area surrounding the infarct. When brain cells are killed or damaged, the functions and parts of the body controlled by these cells are affected in varying degrees depending upon the severity of the stroke. This can result, for example, in difficulty with speech, varying degrees of paralysis, immobility, or loss of memory.

There are two main categories of stroke, ischemic and hemorrhagic. Most strokes are ischemic which means they are caused by a lack of blood supply to part of the brain which can be caused by atherosclerosis. In addition, a blood clot can form and block blood flow to the brain resulting in a thrombotic stroke.

A hemorrhagic stroke occurs when an artery in the brain leaks or ruptures. Blood puts pressure on the surrounding brain tissue, causing damage. In addition, brain cells beyond the rupture site are deprived of their blood supply and are also damaged.

Certain sulfonamide derivatives which potentiate glutamate receptor function, such as AMPA receptors, have been disclosed in the following International Patent Application Publications: WO 98/33496 published August 6, 1998; WO 99/43285 published September 2, 1999; WO 00/06539; WO 00/06537, WO 00/06176, WO 00/06159, WO 00/06158, WO 00/06157, WO 00/06156, WO 00/06149, WO 00/06148, and WO 00/06083, all published February 10, 2000, WO 00/66546, published November 9, 2000, and WO 01/42203, published June 14, 2001.

SUMMARY OF THE INVENTION

The present invention provides the use, for the manufacture of a medicament for treating stroke, of a compound of formula I:

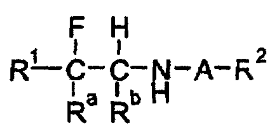

Formula I

wherein:

A represents $SO_2$, $CO_2$, or CONH;
$R^a$ represents (1-6C)alkyl, (2-6C)alkenyl, -(1-4C)alkyl(3-8C)cycloalkyl, or -(1-4C)alkylaromatic;
$R^b$ represents H, (1-6C)alkyl, (2-6C)alkenyl, -(1-4C)alkyl(3-8C)cycloalkyl, or -(1-4C)alkylaromatic; or
$R^a$ and $R^b$ together with the carbon atoms to which they are attached form a (3-8C) saturated carbocyclic ring, a (3-8C) saturated carbocyclic ring containing a heteroatom selected from the group consisting of sulfur or oxygen, or a (5-8C) carbocyclic ring containing one double bond;
$R^1$ represents an unsubstituted or substituted aromatic group, an unsubstituted or substituted heteroaromatic group, or an unsubstituted or substituted (5-8C)cycloalkyl group;
$R^2$ represents (1-6C)alkyl, (3-6C)cycloalkyl, (1-6C)fluoroalkyl, (1-6C)chloroalkyl, (2-6C)alkenyl, (1-4C)alkoxy (1-4C)alkyl, phenyl which is unsubstituted or substituted by halogen, (1-4C)alkyl or (1-4C)alkoxy, or when A represents $SO_2$, a group of formula $R^3R^4N$ in which $R^3$ and $R^4$ each independently represents (1-4C)alkyl or, together with the nitrogen atom to which they are attached form an azetidinyl, pyrrolidinyl, piperidinyl, morpholino, piperazinyl, hexahydroazepinyl or octahydroazocinyl group;

or a pharmaceutically acceptable salt thereof.

The invention further provides the use for the manufacture of a medicament for enhancing recovery following stroke in a patient of a compound of formula I.

The present invention provides the use of a compound of formula I, or a pharmaceutically acceptable salt thereof for the manufacture of a medicament for treating traumatic brain injury, traumatic spinal cord injury, brain damage resulting from cerebral hypoperfusion, or other neurodegenerative conditions.

DETAILED DESCRIPTION OF THE INVENTION

As used herein the term "potentiating glutamate receptor function" refers to any increased responsiveness of glutamate receptors, for example AMPA receptors, to glutamate or an agonist, and includes but is not limited to inhibition of rapid desensitization or deactivation of AMPA receptors to glutamate.

As used herein the term "cerebral hypoperfusion" refers to atherosclerosis of cerebral vessels.

It is understood that compounds of the formulas Ia', Ib', Ic', and In';

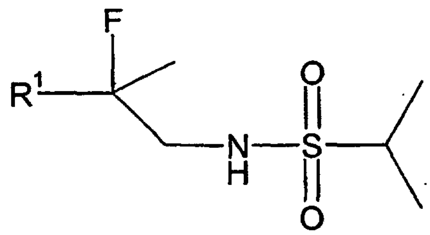

formula Ia'

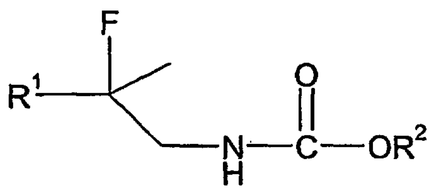

formula Ib'

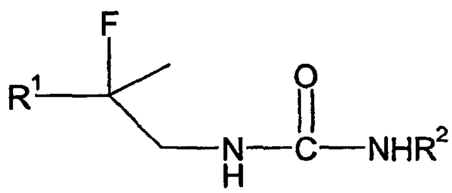

formula Ic'

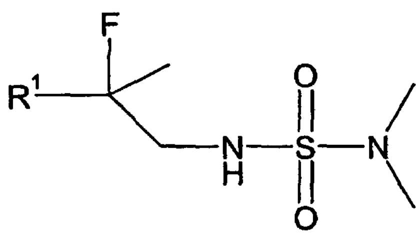

formula In'

wherein $R^1$ represents an unsubstituted or substituted aromatic group, an unsubstituted or substituted heteroaromatic group, or an unsubstituted or substituted (5-8C)cycloalkyl group; or a pharmaceutically acceptable salt thereof; are included within the scope of formula I.

The present invention includes the pharmaceutically acceptable salts of the compounds defined by formula I. A compound of this invention can possess a sufficiently acidic group, a sufficiently basic group, or both functional groups, and accordingly react with any of a number of organic and inorganic bases, and inorganic and organic acids, to form a pharmaceutically acceptable salt.

The term "pharmaceutically acceptable salt" as used herein, refers to salts of the compounds of the above formula which are substantially non-toxic to living organisms. Typical pharmaceutically acceptable salts include those salts prepared by reaction of the compounds of the present invention with a pharmaceutically acceptable mineral or organic acid or an organic or inorganic base. Such salts are known as acid addition and base addition salts. Such salts include the pharmaceutically acceptable salts listed in Journal of Pharmaceutical Science, 66, 2-19 (1977) which are known to the skilled artisan.

Acids commonly employed to form acid addition salts are inorganic acids such as hydrochloric acid, hydrobromic acid,

hydroiodic acid, sulfuric acid, phosphoric acid, and the like, and organic acids such as *p*-toluenesulfonic, methanesulfonic acid, benzenesulfonic acid, oxalic acid, *p*-bromophenylsulfonic acid, carbonic acid, succinic acid, citric acid, benzoic acid, acetic acid, and the like. Examples of such pharmaceutically acceptable salts are the sulfate, pyrosulfate, bisulfate, sulfite, bisulfite, phosphate, monohydrogenphosphate, dihydrogenphosphate, metaphosphate, pyrophosphate, bromide, iodide, acetate, propionate, decanoate, caprate, caprylate, acrylate, ascorbate, formate, hydrochloride, dihydrochloride, isobutyrate, caproate, heptanoate, propiolate, propionate, phenylpropionate, salicylate, oxalate, malonate, succinate, suberate, sebacate, fumarate, malate, maleate, hydroxymaleate, mandelate, nicotinate, isonicotinate, cinnamate, hippurate, nitrate, phthalate, teraphthalate, butyne-1,4-dioate, butyne-1,4-dicarboxylate, hexyne-1,4-dicarboxylate, hexyne-1,6-dioate, benzoate, chlorobenzoate, methylbenzoate, hydroxybenzoate, methoxybenzoate, dinitrobenzoate, o-acetoxybenzoate, naphthalene-2-benzoate, phthalate, *p*-toluenesulfonate, *p*-bromobenzenesulfonate, *p*-chlorobenzenesulfonate, xylenesulfonate, phenylacetate, trifluoroacetate, phenylpropionate, phenylbutyrate, citrate, lactate, α-hydroxybutyrate, glycolate, tartrate, benzenesulfonate, methanesulfonate, ethanesulfonate, propanesulfonate, hydroxyethanesulfonate, 1-naphthalenesulfonate, 2-napththalenesulfonate, 1,5-naphthalenedisulfonate, mandelate, tartarate, and the like. Preferred pharmaceutically acceptable acid addition salts are those formed with mineral acids such as hydrochloric acid and hydrobromic acid, and those formed with organic acids such as maleic acid, oxalic acid and methanesulfonic acid.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, sodium carbonate, sodium bicarbonate, potassium bicarbonate, calcium hydroxide, calcium carbonate, and the like. The potassium and sodium salt forms are particularly preferred.

It should be recognized that the particular counterion forming a part of any salt of this invention is usually not of a critical nature, so long as the salt as a whole is pharmacologically acceptable and as long as the counterion does not contribute undesired qualities to the salt as a whole. It is further understood that the above salts may form hydrates or exist in a substantially anhydrous form.

As used herein, the term "stereoisomer" refers to a compound made up of the same atoms bonded by the same bonds but having different three-dimensional structures which are not interchangeable. The three-dimensional structures are called configurations. As used herein, the term "enantiomer" refers to two stereoisomers whose molecules are nonsuperimposable mirror images of one another. The term "chiral center" refers to a carbon atom to which four different groups are attached. As used herein, the term "diastereomers" refers to stereoisomers which are not enantiomers. In addition, two diastereomers which have a different configuration at only one chiral center are referred to herein as "epimers". The terms "racemate", "racemic mixture" or "racemic modification" refer to a mixture of equal parts of enantiomers.

The term "enantiomeric enrichment" as used herein refers to the increase in the amount of one enantiomer as compared to the other. A convenient method of expressing the enantiomeric enrichment achieved is the concept of enantiomeric excess, or "ee", which is found using the following equation:

$$ee = \frac{E^1 - E^2}{E^1 + E^2} X 100$$

wherein $E^1$ is the amount of the first enantiomer and $E^2$ is the amount of the second enantiomer. Thus, if the initial ratio of the two enantiomers is 50:50, such as is present in a racemic mixture, and an enantiomeric enrichment sufficient to produce a final ratio of 50:30 is achieved, the ee with respect to the first enantiomer is 25%. However, if the final ratio is 90:10, the ee with respect to the first enantiomer is 80%. An ee of greater than 90% is preferred, an ee of greater than 95% is most preferred and an ee of greater than 99% is most especially preferred. Enantiomeric enrichment is readily determined by one of ordinary skill in the art using standard techniques and procedures, such as gas or high performance liquid chromatography with a chiral column. Choice of the appropriate chiral column, eluent and conditions necessary to effect separation of the enantiomeric pair is well within the knowledge of one of ordinary skill in the art. In addition, the specific stereoisomers and enantiomers of compounds of formula I can be prepared by one of ordinary skill in the art utilizing well known techniques and processes, such as those disclosed by J. Jacques, et al., "Enantiomers, Racemates, and Resolutions", John Wiley and Sons, Inc., 1981, and E.L. Eliel and S.H. Wilen," Stereochemistry of Organic Compounds", (Wiley-Interscience 1994), and European Patent Application No. EP-A-838448, published April 29, 1998. Examples of resolutions include recrystallization techniques or chiral chromatography.

Some of the compounds of the present invention have one or more chiral centers and may exist in a variety of stereoisomeric configurations. As a consequence of these chiral centers, the compounds of the present invention occur as racemates, mixtures of enantiomers and as individual enantiomers, as well as diastereomers and mixtures of diastereomers. All such racemates, enantiomers, and diastereomers are within the scope of the present invention.

The terms "R" and "S" are used herein as commonly used in organic chemistry to denote specific configuration of a chiral center. The term "R" (rectus) refers to that configuration of a chiral center with a clockwise relationship of group priorities (highest to second lowest) when viewed along the bond toward the lowest priority group. The term "S" (sinister) refers to that configuration of a chiral center with a counterclockwise relationship of group priorities (highest to second lowest) when viewed along the bond toward the lowest priority group. The priority of groups is based upon their atomic number (in order of decreasing atomic number). A partial list of priorities and a discussion of stereochemistry is contained in "Nomenclature of Organic Compounds: Principles and Practice", (J.H. Fletcher, et al., eds., 1974) at pages 103-120.

As used herein, the term "aromatic group" means the same as aryl, and includes phenyl and a polycyclic aromatic carbocyclic ring such as 1- or 2-naphthyl, 1,2-dihydronaphthyl, 1,2,3,4-tetrahydronaphthyl, and the like.

The term "heteroaromatic group" includes an aromatic 5-6 membered ring containing from one to four heteroatoms selected from oxygen, sulfur and nitrogen, and a bicyclic group consisting of a 5-6 membered ring containing from one to four heteroatoms selected from oxygen, sulfur and nitrogen fused with a benzene ring or another 5-6 membered ring containing one to four atoms selected from oxygen, sulfur and nitrogen. Examples of heteroaromatic groups are thienyl, furyl, oxazolyl, isoxazolyl, oxadiazoyl, pyrazolyl, thiazolyl, thiadiazolyl, isothiazolyl, imidazolyl, triazolyl, tetrazolyl, pyridyl, pyridazinyl, pyrimidyl, benzofuryl, benzothienyl, benzimidazolyl, benzoxazolyl, benzothiazolyl, indolyl, and quinolyl.

The term "substituted" as used in the term "substituted aromatic or heteroaromatic group" herein signifies that one or more (for example one or two) substituents may be present, said substituents being selected from atoms and groups which, when present in the compound of formula I, do not prevent the compound of formula I from functioning as a potentiator of glutamate receptor function.

It is understood that when $R^1$ represents an unsubstituted or substituted (5-8C)cycloalkyl group, mixtures of cis and trans isomers may result which can be separated into the individual cis and trans isomers by one of ordinary skill in the art, using standard techniques and procedures such as reverse phase or normal phase high performance liquid chromatography or flash chromatography, with a suitable stationary phase and a suitable eluent. Examples of suitable stationary phases are silica gel, alumina, and the like. Examples of suitable eluents are ethyl acetate/hexane, ethyl acetate/toluene, methanol/dichloromethane, and the like. Such individual cis and trans isomers are included within the scope of the present invention.

Examples of substituents which may be present in a substituted aromatic; heteroaromatic group or (5-8C) cycloalkyl group include halogen; nitro; cyano; hydroxyimino; (1-10C) alkyl; (2-10C)alkenyl; (2-10C)alkynyl; (3-8C) cycloalkyl; hydroxy(3-8C)cycloalkyl; oxo(3-8C)cycloalkyl; halo(1-10C)alkyl; $(CH_2)_yX^1R^9$ in which y is 0 or an integer of from 1 to 4, $X^1$ represents O, S, $NR^{10}$, CO, COO, OCO, $CONR^{11}$, $NR^{12}CO$, $NR^{12}COCOO$, $OCONR^{13}$, $R^9$ represents hydrogen, (1-10C) alkyl, (3-10C)alkenyl, (3-10C)alkynyl, pyrrolidinyl, tetrahydrofuryl, morpholino or (3-8C)cycloalkyl and $R^{10}$, $R^{11}$, $R^{12}$ and $R^{13}$ each independently represents hydrogen or (1-10C)alkyl, or $R^9$ and $R^{10}$, $R^{11}$, $R^{12}$ or $R^{13}$ together with the nitrogen atom to which they are attached form an azetidinyl, pyrrolidinyl, piperidinyl or morpholino group; N-(1-4C)alkylpiperazinyl; N-phenyl(1-4C)alkylpiperazinyl; thienyl; furyl; oxazolyl; isoxazolyl; pyrazolyl; imidazolyl; thiazolyl; pyridyl; pyridazinyl; pyrimidinyl; dihydrothienyl; dihydrofuryl; dihydrothiopyranyl; dihydropyranyl; dihydrothiazolyl; (1-4C)alkoxycarbonyl dihydrothiazolyl; (1-4C)alkoxycarbonyl dimethyl-dihydrothiazolyl; tetrahydrothienyl; tetrahydrofuryl; tetrahydrothiopyranyl; tetrahydropyranyl; indolyl; benzofuryl; benzothienyl; benzimidazolyl; and a group of formula $R^{14}-(L^a)_n-X^2-(L^b)_m$ in which $X^2$ represents a bond, O, NH, S, SO, $SO_2$, CO, CH(OH), CONH, NHCO, NHCONH, NHCOO, COCONH, $OCH_2CONH$, or CH=CH, $L^a$ and $L^b$ each represent (1-4C)alkylene, one of n and m is 0 or 1 and the other is 0, and $R^{14}$ represents a phenyl or heteroaromatic group which is unsubstituted or substituted by one or two of halogen; nitro; cyano; (1-10C) alkyl; (2-10C)alkenyl; (2-10C)alkynyl; (3-8C)cycloalkyl; 4-(1,1-dioxotetrahydro-1,2-thiazinyl); halo(1-10C)alkyl; cyano(2-10C)alkenyl; phenyl; and $(CH_2)_zX^3R^{15}$ in which z is 0 or an integer of from 1 to 4, $X^3$ represents O, S, $NR^{16}$, CO, CH(OH), COO, OCO, $CONR^{17}$, $NR^{18}CO$, $NHSO_2$, $SO_2NH$, $NHSO_2NR^{17}$, $OCONR^{19}$ or $NR^{19}COO$, $R^{15}$ represents hydrogen, (1-10C)alkyl, phenyl(1-4C)alkyl, halo(1-10C)alkyl, (1-4C)alkoxycarbonyl(1-4C)alkyl, (1-4C)alkylsulfonylamino(1-4C)alkyl, N-(1-4C)alkoxycarbonyl)(1-4C)alkylsulfonylamino(1-4C) alkyl, (3-10C)alkenyl, (3-10C)alkynyl, (3-8C)cycloalkyl, camphoryl, or an aromatic or heteroaromatic group which is unsubstituted or substituted by one or two of halogen, (1-4C)alkyl, halo(1-4C)alkyl, di(1-4C)alkylamino and (1-4C) alkoxy, and $R^{16}$, $R^{17}$, $R^{18}$ and $R^{19}$ each independently represents hydrogen or (1-10C)alkyl, or $R^{15}$ and $R^{16}$, $R^{17}$, $R^{18}$ or $R^{19}$ together with the nitrogen atom to which they are attached form an azetidinyl, pyrrolidinyl, piperidinyl or morpholino group.

The term (1-10C)alkyl includes (1-8C)alkyl, (1-6C)alkyl and (1-4C)alkyl. Particular values are methyl, ethyl, propyl, isopropyl, butyl, isobutyl, t-butyl, pentyl, hexyl, heptyl, octyl, nonyl and decyl.

The term (2-10C)alkenyl includes (3-10C)alkenyl, (2-8C)alkenyl, (2-6C)alkenyl and (2-4C)alkenyl. Particular values are vinyl and prop-2-enyl.

The term (2-10C)alkynyl includes (3-10C)alkynyl, (2-8C)alkynyl, (2-6C)alkynyl and (3-4C)alkynyl. A particular value is prop-2-ynyl.

The term $C_1$-$C_6$ alkoxy refers to a straight or branched alkyl chain having from one to six carbon atoms attached to an oxygen atom. Typical $C_1$-$C_6$ alkoxy groups include methoxy, ethoxy, propoxy, isopropoxy, butoxy, t-butoxy, pentoxy and the like. The term $C_1$-$C_6$ alkoxy includes within its definition the term $C_1$-$C_4$ alkoxy.

The term (3-8C)cycloalkyl, as such or in the term (3-8C)cycloalkyloxy, includes monocyclic and polycyclic groups. Particular values are cyclopropyl, cyclobutyl, cyclopentyl, cyclohexyl and bicydo[2.2.2]octane. The term includes (3-6C)cycloalkyl: cyclopropyl, cyclobutyl, cyclopentyl and cyclohexyl.

As used herein the terms "integer of from 1 to 4" or "integer of from 1 to 3" includes the integers 1, 2, 3, and 4, or the integers 1, 2, and 3, respectively.

The term (5-8C)cycloalkyl includes cyclopentyl, cyclohexyl, cycloheptyl and cyclooctyl.

The term hydroxy(3-8C)cycloalkyl includes hydroxy-cyclopentyl, such as 3-hydroxycyclopentyl.

The term oxo(3-8C)cycloalkyl includes oxocyclopentyl, such as 3-oxocyclopentyl.

The terms "halogen", "Hal" or "halide" include fluorine, chlorine, bromine and iodine unless otherwise specified.

The term halo(1-10C)alkyl includes fluoro(1-10C)alkyl, such as trifluoromethyl and 2,2,2-trifluoroethyl, and chloro(1-10C)alkyl such as chloromethyl.

The term cyano(2-10C)alkenyl includes 2-cyanoethenyl.

The term (2-4C)alkylene includes ethylene, propylene and butylene. A preferred value is ethylene.

The term thienyl includes thien-2-yl and thien-3-yl.

The term furyl includes fur-2-yl and fur-3-yl.

The term oxazolyl includes oxazol-2-yl, oxazol-4-yl and oxazol-5-yl.

The term isoxazolyl includes isoxazol-3-yl, isoxazol-4-yl and isoxazol-5-yl.

The term oxadiazolyl includes [1,2,4]oxadiazol-3-yl and [1,2,4]oxadiazol-5-yl.

The term pyrazolyl includes pyrazol-3-yl, pyrazol-4-yl and pyrazol-5-yl.

The term thiazolyl includes thiazol-2-yl, thiazol-4-yl and thiazol-5-yl.

The term thiadiazolyl includes [1,2,4]thiadiazol-3-yl, and [1,2,4]thiadiazol-5-yl.

The term isothiazolyl includes isothiazol-3-yl, isothiazol-4-yl and isothiazol-5-yl.

The term imidazolyl includes imidazol-2-yl, imidazolyl-4-yl and imidazolyl-5-yl.

The term triazolyl includes [1,2,4]triazol-3-yl and [1,2,4]triazol-5-yl.

The term tetrazolyl includes tetrazol-5-yl.

The term pyridyl includes pyrid-2-yl, pyrid-3-yl and pyrid-4-yl.

The term pyridazinyl includes pyridazin-3-yl, pyridazin-4-yl, pyridazin-5-yl and pyridazin-6-yl.

The term pyrimidyl includes pyrimidin-2-yl, pyrimidin-4-yl, pyrimidin-5-yl and pyrimidin-6-yl.

The term benzofuryl includes benzofur-2-yl and benzofur-3-yl.

The term benzothienyl includes benzothien-2-yl and benzothien-3-yl.

The term benzimidazolyl includes benzimidazol-2-yl.

The term benzoxazolyl includes benzoxazol-2-yl.

The term benzothiazolyl includes benzothiazol-2-yl.

The term indolyl includes indol-2-yl and indol-3-yl.

The term quinolyl includes quinol-2-yl.

The term dihydrothiazolyl includes 4,5-dihydrothiazol-2-yl, and the term (1-4C)alkoxycarbonyldihydrothiazolyl includes 4-methoxycarbonyl-4,5-dihydrothiazol-2-yl.

The term -(1-4C)alkyl(3-8C)cycloalkyl includes the following:

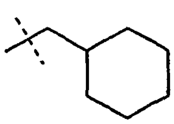 , 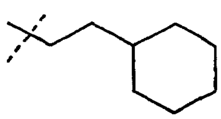 , 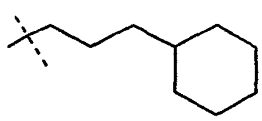 , 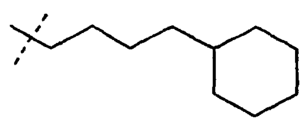

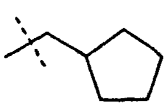 , 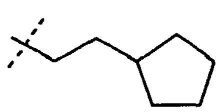 , 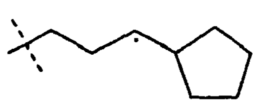 and 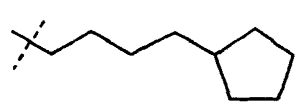 .

The term -(1-4C)alkylaromatic includes the following:

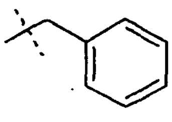 , 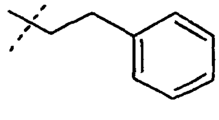 , 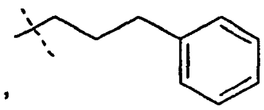 . 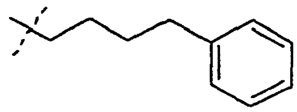 ,

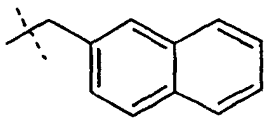 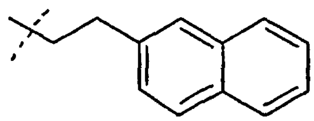 , 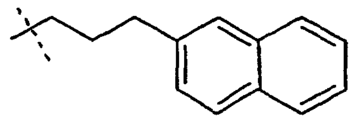

and

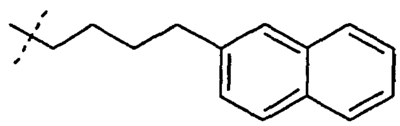

Preferably $R^a$ is methyl, ethyl, propyl, n-butyl, sec-butyl, pentyl, and hexyl with methyl being most preferred.

Preferably $R^b$ is hydrogen, methyl, ethyl, propyl, n-butyl, sec-butyl, pentyl, and hexyl, with hydrogen being most preferred.

Preferably $R^3$ and $R^4$ each represent methyl.

Examples of values for $R^2$ are methyl, ethyl, propyl, 2-propyl, butyl, 2-methylpropyl, cyclohexyl, trifluoromethyl, 2,2,2-trifluoroethyl, chloromethyl, ethenyl, prop-2-enyl, methoxyethyl, phenyl, 4-fluorophenyl, or dimethylamino. Preferably $R^2$ is ethyl, 2-propyl or dimethylamino.

Examples of values for $R^9$ are hydrogen, methyl, ethyl, propyl, isopropyl, t-butyl, ethenyl, cyclopropyl, cyclobutyl, cyclopentyl, cyclohexyl, 2-pyrrolidinyl, morpholino or 2-tetrahydrofuryl.

$R^9$ is preferably (1-4C)alkyl, (2-4C)alkenyl, (3-6C)cycloalkyl, pyrrolidinyl, morpholino or tetrahydrofuryl.

Examples of values for $R^{15}$ are hydrogen, methyl, ethyl, propyl, isopropyl, butyl, t-butyl, benzyl, 2,2,2-trifluoroethyl, 2-methoxycarbonylethyl, cyclohexyl, 10-camphoryl, phenyl, 2-fluorophenyl, 3-fluorophenyl, 2-trifluoromethylphenyl, 4-trifluoromethylphenyl, 2-methoxyphenyl, 3-methoxyphenyl, 4-methoxyphenyl, 1-(5-dimethylamino)naphthyl, and 2-thienyl.

$X^1$ preferably represents O, CO, CONH or NHCO.

z is preferably 0.

Particular values for the groups $(CH_2)_yX^1R^9$ and $(CH_2)_zX^3R^{15}$ include (1-10C)alkoxy, including (1-6C)alkoxy and (1-4C)alkoxy, such as methoxy, ethoxy, propoxy, isopropoxy and isobutoxy; (3-10C)alkenyloxy, including (3-6C) alkenyloxy, such as prop-2-enyloxy; (3-10C)alkynyloxy, including (3-6C)alkynyloxy, such as prop-2-ynyloxy; and (1-6C) alkanoyl, such as formyl and ethanoyl.

Examples of particular values for y are 0 and 1.

Examples of particular values for z are 0, 1, 2 and 3.

$L^a$ and $L^b$ preferably each independently represents $CH_2$.

$X^2$ preferably represents a bond, O, NH, CO, CH(OH), CONH, NHCONH or $OCH_2CONH$, with a bond, O, and CONH being especially preferred.

Preferably the group $(CH_2)_yX^1R^9$ represents CHO; $COCH_3$, $OCH_3$; $OCH(CH_3)_2$; $NHCOR^9$ in which $R^9$ represents methyl, ethyl, isopropyl, t-butyl, ethenyl, cyclopropyl, cyclobutyl, cyclopentyl, cyclohexyl, 2-pyrolidinyl or morpholino; $CONHR^9$ in which $R^9$ represents cyclopropyl or cyclopentyl; NHCOCOOCH3; or 2-tetrahydrofurylmethoxy.

Preferably the group $(CH_2)_zX^3R^{15}$ represents $NH_2$; $CH_2NH_2$; $(CH_2)_2NH_2$; $(CH_2)_3NH_2$; $CONH_2$; $CONHCH_3$; $CON(CH_3)_2$; $N(C_2H_5)_2$; $CH_2OH$; $CH(OH)CH_3$; $CH(OH)CH_2CH_2$; CHO; $COCH_3$; COOH; $COOCH_3$; $CH_2NHCOOC(CH_3)_3$; $(CH_2)_2NHCOOC(CH_3)_3$; $SO_2NH_2$; $NHSO_2CH_3$; $NHSO_2CH(CH_3)_2$; a group of formula $(CH_2)_2NHSO_2R^{15}$ in which $R^{15}$ represents $CH_3$, $CH_2CH_3$, $CH(CH_3)_2$, $(CH_2)_2CH_3$, $(CH_3)_3CH_3$, benzyl, $CH_2CF_3$, 2-methoxycarbonylethyl,

cyclohexyl, 10-camphoryl, phenyl, 2-fluorophenyl, 4-fluorophenyl, 2-trifluoromethylphenyl, 4-trifluoromethylphenyl, 4-methoxyphenyl, 1-(2-dimethylamino)naphthyl or 2-thienyl; $CH(OH)CH_2NHSO_2CH_3$; $(CH_2)_3NHSO_2CH(CH_3)_2$; $COCH_2N(OCOC(CH_3)_2SO_2CH_3$; $COCH_2NHSO_2CH_3$; $(CH_2)_2NHCOR^{15}$ in which $R^{15}$ represents $CH_3$, $CH(CH_3)_2$, $CH_2CH(CH_3)_2$, phenyl, 3-fluorophenyl, 4-fluorophenyl, benzyl, 2-methoxyphenyl, 4-methoxyphenyl, 2-thienyl, CH=CH, CH=CHCN, $OCH_3$ or $O(CH_2)_3CH_3$.

Examples of particular values for $(L^a)_n$-$X^2$-$(L^b)_m$ are a bond, O, NH, S, SO, $SO_2$, CO, $CH_2$, $COCH_2$, COCONH, $CH(OH)CH_2$, CONH, NHCO, NHCONH, $CH_2O$, $OCH_2$, $OCH_2CONH$, $CH_2NH$, $NHCH_2$ and $CH_2CH_2$, with a bond, CONH, and $CH_2O$ being especially preferred.

$R^{14}$ is preferably an unsubstituted or substituted phenyl, naphthyl, furyl, thienyl, isoxazolyl, thiazolyl, tetrazolyl, pyridyl, pyrimidyl benzothienyl or benzothiazolyl group.

Examples of particular values for $R^{14}$ are phenyl, 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, 4-iodophenyl, 2,3-difluorophenyl, 2,4-difluorophenyl, 3,5-difluorophenyl, 3,4-dichlorophenyl, 3,5-dichlorophenyl, 4-cyanophenyl, 3-nitrophenyl, 4-hydroxyiminophenyl, 2-methylphenyl, 4-methylphenyl, 4-ethylphenyl, 3-propylphenyl, 4-t-butylphenyl, 2-prop-2-enylphenyl, 4-(4-(1,1-dioxotetrahydro-1,2-thiazinyl)phenyl, 2-trifluoromethylphenyl, 3-trifluoromethylphenyl, 4-trifluoromethylphenyl, 2-bromomethylphenyl, 2-fluoro-4-trifluoromethylphenyl, 4-(2-cyanoethenyl)phenyl, 4-phenyl, 2-formylphenyl, 3-formylphenyl, 4-formylphenyl, 2-acetylphenyl, 3-acetylphenyl, 4-acetylphenyl, 2-propanoylphenyl, 2-(2-methyl-propanoyl)phenyl, 2-methoxyphenyl, 3-methoxyphenyl, 4-methoxyphenyl, 4-butoxyphenyl, 2-hydroxymethylphenyl, 4-hydroxymethylphenyl, 2-(1-hydroxyethyl)phenyl, 3-(1-hydroxyethyl)phenyl, 4-(1-hydroxyethyl)phenyl, 2-(1-hydroxypropyl)phenyl, 4-(1-hydroxypropyl)phenyl, 2-(1-hydroxy-2,2-dimethyl-propyl)phenyl, 4-trifluoromethoxyphenyl, 2-aminophenyl,4-aminophenyl, 4-N,N-diethylaminophenyl, 4-aminomethylphenyl, 4-(2-aminoethyl)phenyl, 4-(3-aminopropyl)phenyl, 4-carboxyphenyl, 4-carbamoylphenyl, 4-N-methylcarbamoylphenyl, 4-N,N-dimethylcarbamoylphenyl, 2-isopropylaminomethylphenyl, 4-t-butoxycarbonylaminomethylphenyl, 4-(2-isopropoxy-carboxamido)ethylphenyl, 4-(2-t-butoxycarboxamido)ethyl-phenyl, 4-isopropylsulfonylaminophenyl, 4-(2-methane-sulfonylamino)ethylphenyl, 4-(2-ethylsulfonylamino)ethyl-phenyl, 4-(3-isopropylsulfonylamino)propylphenyl, 4-(1-(2-(2-propane)sulfonylamino) propyl)phenyl, 4-(2-propylsulfonylamino)ethylphenyl, 4-(2-isopropylsulfonylamino)ethylphenyl, 4-(2-butylsulfonylamino)ethylphenyl, 4-(1-isopropyl-sulfonylaminomethyl)ethylphenyl, 4-(1-hydroxy-2-methane-sulfonylamino)ethylphenyl, 4-(2-(2,2,2-trifluoroethyl)-sulfonylaminoethyl)phenyl, 4-(2-cyclohexylsulfonylamino)-ethylphenyl, 4-(2-(2,2,2-trifluoroethyl)sulfonylamino)-ethylphenyl, 4-(2-N,N-dimethylaminosulfonylamino)-ethylphenyl, 4-(2-phenylsulfonylaminoethyl) phenyl,4-(2-(2-fluorophenyl)sulfonylaminoethyl)phenyl,4-(2-(4-fluorophenyl)sulfonylaminoethyl)phenyl,4-(2-(2-trifluoromethylphenyl)sulfonylaminoethyl)phenyl, 4-(2-(4-trifluoromethylphenyl)sulfonylaminoethyl)phenyl, 4-(2-(4-methoxyphenyl)sulfonylaminoethyl)phenyl, 4-(2-(1-(5-dimethylamino)napthalenesulfonylamino)ethyl)phenyl, 4-(2-(2-thienyl) sulfonylamino)ethyl)phenyl, 4-(2-benzamidoethyl)-phenyl, 4-(2-(4-fluorobenzamido)ethyl)phenyl, 4-(2-(3-methoxybenzamido)ethyl)phenyl, 4-(2-(3-fluorobenzamido)-ethyl)phenyl, 4-(2-(4-methoxybenzamido)ethyl)phenyl, 4-(2-(2-methoxybenzamido)ethyl)phenyl, 4-(1-(2-(2-methoxycarbonylethanesulfonylamino)ethyl)phenyl, 4-(1-(2-(10-camphorsulfonylamino)ethyl)phenyl, 4-(1-(2-(benzylsulfonyl-amino)ethyl)phenyl, 4-(2-phenylacetamido)ethyl)phenyl, 4-methanesulfonylaminoethanoylphenyl, 4-(N-(t-butoxy-carbonyl)methanesulfonylaminoethanoyl)phenyl, 4-(2-(2-thienylcarboxamido)ethyl)phenyl, thien-2-yl, 5-hydroxy-methylthien-2-yl, 5-formylthien-2-yl, thien-3-yl, 5-hydroxymethylthien-3-yl, 5-formylthien-3-yl, 2-bromothien-3-yl, fur-2-yl, 5-nitrofur-2-yl, fur-3-yl, isoxazol-5-yl, 3-bromoisoxazol-5-yl, isoxazol-3-yl, 5-trimethylsilylisoxazol-3-yl, 5-methylisoxazol-3-yl, 5-hydroxymethylisoxazol-3-yl, 5-methyl-3-phenylisoxazol-4-yl, 5-(2-hydroxyethyl)isoxazol-3-yl, 5-acetylisoxazol-3-yl, 5-carboxyisoxazol-3-yl, 5-N-methylcarbamoylisoxazol-3-yl, 5-methoxycarbonylisoxazol-3-yl, 3-bromo[1,2,4]oxadiazol-5-yl, pyrazol-1-yl, thiazol-2-yl, 4-hydroxymethylthiazol-2-yl, 4-methoxycarbonylthiazol-2-yl, 4-carboxythiazol-2-yl, imidazol-1-yl, 2-sulfhydrylimidazol-1-yl, [1,2,4]triazol-1-yl, tetrazol-5-yl, 2-methyltetrazol-5-yl, 2-ethyltetrazol-5-yl, 2-isopropyl-tetrazol-5-yl, 2-(2-propenyl)tetrazol-5-yl, 2-benzyl-tetrazol-5-yl, pyrid-2-yl, 5-ethoxycarbonylpyrid-2-yl, pyrid-3-yl, 6-chloropyrid-3-yl, pyrid-4-yl, 5-trifluoro-methylpyrid-2-yl, 6-chloropyridazin-3-yl, 6-methylpyridazin-3-yl, 6-methoxypyrazin-3-yl, pyrimidin-5-yl, benzothien-2-yl, benzothiazol-2-yl, and quinol-2-yl.

Examples of an unsubstituted or substituted aromatic or heteroaromatic group represented by $R^1$ are unsubstituted or substituted phenyl, furyl, thienyl (such as 3-thienyl) and pyridyl (such as 3-pyridyl).

Examples of an unsubstituted or substituted (5-8C)cycloalkyl group represented by $R^1$ are unsubstituted or substituted cyclopentyl, cyclohexyl, cycloheptyl and cyclooctyl, with cyclohexyl being preferred.

More preferably, $R^1$ represents 2-naphthyl or a group of formula

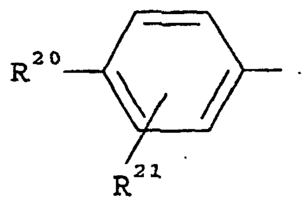 , 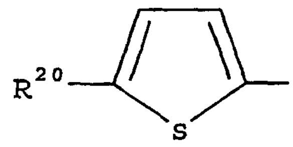 , 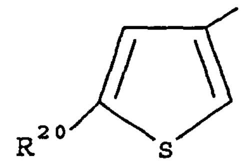

or

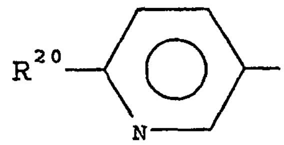

in which

$R^{20}$ represents halogen; nitro; cyano; hydroxyimino; (1-10C)alkyl; (2-10C)alkenyl; (2-10C)alkynyl; (3-8C)cycloalkyl; hydroxy(3-8C)cycloalkyl; oxo(3-8C)cycloalkyl; halo(1-10C)alkyl; $(CH_2)_yX^1R^9$ in which y is 0 or an integer of from 1 to 4, $X^1$ represents O, S, $NR^{10}$, CO, COO, OCO, $CONR^{11}$, $NR^{12}CO$, $NR^{12}COCOO$, $OCONR^{13}$, $R^9$ represents hydrogen, (1-10C) alkyl, (3-10C)alkenyl, (3-10C)alkynyl, pyrrolidinyl, tetrahydrofuryl, morpholino or (3-8C)cycloalkyl and $R^{10}$, $R^{11}$, $R^{12}$ and $R^{13}$ each independently represents hydrogen or (1-10C)alkyl, or $R^9$ and $R^{10}$, $R^{11}$, $R^{12}$ or $R^{13}$ together with the nitrogen atom to which they are attached form an azetidinyl, pyrrolidinyl, piperidinyl or morpholino group; N-(1-4C)alkylpiperazinyl; N-phenyl(1-4C)alkylpiperazinyl; thienyl; furyl; oxazolyl; isoxazolyl; pyrazolyl; imidazolyl; thiazolyl; tetrazolyl; pyridyl; pyridazinyl; pyrimidinyl; dihydrothienyl; dihydrofuryl; dihydrothiopyranyl; dihydropyranyl; dihydrothiazolyl; (1-4C)alkoxycarbonyldihydrothiazolyl; (1-4C)alkoxycarbonyldimethyl-dihydrothiazolyl; tetrahydrothienyl; tetrahydrofuryl; tetrahydrothiopyranyl; tetrahydropyranyl; indolyl; benzofuryl; benzothienyl; benzimidazolyl; benzothiazolyl; and a group of formula $R^{14}\text{-}(L^a)_n\text{-}X^2\text{-}(L^b)_m$ in which $X^2$ represents a bond, O, NH, S, SO, $SO_2$, CO, CH (OH), CONH, NHCONH, NHCOO, COCONH, $OCH_2CONH$ or CH=CH, NHCO, $L^a$ and $L^b$ each represent (1-4C) alkylene, one of n and m is 0 or 1 and the other is 0, and $R^{14}$ represents a phenyl or hetero-aromatic group which is unsubstituted or substituted by one or two of halogen; nitro; cyano; (1-10C)alkyl; (2-10C)alkenyl; (2-10C)alkynyl; (3-8C) cycloalkyl; 4-(1,1-dioxotetrahydro-1,2-thiazinyl); halo(1-10C)alkyl; cyano(2-10C)alkenyl; phenyl; $(CH_2)_zX^3R^{15}$ in which z is 0 or an integer of from 1 to 4, $X^3$ represents O, S, $NR^{16}$, CO, CH(OH), COO, OCO, $CONR^{17}$, $NR^{18}CO$, $NHSO_2$, $NHSO_2NR^{17}$, NHCONH, $OCONR^{19}$ or $NR^{19}COO$, $R^{15}$ represents hydrogen, (1-10C)alkyl, phenyl(1-4C)alkyl, halo (1-10C)alkyl, (1-4C)alkoxycarbonyl(1-4C)alkyl, (1-4C)alkylsulfonylamino(1-4C)alkyl, (N-(1-4C)alkoxycarbonyl)(1-4C) alkylsulfonylamino(1-4C)alkyl, (3-10C)alkenyl, (3-10C)alkynyl, (3-8C)cycloalkyl, camphoryl or an aromatic or heteroaromatic group which is unsubstituted or substituted by one or two of halogen, (1-4C)alkyl, halo(1-4C)alkyl, di(1-4C) alkylamino and (1-4C)alkoxy, and $R^{16}$, $R^{17}$, $R^{18}$ and $R^{19}$ each independently represents hydrogen or (1-10C)alkyl, or $R^{15}$ and $R^{16}$, $R^{17}$, $R^{18}$ or $R^{19}$ together with the nitrogen atom to which they are attached form an azetidinyl, pyrrolidinyl, piperidinyl or morpholino group; and

$R^{21}$ represents a hydrogen atom, a halogen atom, a (1-4C)alkyl group or a (1-4C)alkoxy group.

Examples of particular values for $R^{20}$ are fluorine, chlorine, bromine, cyano, hydroxyimino, methyl, ethyl, propyl, 2-propyl, butyl, 2-methylpropyl, 1,1-dimethylethyl, cyclopentyl, cyclohexyl, 3-hydroxycyclopentyl, 3-oxocyclopentyl, methoxy, ethoxy, propoxy, 2-propoxy, acetyl, acetylamino, ethylcarboxamido, propylcarboxamido, 1-butanoylamido, t-butylcarboxamido, acryloylamido, 2-pyrrolidinylcarboxamido, 2-tetrahydrofurylmethoxy, morpholinocarboxamido, methyloxalylamido, cyclo-propylcarboxamido, cyclobutylcarboxamido, cyclopentyl-carboxamido, cyclohexylcarboxamido, cyclopropylcarbamoyl, cyclopentylcarbamoyl, pyrrolidin-1-yl, morpholino, piperidin-1-yl, N-methylpiperazinyl, N-benzylpiperazinyl, 2-thienyl, 3-thienyl, 2-furyl, 3-furyl, isoxazol-3-yl, thiazol-2-yl, tetrazol-5-yl, pyrid-2-yl, pyrid-3-yl, pyrid-4-yl, pyrimidin-5-yl, 4,5-dihydrothiazol-2-yl, 4,5-dihydro-4-methoxycarbonylthiazol-2-yl, 4,5-dihydro-4-methoxy-carbonyl-5,5-dimethylthiazol-2-yl, benzothien-2-yl, benzothiazol-2-yl, phenyl, 2-fluorophenyl, 3-fluorophenyl, 2,3-difluorophenyl, 4-chlorophenyl, 3,4-dichlorophenyl, 3,5-dichlorophenyl, 3-nitrophenyl, 4-cyanophenyl, 2-methylphenyl, 4-methylphenyl, 4-(4-(1,1-dioxotetrahydro-1,2-thiazinyl)phenyl, 3-trifluoromethylphenyl, 4-trifluoro-methylphenyl, 4-(2-cyanoethenyl)phenyl, 2-formylphenyl, 3-formylphenyl, 4-formylphenyl, 3-acetylphenyl, 4-acetylphenyl, 4-carboxyphenyl, 2-methoxyphenyl, 4-methoxyphenyl, 2-hydroxymethylphenyl, 4-hydroxymethylphenyl, 3-(1-hydroxyethyl) phenyl, 4-(1-hydroxyethyl)phenyl, 4-(1-hydroxypropyl)phenyl, 2-aminophenyl, 4-aminophenyl, 4-N,N-diethylaminophenyl, 4-aminomethylphenyl, 4-(2-aminoethyl)-phenyl, 4-(3-aminopropyl)phenyl, 4-(2-acetylaminoethyl)-phenyl, 4-t-butoxycarboxylaminoethyl)phenyl, 4-(2-t-butoxycarboxylaminoethyl)phenyl, benzylsulfonylamino, 4-isopropylsulfonylaminophenyl, 4-(2-methanesulfonylaminoethyl)phenyl, 4-(2-ethylsulfonylaminoethyl)phenyl, 4-(2-propylsulfonylaminoethyl)phenyl, 4-(2-butylsulfonyl-aminoethyl)phenyl, 4-(2-isopropy)sulfonylaminoethyl)phenyl, 4-(1-hydroxy-2-methanesulfonylaminoethyl)phenyl, 4-(2-dimethylaminosulfonylaminoethyl)phenyl, 4-(1-(2-(2-propyl)sulfonylaminopropyl)phenyl, 4-(2-(2,2,2-trifluoroethyl)sulfonylaminoethyl)phenyl, 4-(2-cyclohexylsulfonyl-aminoethyl)phenyl, 4-(2-phenylsulfonylaminoethyl)phenyl, 4-(2-(2-fluorophenyl)sulfonylaminoethyl)phenyl, 4-(2-(4-fluorophenyl)sulfonylaminoethyl)phenyl, 4-(2-(2-trifluoromethylphenyl)sulfonylaminoethyl)phenyl, 4-(2-(4-trifluoromethylphenyl)sulfonylaminoethyl)phenyl, 4-(2-(4-methoxyphenyl)sulfonylaminoethyl)phenyl, 4-(2-(1-(5-dimethylamino)napthalenesulfonylamino)ethyl)phenyl, 4-(2-(2-thienyl)sulfonylamino)ethyl)phenyl, 4-(2-benzamidoethyl)-phenyl, 4-(2-(4-fluorobenzamido)ethyl)phenyl, 4-(2-(3-methoxybenzamido)ethyl)phenyl, 4-(2-(3-fluorobenzamido)-ethyl)phenyl, 4-(2-(4-methoxybenzamido)ethyl)phenyl, 4-(2-(2-methoxybenzamido)ethyl)phenyl, 4-(2-(2-thienyl-carboxamido)ethyl)phenyl, 4-carbamoylphenyl, 4-methyl-carbamoylphenyl, 4-dimethylcarbamoylphenyl, 4-(2-(2-methylpropaneamido)ethyl)phenyl, 4-(2-(3-methyl-butaneamido)ethyl)phenyl, benzoylmethyl, benzamido, 2-fluorobenzamido, 3-flurobenzamido, 4-fluorobenzamido, 2,4-difluorobenzamido, 3-chlorobenzamido, 4-chlorobenzamido, 4-bromobenzamido, 4-iodobenzamido, 4-cyanobenzamido, 3-methylbenzamido, 4-methylbenzamido, 4-ethylbenzamido, 4-propylbenzamido, 4-t-butylbenzamido, 4-vinylbenzamido, 2-trifluoromethylbenzamido, 3-trifluoromethylbenzamido, 4-trifluoromethylbenzamido, 2-fluoro-4-trifluoromethylbenzamido, 2-methoxybenzamido, 3-methoxybenzamido, 4-methoxybenzamido, 4-butoxybenzamido, 4-phenylphenyl-carboxamido, 4-benzylcarboxamido, 4-phenoxymethyl-carboxamido, 2-fluorobenzylamino, benzyloxy, 2-fluorobenzyloxy, 2-hydroxy-2-phenylethyl, 2-fluorophenylcarbamoyl, 4-(1-(2-(2-methoxycarbonylethanesulfonylamino)ethyl)phenyl, 4-(1-(2-(10-camphorsulfonylamino)ethyl)phenyl, 4-(1-(2-(benzylsulfonylamino)ethyl)phenyl, 4-(2-phenylacetamido)-ethyl)phenyl, 4-(methanesulfonylaminoethanoyl)phenyl, 4-(N-t-butoxycarbonyl)methanesulfonylaminoethanoyl)phenyl, 2-thienylcarboxamido, 2-furylcarboxamido, 3-(5-methyl-isoxazolyl)carboxamido, 5-isoxazolylcarboxamido, 2-benzothienylcarboxamido, 4-(5-methyl-3-phenylisoxazolyl)-carboxamido, 4-pyridylcarboxamido, 2-(5-nitrofuryl)-carboxamido; 2-pyridylcarboxamido, 6-chloro-2-pyridyl-carboxamido, 2-thienylsulfonamido, 2-thienylmethylamino, 3-thienylmethylamino, 2-furylmethylamino, 3-furylmethylamino, 3-acetylureido and 2-(2-thienyl)ethylureido.

Examples of particular values for $R^{21}$ are hydrogen and chlorine. $R^{21}$ is preferably ortho to $R^{20}$.

Examples of particular values for $R^1$ are 2-naphthyl, 4-bromophenyl, 4-cyanophenyl, 4-benzamidophenyl, 4-methylphenyl, 4-isopropyl-phenyl, 4-isobutylphenyl, 4-*t*-butylphenyl, 4-methoxyphenyl, 4-isopropoxyphenyl, 4-cyclopentylphenyl, 4-cyclohexylphenyl, 4-(2-hydroxymethylphenyl)phenyl, 4-(4-hydroxymethylphenyl)-phenyl, 4-(2-furyl)phenyl, 4-(3-furyl)phenyl, 4-(2-thienyl)-phenyl, 4-(3-thienyl)phenyl, 4-(pyrrolidin-1-yl)phenyl, 4-(piperidin-1-yl)phenyl, 3-chloro-4-piperidin-1-ylphenyl, 4-benzyloxyphenyl, 4-(2-fluorophenyl)phenyl, 4-(3-fluoro-phenyl)phenyl, 4-(2-formylphenyl)phenyl, 4-(3-formylphenyl)-phenyl, 4-(4-formylphenyl)phenyl, 4-(4-methylphenyl)phenyl, and 4-(2-methoxyphenyl)phenyl.

The compounds of formula I can be prepared following the various procedures set forth below. The reagents and starting materials are readily available to one of ordinary skill in the art. All substituents, unless otherwise specified are as previously defined.

Scheme I

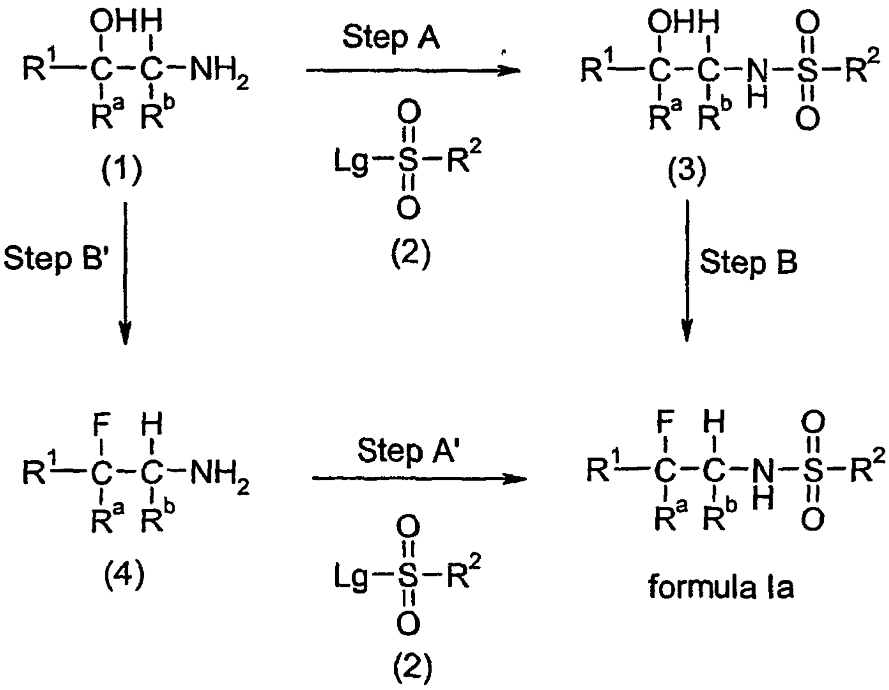

In Scheme I, step A the compound of structure (1) is combined with the compound of structure (2) under conditions well known in the art to provide the compound of structure (3). More specifically, for example, the compound (1) is dissolved in a suitable organic solvent. Examples of suitable organic solvents include methylene chloride, tetrahydrofuran, and the like. The solution is treated with a slight excess of a suitable base, and then cooled to about -78°C to about 0°C. Examples of suitable bases include triethylamine, pyridine, 1,8-diazabicyclo[5.4.0]undec-7-ene (DBU),and the like. To the stirring solution is added one equivalent of compound (2). The term "Lg" as used herein refers to a suitable leaving group. Examples of suitable leaving groups include, Cl, Br, methanesulfonyloxy, triflupromethanesulfonyloxy, benzenesulfonyloxy, p-toluenesulfonyloxy, and the like. Cl is the preferred leaving group. The reaction mixture is stirred at about 0°C to about 50°C for about 0.5 hours to about 16 hours. The compound (3) is then isolated and purified by techniques well known in the art, such as extraction techniques and chromatography. For example, the mixture is washed with 10% sodium bisulfate, the layers separated and the aqueous extracted with several times with a suitable organic solvent, such as methylene chloride. The organic extracts are combined, dried over anhydrous sodium sulfate, filtered and concentrated under vacuum. The residue is then purified by flash chromatography on silica gel with a suitable eluent such as ethyl acetate/hexane to provide the compound (3).

In Scheme 1, step B the compound of structure (3) is fluorinated under conditions well known in the art to provide the compound of formula Ia. For example, compound (3) is dissolved in a suitable organic solvent, such as methylene chloride and the solution is cooled to about -78°C under an inert atmosphere, such as nitrogen. To this solution is added slowly, about one equivalent of diethylaminosulfur trifluoride (DAST) dissolved in a suitable organic solvent, such as methylene chloride with stirring. The reaction is then allowed to warm to room temperature (about 22°C) and the compound of formula Ia is then isolated and purified using techniques and procedures well known in the art, such as extraction techniques and chromatography. For example, the reaction is diluted with water and methylene chloride. The layers are separated and the organic layer is washed with water, dried over anhydrous sodium sulfate, filtered and concentrated under vacuum to provide the crude compound of formula Ia. This crude material can then be purified by standard techniques, such as recrystallization from a suitable eluent, or flash chromatography or radial chromatography (radial chromatography is carried out using a Chromatotron, Harrison Research Inc., 840 Moana Court, Palo Alto CA 94306) on silica gel, with a suitable eluent, such as hexane/ethyl acetate to provide purified compound of formula Ia.

Alternatively, in Scheme I, step B' the compound (1) is fluorinated in a manner analogous to the procedure described in step B above with DAST to provide the compound of structure (4). In Scheme I, step A' compound (4) is converted to the compound of formula Ia in a manner analogous to the procedure described in step A above.

Scheme II

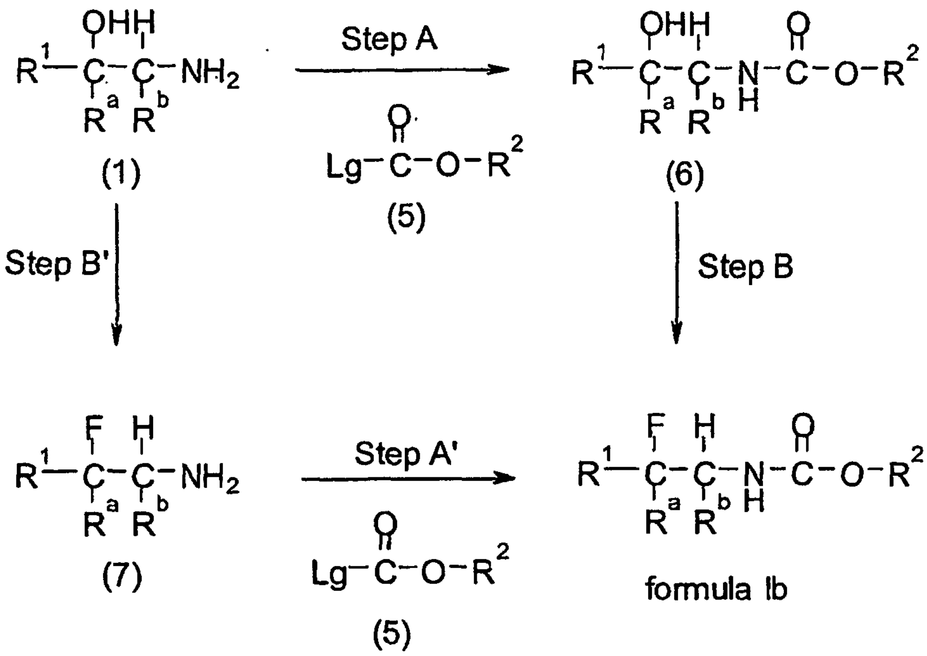

(1) (5) (6) (7) (5) formula Ib

In Scheme II, step A compound (1) is converted to the carbamate (6) under standard carbamate forming conditions well known to one of ordinary skill in the art. For examples of standard carbamate forming conditions see J. March, "Advanced Organic Chemistry: Reactions, Mechanisms, and Structure," 2nd Edition, McGraw Hill Inc., (1977) pages 382-383, and T.W Green, "Protective Groups in Organic Synthesis," John Wiley & Sons, Inc. (1981) pages 223-248.

More specifically, for example, compound (1) is dissolved in a suitable organic solvent, such as tetrahydrofuran or methylene chloride and treated with an equivalent of a compound (5) wherein "Lg" represents a suitable leaving group. Examples of suitable leaving groups are Cl, Br, I, and the like. The reaction can be performed at a temperature of from about -10°C to about 50°C, preferably at a temperature of about 0°C to about 25°C. After about 2 hours to about 12 hours, the carbamate (6) is isolated and purified by techniques well known in the art, such as extraction techniques and chromatography. For example, the reaction is diluted with a suitable organic solvent, such as methylene chloride, rinsed with saturated sodium bicarbonate, brine, dried over anhydrous magnesium sulfate, filtered and concentrated under vacuum. The crude product can then be purified by flash chromatography on silica gel with a suitable eluent, such ethyl acetate/hexanes to provide the purified carbamate (6).

In Scheme II, step B carbamate (6) is converted to the compound of formula Ib in a manner analogous to the procedure set forth in Scheme I, step B.

Alternatively, in Scheme II, step B' the compound (1) is fluorinated in a manner analogous to the procedure described in step B above with DAST to provide the compound of structure (7). In Scheme II, step A' compound (7) is converted to the compound of formula Ib in a manner analogous to the procedure described in step A above.

## Scheme III

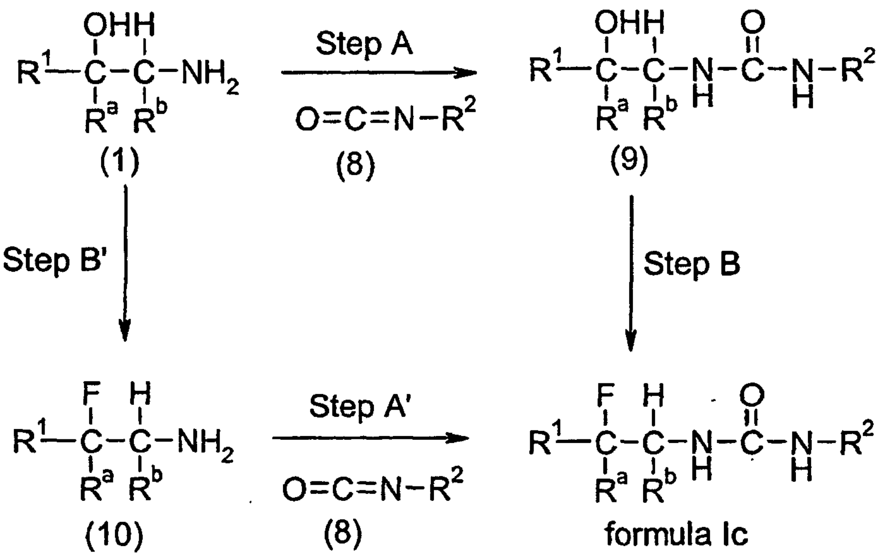


(1) (8) (9) (10) (8) formula Ic

In Scheme III, step A urea (9) is prepared from compound (1) under standard urea forming conditions well known to one of ordinary skill in the art. For examples of standard urea forming conditions see J. March, "Advanced Organic Chemistry: Reactions, Mechanisms, and Structure," 2nd Edition, McGraw Hill Inc., (1977) page 823, and T.W Green, "Protective Groups in Organic Synthesis," John Wiley & Sons, Inc. (1981) pages 248-49.

More specifically, for example, a compound (1) is dissolved in a suitable organic solvent, such as methylene chloride, and the solution is treated with about 1.1 equivalents of an isocyanate (8). The reaction can be performed at a temperature of about -10°C to about 50°C for about 2 hours to about 12 hours to provide the urea (9). The urea (9) can be isolated and purified by techniques well known in the art, such as extraction techniques and chromatography. For example, the reaction is diluted with a suitable organic solvent, such as methylene chloride, rinsed with water, brine, dried over anhydrous sodium sulfate, filtered and concentrated under vacuum. The crude product can then be purified by flash chromatography on silica gel with a suitable eluent, such ethyl acetate/hexanes to provide the purified urea (9).

In Scheme III, step B urea (9) is converted to the compound of formula Ic in a manner analogous to the procedure set forth in Scheme I, step B.

Alternatively, in Scheme III, step B' the compound (1) is fluorinated in a manner analogous to the procedure described in step B above with DAST to provide the compound of structure (10). In Scheme III, step A' compound (10) is converted to the compound of formula 1c in a manner analogous to the procedure described in step A above.

The compounds of formula I in which $R^1$ represents a 4-bromophenyl group, a 4-iodophenyl group or a 4-(triflate)phenyl group may conveniently be converted into other compounds of formula I in which R represents another 4-substituted phenyl group under conditions well known in the art, such as by reaction with an appropriate boronic acid derivative, for example, a benzeneboronic acid derivative. See for example, International Publication Number WO 98/33496, published August 6, 1998, the disclosure of which is hereby incorporated by reference. More specifically, the reaction is conveniently performed in the presence of a tetrakis (triarylphosphine)palladium(0) catalyst, such as tetrakis (triphenylphosphine)palladium(0) and a base such as potassium carbonate. Convenient solvents for the reaction include aromatic hydrocarbons, such as toluene. The temperature at which the reaction is conducted is conveniently in the range of from 0 to 150°C, preferably 75 to 120°C. Bis aromatic intermediates useful in the preparation of compounds of formula I may be prepared by reacting a bromoaromatic or bromoheteroaromatic compound with an aromatic or heteroaromatic boronic acid in an analogous manner.

Alternatively, the coupling reaction may be carried out using palladium diacetate with a suitable organic solvent, such as n-propanol or acetone. See for example, *Organic Synthesis* 1998, 75, 61; Goodson, F. E.; Wallow, T. I.; Novak, B. M. and *Organic Synthesis* 1998, 75, 53; Huff, B. E.; Koenig, T. M.; Mitchell, D.; Staszak, M. A. wherein analogous coupling conditions are employed.

The boronic acid derivative used as a starting material may be prepared by reacting a trialkyl borate, such as triisopropyl borate with an appropriate organolithium compound at reduced temperature. For example, 2-fluorobenzeneboronic acid may be prepared by reacting 2-fluorobromobenzene with butyllithium in tetrahydrofuran at about -78°C to afford 2-fluorophenyl lithium, and then reacting this organolithium compound with triisopropyl borate. This is followed by hydrolysis with aqueous HCl.

Alternatively, the compounds of formula I in which $R^1$ represents a 4-bromophenyl group may be converted to a 4-(trimethylstannyl)phenyl or 4-(tri-*n*-butylstannyl)phenyl group by treatment of the corresponding bromide with a palladium(0) catalyst, such as tetrakis(triphenylphosphine)-palladium(0) and hexaalkyldistannane, where the alkyl group is methyl or n-butyl, in an aprotic solvent such as toluene in the presence of a tertiary amine base such as triethylamine, at temperatures ranging from 80 to 140°C, preferably from 90 to 110°C.

The compounds of formula I in which $R^1$ represents a 4-(tri-n-butylstannyl)phenyl group may then be reacted with an aryl- or heteroarylbromide, such as 2-bromothiophene-5-carboxaldehyde, or an aryl- or heteroaryliodide, or an aryl- or heteroaryltriflate, in the presence of a palladium(0) catalyst, such as tetrakis(triphenylphosphine)palladium(0), or a palladium(II) catalyst, such as bis(triphenytphosphine)-palladium(II) dichloride, in an aprotic solvent, such as dioxane, at temperatures ranging from 80 to 140°C, preferably from 90 to 110°C, to afford the corresponding 4-(aryl) phenyl or 4-(heteroaryl)phenyl substituted compound.

The compounds of formula I in which $R^1$ represents a 4-bromophenyl group may be converted into other compounds of formula I in which $R^1$ represents a 4-substituted alkyl- or cycloalkylphenyl group, such as 4-cyclopentylphenyl by treatment of the corresponding bromide with an appropriate alkyl- or cycloalkyl Grignard reagent, such as cyclopentyl-magnesium bromide, in the presence of a palladium(II) catalyst, such as [1,1'-bis(diphenylphosphino)ferrocene]-dichloropalladium(II)($PdCl_2$(dppf)), in an aprotic solvent, such as diethyl ether at temperatures ranging from -78°C to 25°C.

The compounds of formula I in which $R^1$ represents a 4-bromophenyl group may be converted into a 4-substituted carboxyaldehydephenyl(formylphenyl) group by reaction of the corresponding bromide with the carbon monoxide gas which is bubbled into the reaction under atmospheric pressure in the presence of a palladium(II) catalyst, such as bis(triphenyl-phosphine)palladium(II) dichloride and sodium formate in an aprotic solvent, such as dimethylformamide at temperatures ranging from 70 to 110°C, preferably at 90°C.

The compounds of formula I in which $R^1$ represents a 4-hydroxyphenyl group may be converted into other compounds of formula I in which $R^1$ represents an alkoxy group by treatment of the corresponding hydroxyphenyl group with an appropriate alkylhalide such as benzylbromide in the presence of sodium hydride in an aprotic solvent such as dimethylformamide at temperatures ranging from 25 to 100°C, preferably from 50 to 90°C.

The compounds of structure (1a) can be prepared following the procedure described in Scheme IV. The reagents and starting materials are readily available to one of ordinary skill in the art. All substituents, unless otherwise specified are as previously defined.

Scheme IV

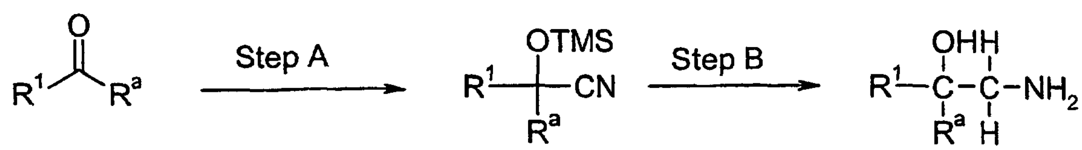


(11) (12) (1a)

TMS = trimethylsilyl

In Scheme IV, step A the compound of structure (11) is converted to the compound of structure (12) under standard conditions. For example, see Greenlee and Hangauer, *Tetrahedron Lett.,* 24(42), 4559 (1983). For example, compound (11) is dissolved in a suitable organic solvent, such as dry tetrahydrofuran, containing excess 18-crown-6, and excess potassium cyanide. To this mixture at room temperature is added dropwise about 1.2 equivalents of cyanotrimethylsilane. The reaction mixture is allowed to stir for about 1 to 4 hours to provide compound (12). Compound (12) is then carried on directly to step B without isolation.

Alternatively, in Scheme IV, step A, for example, compound (11) is combined with a catalytic amount of zinc iodide followed by slow addition of excess trimethylsilyl cyanide with the generation of heat. The resulting solution is stirred at room temperature under nitrogen for about 8 to 16 hours. The mixture is then diluted with a suitable organic solvent, such as chloroform, washed with saturated sodium bicarbonate, water, brine, dried over anhydrous magnesium sulfate, filtered, and concentrated under vacuum to provide compound (12).

In Scheme IV, step B compound (12) is converted to compound of structure (1a). For example, compound (11) prepared above, still in solution, is treated with a solution of about 1.4 equivalents of borane in dimethylsulfide. The reaction mixture is then heated to reflux for about 16 hours and then cooled to room temperature. The reaction mixture is then cautiously treated with anhydrous HCl in methanol and allowed to stir for about one hour. The product

(1a) is then isolated and purified using standard techniques and procedures. For example, the solvent is removed under vacuum and the residue triturated with a suitable organic solvent, such at methy t-butyl ether and the solid is collected by filtration. The solid is then suspended in methylene chloride/tetrahydrofuran mixture (1:2.4) and treated with 1N NaOH until about pH 12.3 is reached. The phases are separated and the organic phase is rinsed with brine. The organic phase is then concentrated under vacuum and the residue triturated with diethyl ether to provide the purified compound (1a). Compound (1a) is then used in Schemes I, II and III in a manner analogous to the procedures described for compound (1).

More specifically, compounds of structure (1a') can be prepared as disclosed in Scheme V. The reagents and starting materials are readily available to one of ordinary skill in the art. All substituents, unless otherwise specified are as previously defined.

Scheme V

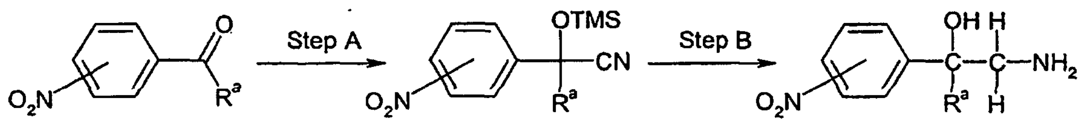


(11a) (12a) (1a')

In Scheme V, step A, the compound of structure (11a) is converted to the compound of structure (12a) in a manner analogous to the procedure described in Scheme IV, step A.

In Scheme V, step B, the compound of structure (12a) is converted to the compound of structure (1a') in a manner analogous to the procedure described in Scheme IV, step B. Compound (1a') can then be converted to a sulfonamide, carbamate or urea in a manner analogous to the procedures described in Schemes I, II and III above. The resulting sulfonamide, carbamate, or urea can then be fluorinated with DAST in a manner analogous to the procedures set forth above in Schemes I, II and III. The resulting fluorinated product possessing a nitro substituent on the phenyl group can then be hydrogenated under standard conditions well known to one of ordinary skill in the art to provide the corresponding amino substituted compound. For example, see J. March, "Advanced Organic Chemistry: Reactions, Mechanisms, and Structure," second edition, 1977, 1968 McGraw-Hill, Inc., pages 1125-1126.

In addition, compounds of structure (1a") can be prepared as disclosed in Scheme Va. The reagents and starting materials are readily available to one of ordinary skill in the art. All substituents, unless otherwise specified are as previously defined.

Scheme Va

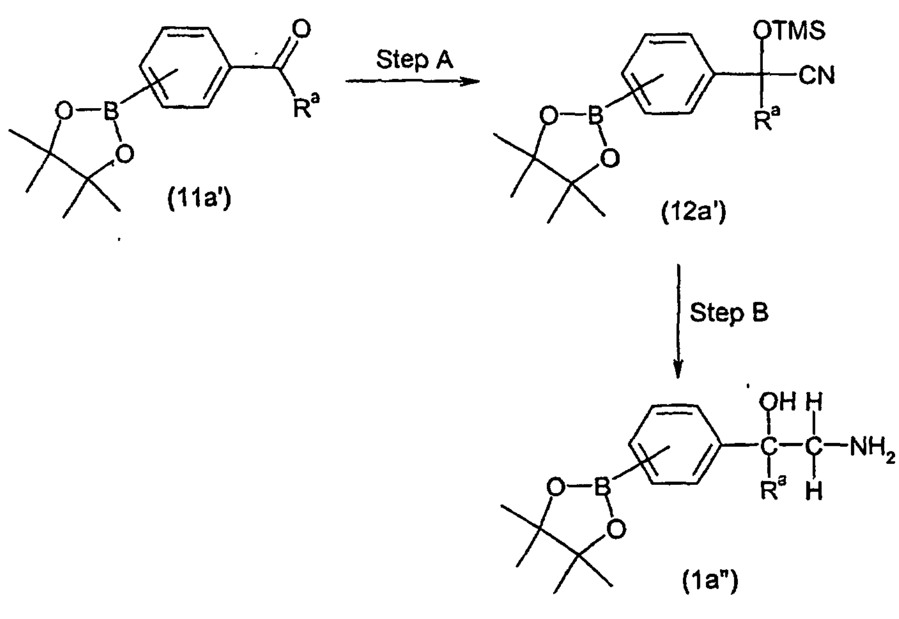


(11a') (12a') (1a")

In Scheme Va, step A, the compound of structure (11a') is converted to the compound of structure (12a') in a manner analogous to the procedure described in Scheme IV, step A.

In Scheme Va, step B, the compound of structure (12a') is converted to the compound of structure (1a") in a manner analogous to the procedure described in Scheme IV, step B. Compound (1a") can then be converted to a sulfonamide, carbamate or urea in a manner analogous to the procedures described in Schemes I, II and III above. The resulting sulfonamide, carbamate, or urea can then be fluorinated with DAST in a manner analogous to the procedures set forth above in Schemes I, II and III.

The compounds of formula Id and formula Ie can be prepared following the procedure described in Scheme VI. The reagents and starting materials are readily available to one of ordinary skill in the art. All substituents, unless otherwise specified are as previously defined.

Scheme VI

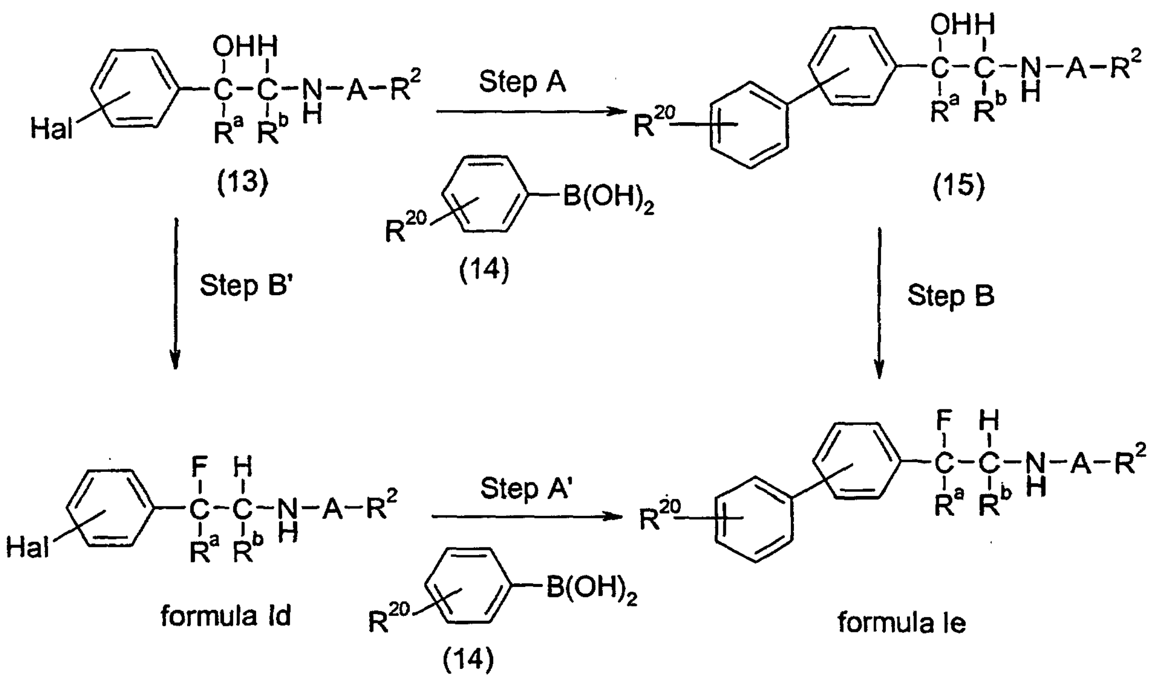


In Scheme VI, step A the compound of structure (13) is coupled with compound of structure (14) under standard conditions to provide compound of structure (15). For example, compound (13) is combined with about 1.5 equivalents of compound (14), about 1.5 equivalents of potassium carbonate, and about 0.06 equivalents of tetrakis(triphenyl phosphine)palladium(0) in a suitable solvent or solvent mixture, such as dioxane/water (3:1). The mixture is then heated at about 100°C for about 18 hours. The reaction is then cooled and compound (15) is isolated and purified using standard techniques and procedures, such as extraction techniques and chromatography. For example, the reaction mixture is extracted with a suitable organic solvent, such as ethyl acetate, the organic extracts are combined, washed with water, dried over anhydrous sodium sulfate, filtered, and concentrated under vacuum. The crude material is then purified by chromatography on silica gel with a suitable eluent, such as hexane/ethyl acetate to provide purified compound (15).

In Scheme VI, step B, compound (15) is fluorinated under standard conditions to provide the compound of formula Ie. For example, compound (15) is dissolved in a suitable organic solvent, such as methylene chloride is added to about one equivalent of DAST at about -78°C with stirring under an atmosphere of nitrogen. The reaction is allowed to warm to room temperature and the compound of formula Ie is isolated and purified using standard techniques, such as extraction techniques and chromatography. For example, the reaction mixture is diluted with water and a suitable organic solvent, such as methylene chloride. The layers are separated and the organic layer is washed with water, dried over anhydrous sodium sulfate, filtered and concentrated under vacuum. The crude material is then purified by chromatography on silica gel with a suitable eluent, such as hexane/ethyl acetate to provide the purified compound of formula Ie.

Alternatively, in Scheme VI, step B' the compound (13) is fluorinated to provide the compound of formula Id in a manner analogous to the procedure described above in step B.

In addition, in Scheme VI, step A' the compound of formula Id is converted to the compound of formula Ie in a manner analogous to the procedure described above in step A.

More specifically, compounds of formula Ig and formula Ih can be prepared as shown in Scheme VII. Reagents and starting materials are readily available to one of ordinary skill in the art, for example, see International Patent Application No. PCT/US99/03449, published September 2, 1999. All substituents, unless otherwise specified, are previously defined.

Scheme VII

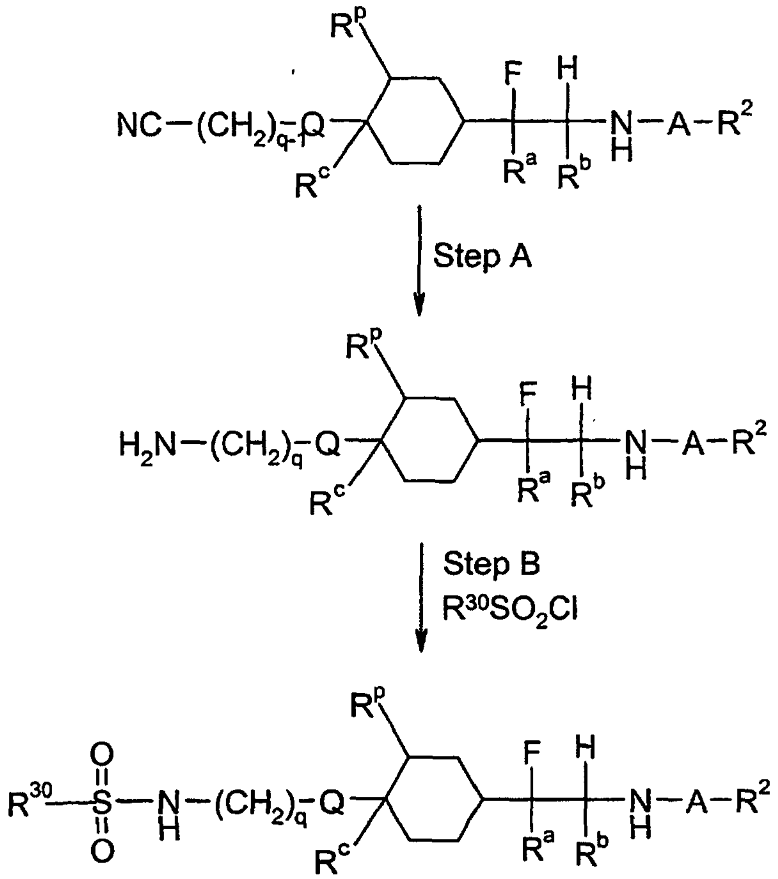


formula If

formula Ig

formula Ih

In Scheme VII, step A the compound of formula If wherein Q represents a (3-8C)cycloalkyl, an aromatic group, unsubstituted or substituted, such as phenyl, or a heteroaromatic group, unsubstituted or substituted, $R^c$ represents hydrogen, hydroxy, (1-4C)alkoxy, (1-4C)alkoxycarbonyl, or together with $R^p$ a bond, $R^p$ represents hydrogen, hydroxy, or together with $R^c$ a bond, q is an integer 1, 2, 3 or 4, $R^{30}$ represents (1-6C)alkyl, (3-6C)cycloalkyl, fluoro(1-6C)alkyl, chloro(1-6C)alkyl, (2-6C)alkenyl, (1-4C)alkoxy(1-4C)alkyl, phenyl which is unsubstituted or substituted by halogen, (1-4C)alkyl or (1-4C)alkoxy, (1-4C)alkylphenyl wherein the phenyl group is unsubstituted or substituted by halogen, (1-4C)alkyl or (1-4C)alkoxy, and the remaining substituents are defined as hereinabove, is converted to the amine of formula Ig under conditions well known in the art. For example, compound If is dissolved in a suitable organic solvent, such as tetrahydrofuran and heat to reflux. To the refluxing solution is added about 1.1 equivalents of a borane reagent, such as borane dimethylsulfide complex. The reaction mixture is then heated at reflux for about 1 to 2 hours, cooled to room temperature and then treated with 6N HCl. The reaction is again heated at reflux for about 1 hour, cooled and the pH is adjusted to about pH 10 with aqueous sodium hydroxide. The product, compound Ig, is then isolated and purified by standard techniques such as extraction and chromatography.

For example, the reaction mixture is diluted with water and extracted with a suitable organic solvent, such as dichloromethane. The organic extracts are combined, dried over anhydrous magnesium sulfate, filtered and concentrated under vacuum to provide formula Ig.

In Scheme VII, step B the compound of formula Ig is sulfonylated to provide the compound of formula Ih under conditions well known in the art. For example, compound Ig is dissolved in a suitable organic solvent, such as dichloromethane, followed by addition of about 1.05 equivalents of triethylamine. The solution is cooled to about 0°C and

treated with about 1.05 equivalents of a suitable sulfonyl chloride of formula $R^{30}SO_2Cl$, such as methanesulfonyl chloride. The reaction is then allowed to warm to room temperature over 2 hours with stirring. The product, compound Ih, is then isolated and purified using techniques well known to one of ordinary skill in the art, such as extraction and chromatography.

For example, the reaction mixture is then diluted with a suitable organic solvent, such as dichloromethane and 10% aqueous sodium bisulfate. The organic layer is separated and the aqueous layer is extracted with dichloromethane. The organic layer and extracts are then combined, dried over anhydrous sodium sulfate, filtered and concentrated under vacuum to provide compound Ih. Compound Ih can then be purified by flash chromatography on silica gel with a suitable eluent, such as ethyl acetate/hexane to provide purified compound Ih.

More specifically, compounds of formula Ij and formula Ik can be prepared as shown in Scheme VIII. Reagents and starting materials are readily available to one of ordinary skill in the art. All substituents, unless otherwise specified, are previously defined.

Scheme VIII

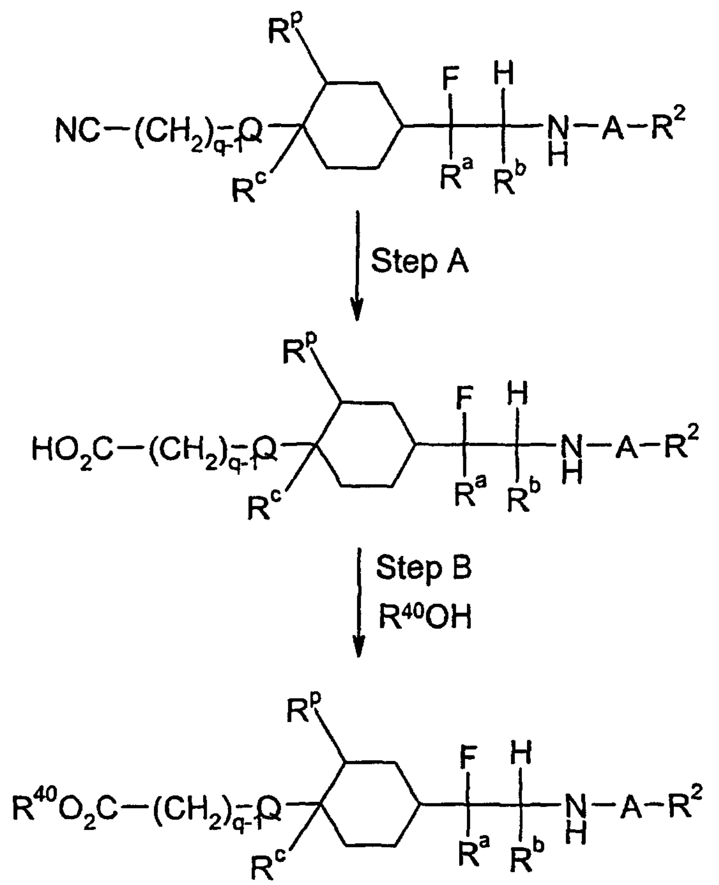


formula If

formula Ij

formula Ik

In Scheme VIII, step A the compound of formula If can be hydrolyzed under standard conditions to provide the compound of formula Ij. For example, compound If is dissolved in a suitable organic solvent, such as dioxane and treated with a suitable base, such as sodium hydroxide. The reaction mixture is then heated at about 100°C for about 24 hours. The reaction mixture is then cooled to room temperature and acidified with 10% sodium bisulfate. Compound Ij is then isolated and purified by techniques well known in the art, such as extraction and chromatography.

For example, the reaction mixture is extracted with a suitable organic solvent, such as ethyl acetate, the organic extracts are combined, dried over anhydrous magnesium sulfate, filtered and concentrated under vacuum to provide compound Ij. Compound Ij can be purified by flash chromatography on silica gel with a suitable eluent, such as methanol/chloroform.

In Scheme VIII, step B the compound of formula Ij can be esterified under conditions well known in the art to provide the compound of formula Ik. For example, compound Ij is dissolved in a suitable organic solvent of formula $R^{40}OH$, wherein $R^{40}$ represents (1-6C)alkyl, (3-6C)cycloalkyl, fluoro(1-6C)alky, chloro(1-6C)alkyl, (2-6C)alkenyl, (1-4C)alkoxy(1-4C)alkyl, phenyl which is unsubstituted or substituted by halogen, (1-4C)alkyl or (1-4C)alkoxy, (1-4C)alkylphenyl wherein the phenyl group is unsubstituted or substituted by halogen, (1-4C)alkyl or (1-4C)alkoxy, such as ethanol and HCl gas is bubbled through the solution until the mixture is saturated. The reaction mixture is then heated at 60°C for about 24 hours, then cooled to room temperature and concentrated under vacuum. Additional ethanol is added to the residue and the mixture is again concentrated under vacuum to provide the ethyl ester of compound Ik. Compound Ik can be then be purified by flash chromatography on silica gel with a suitable eluent, such ethyl acetate/hexane.

More specifically, compounds of formula Im can be prepared as shown in Scheme IX. Reagents and starting materials are readily available to one of ordinary skill in the art. All substituents, unless otherwise specified, are previously defined.

Scheme IX

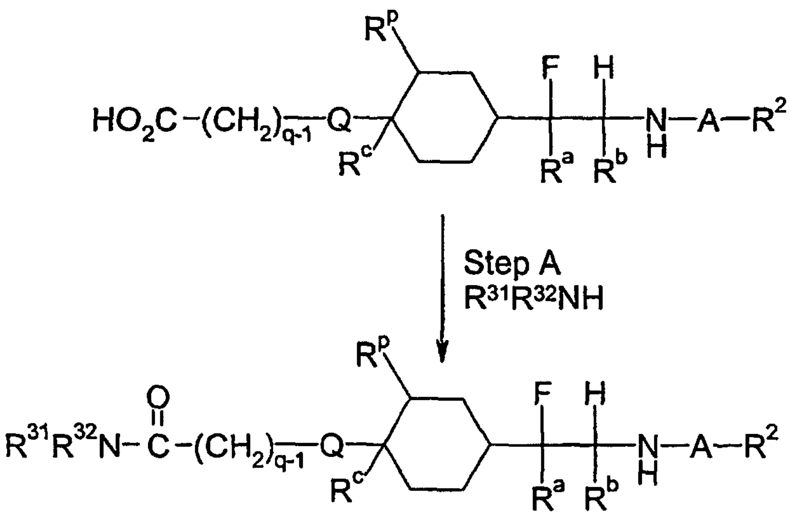


formula Ij

formula Im

In Scheme IX, step A compound Ij is readily converted to the amide of formula Im under conditions well known in the art. For example, compound Ij is dissolved in a suitable organic solvent, such as tetrahydrofuran and treated with an excess of thionyl chloride. The reaction mixture is stirred at room temperature for about 16 hours and then concentrated under vacuum. The residue is then dissolved in a suitable organic solvent, such as methylene chloride. The solution is added to a solution of one equivalent of a suitable amine of formula $R^{31}R^{32}NH$, wherein $R^{31}$ and $R^{32}$ independently represent (1-4C)alkyl or together with the nitrogen atom to which they are attached form an azetidinyl, pyrrolidinyl, piperidinyl, morpholino, piperazinyl, hexahydroazepinyl or octahydroazocinyl group, such as dimethylamine in dichloromethane with stirring. The mixture is stirred for about 2 hours at about 0°C and then 10% aqueous sodium bisulfate is added. Compound Im is then isolated and purified by techniques well known in the art, such as extraction and flash chromatography.

For example, the reaction mixture is then extracted with a suitable organic solvent, such as methylene chloride, the organic extracts are combined, dried over anhydrous sodium sulfate, filtered and concentrated under vacuum to provide compound Im. This can then be purified by flash chromatography on silica gel with a suitable eluent, such as ethyl acetate/hexane to provide the purified compound Im.

Compounds of formula In can be prepared as shown in Scheme X. Reagents and starting materials are readily available to one of ordinary skill in the art. All substituents, unless otherwise specified, are previously defined.

Scheme X

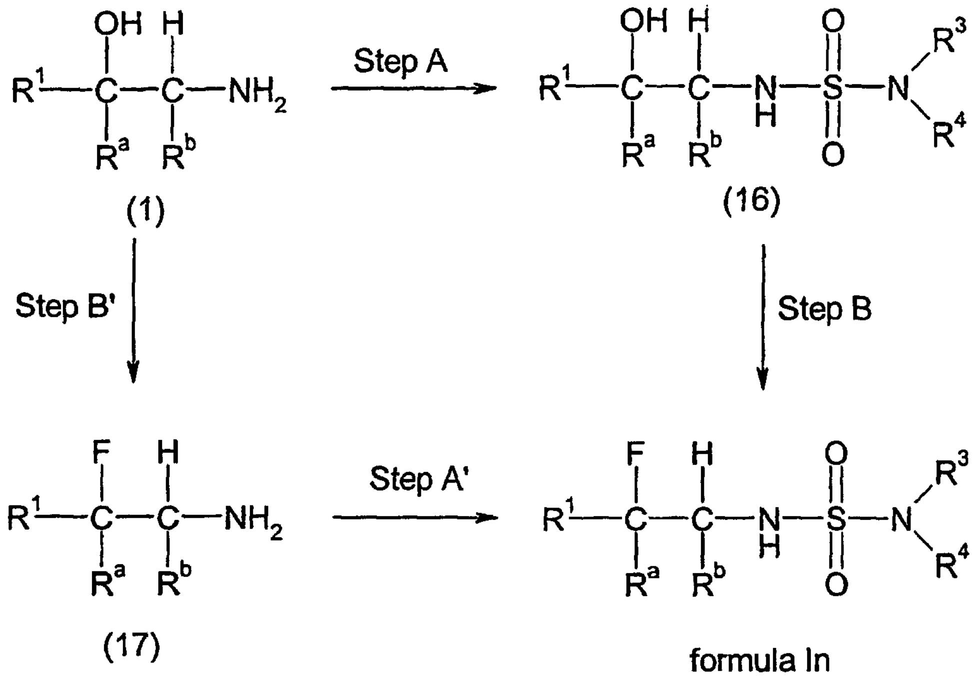


(1) (16) (17) formula In

In Scheme X, step A, the compound of structure (1) is combined with a compound of formula $ClSO_2NR^3R^4$ under standard conditions to provide the compound of structure (16). For example, compound (1) is dissolved in a suitable organic solvent, such as tetrahydrofuran and treated with about one equivalent of a suitable base, such as DBU at about 0°C. The solution is then treated with about one equivalent of a compound of fomula $ClSO_2NR^3R^4$. The reaction is then allowed to warm to room temperature and stirred for about 4 to 16 hours. The reaction is then concentrated under vacuum to provide the crude product (16) which can then be purified by chromatography on silica gel with a suitable eluent, such as ethyl acetate/hexane.

In Scheme X, step B, compound (16) is converted to the compound of formula In in a manner analogous to the procedure set forth in Scheme I, step B.

Alternatively, in Scheme X, step B' the compound (1) is fluorinated in a manner analogous to the procedure described in Scheme I, step B with DAST to provide the compound of structure (17). In Scheme X, step A' compound (17) is converted to the compound of formula In in a manner analogous to the procedure described above in step A.

Compounds of formula Ip can be prepared as shown in Scheme XI. Reagents and starting materials are readily available to one of ordinary skill in the art. All substituents, unless otherwise specified, are previously defined.

## Scheme XI

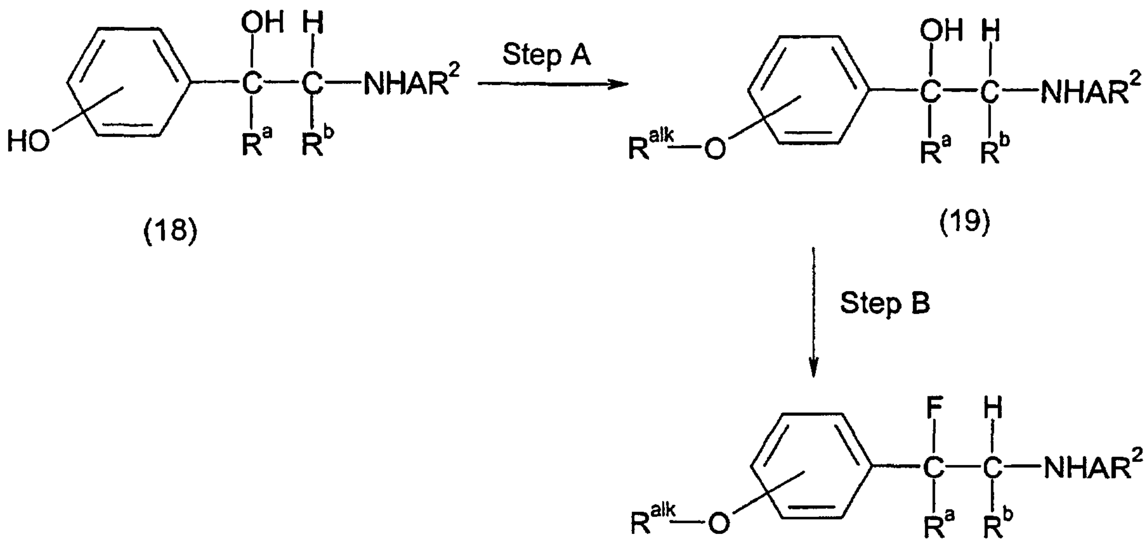


(18)

(19)

formula Ip

In Scheme XI, step A, the phenol of structure (18) is alkylated with a suitable alkylating agent of structure $R^{alk}$-Hal under standard conditions wherein Hal is Br or Cl, and $R^{alk}$ is an unsubstituted or substituted aromatic group, an unsubstituted or substituted heteroaromatic group, an unsubstituted or substituted (5-8C)cycloalkyl group, (1-10C) alkyl; (2-10C)alkenyl; or (2-10C)alkynyl to provide the compound of structure (19). For example, compound (18) is added to about one equivalent of a suitable base, such as sodium hydride, in a suitable organic solvent, such as dimethylformamide. The reaction mixture is stirred for about 30 minutes at room temperature and treated with about one equivalent of a suitable alkylating agent $R^{alk}$-Hal followed by addition of sodium iodide. The reaction is heated at about 100°C for about 2 hours and then cooled. The reaction is diluted with water, extracted with a suitable organic solvent, such as ethyl acetate, the organic extracts are combined, washed with water, dried over anhydrous sodium sulfate, filtered, and concentrated to provide crude compound (19). The crude material can be purified by chromatography on silica gel with a suitable eluent, such as ethyl acetate/hexane.

In Scheme X, step B, the compound (19) is fluorinated in a manner analogous to the procedure described in Scheme I, step B with DAST to provide the compound of formula Ip.

Compounds of formula Iq can be prepared as shown in Scheme XII. Reagents and starting materials are readily available to one of ordinary skill in the art. All substituents, unless otherwise specified, are previously defined.

## Scheme XII

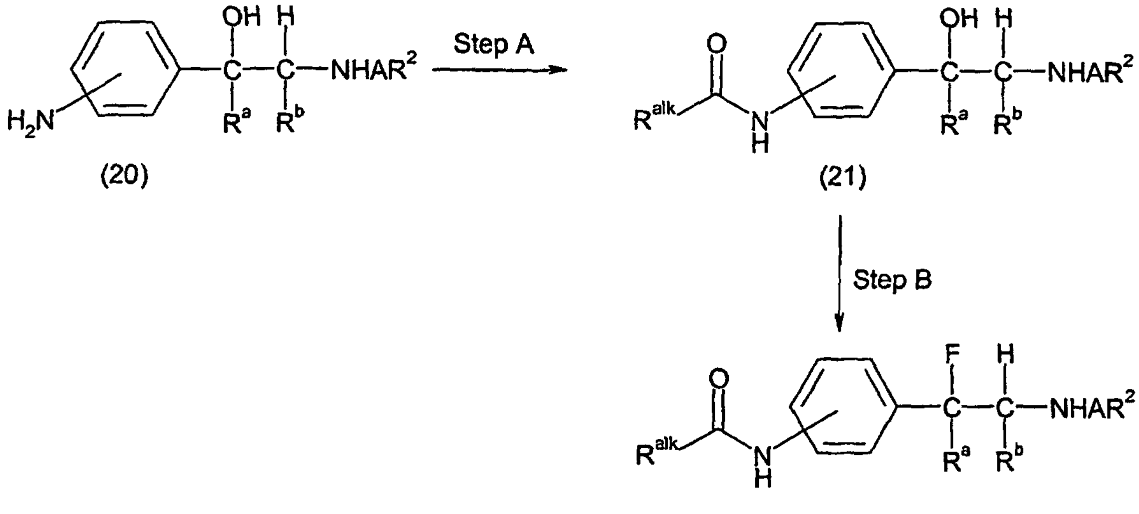


(20) (21)

formula Iq

In Scheme XII, step A, the compound of structure (20) is converted to the amide of structure (21) under standard amide coupling conditions well known in the art. For example, the compound (20) is dissolved in a suitable organic solvent, such as methylene chloride, and treated with a catalytic amount of dimethylaminopyridine (DMAP), about 1.6 equivalents of 1-[3-(dimethylamino)propyl]-3-ethylcarbodiimide, and about one equivalent of a suitable acid of formula $R^{alk}CO_2H$. $R^{alk}$ is an unsubstituted or substituted aromatic group, an unsubstituted or substituted heteroaromatic group, an unsubstituted or substituted (5-8C)cycloalkyl group, (1-10C) alkyl; (2-10C)alkenyl; or (2-10C)alkynyl. The reaction mixture is stirred at room temperature for about 4 to 64 hours and poured into water. The quenched reaction mixture is then extracted with a suitable organic solvent, such as ethyl acetate, the combined organic extracts are washed with water, dried over anhydrous sodium sulfate, filtered, and concentrated under vacuum to provide crude (21). The crude material can be purified by chromatography on silica gel with a suitable eluent, such as ethyl acetate/ hexane.

Alternatively, in Step A, compound 20 is dissolved in a suitable organic solvent, such as tetrahydrofuran with about 1.2 equivalents of triethylamine added. The solution is then treated dropwise with about one equivalent of an acid chloride of formula $R^{alk}COCl$. $R^{alk}$ is an unsubstituted or substituted aromatic group, an unsubstituted or substituted heteroaromatic group, an unsubstituted or substituted (5-8C)cycloalkyl group, (1-10C) alkyl; (2-10C)alkenyl; or (2-10C) alkynyl. The reaction mixture is stirred at room temperature for about 2 to 24 hours and poured into water. The quenched reaction mixture is then extracted with a suitable organic solvent, such as ethyl acetate, the combined organic extracts are washed with water, dried over anhydrous sodium sulfate, filtered, and concentrated under vacuum to provide crude (21). The crude material can be purified by chromatography on silica gel with a suitable eluent, such as ethyl acetate/ hexane.

In Scheme XII, step B, the compound (21) is fluorinated in a manner analogous to the procedure described in Scheme I, step B with DAST to provide the compound of formula Iq.

The ability of compounds of formula I to potentiate glutamate receptor-mediated response may be determined using fluorescent calcium indicator dyes (Molecular Probes, Eugene, Oregon, Fluo-3) and by measuring glutamate-evoked efflux of calcium into GluR4 transfected HEK293 cells, as described in more detail below.

In one test, 96 well plates containing confluent monolayers of HEK 293 cells stably expressing human GluR4B (obtained as described in European Patent Application Publication Number EP-A1-583917) are prepared. The tissue culture medium in the wells is then discarded, and the wells are each washed once with 200 µl of buffer (glucose, 10mM, sodium chloride, 138mM, magnesium chloride, 1mM, potassium chloride, 5mM, calcium chloride, 5mM, N-[2-hydroxyethyl]-piperazine-N-[2-ethanesulfonic acid], 10mM, to pH 7.1 to 7.3). The plates are then incubated for 60 minutes in the dark with 20 µM Fluo3-AM dye (obtained from Molecular Probes Inc., Eugene, Oregon) in buffer in each well. After the incubation, each well is washed once with 100 µl buffer, 200 µl of buffer is added and the plates are incubated for 30 minutes.

Solutions for use in the test are also prepared as follows. 30 µM, 10 µM, 3 µM and 1 µM dilutions of test compound are prepared using buffer from a 10 mM solution of test compound in DMSO. 100 µM cyclothiazide solution is prepared by adding 3 µl of 100 mM cyclothiazide to 3 ml of buffer. Control buffer solution is prepared by adding 1.5 µl DMSO to 498.5 µl of buffer.

Each test is then performed as follows. 200 µl of control buffer in each well is discarded and replaced with 45 µl of control buffer solution. A baseline fluorescent measurement is taken using a FLUOROSKAN II fluorimeter (Obtained from Labsystems, Needham Heights, MA, USA, a Division of Life Sciences International Plc). The buffer is then removed and replaced with 45 µl of buffer and 45 µl of test compound in buffer in appropriate wells. A second fluorescent reading is taken after 5 minutes incubation. 15 µl of 400 µM glutamate solution is then added to each well (final glutamate concentration 100 µM), and a third reading is taken. The activities of test compounds and cyclothiazide solutions are determined by subtracting the second from the third reading (fluorescence due to addition of glutamate in the presence or absence of test compound or cyclothiazide) and are expressed relative to enhance fluorescence produced by 100 µM cyclothiazide.

In another test, HEK293 cells stably expressing human GluR4 (obtained as described in European Patent Application Publication No. EP-A1-0583917) are used in the electrophysiological characterization of AMPA receptor potentiators. The extracellular recording solution contains (in mM): 140 NaCl, 5 KCl, 10 HEPES, 1 $MgCl_2$, 2 $CaCl_2$, 10 glucose, pH = 7.4 with NaOH, 295 mOsm kg-1. The intracellular recording solution contains (in mM): 140 CsCl, 1 $MgCl_2$, 10 HEPES, (N-[2-hydroxyethyl]piperazine-N1-[2-ethanesulfonic acid]) 10 EGTA (ethylene-bis(oxyethylene-nitrilo)tetraacetic acid), pH = 7.2 with CsOH, 295 mOsm kg-1. With these solutions, recording pipettes have a resistance of 2-3 MΩ. Using the whole-cell voltage clamp technique (Hamill et al.(1981)Pflügers Arch., 391: 85-100), cells are voltage-clamped at -60mV and control current responses to 1 mM glutamate are evoked. Responses to 1 mM glutamate are then determined in the presence of test compound. Compounds are deemed active in this test if, at a test concentration of 10 µM or less, they produce a greater than 10% increase in the value of the current evoked by 1 mM glutamate.

As used herein the term "suitable AMPA receptor potentiator" refers to a compound which inhibits the rapid desensitization or deactivation of AMPA receptors to glutamate. In addition, a suitable AMPA receptor potentiator producees a greater than 10% increase in the value of the current evoked by 1 mM glutamate in the above-cited test.

In order to determine the potency of test compounds, the concentration of the test compound, both in the bathing solution and co-applied with glutamate, is increased in half log units until the maximum effect was seen. Data collected in this manner are fit to the Hill equation, yielding an $EC_{50}$ value, indicative of the potency of the test compound. Reversibility of test compound activity is determined by assessing control glutamate 1 mM responses. Once the control responses to the glutamate challenge are re-established, the potentiation of these responses by 100 µM cyclothiazide is determined by its inclusion in both the bathing solution and the glutamate-containing solution. In this manner, the efficacy of the test compound relative to that of cyclothiazide can be determined.

Stroke Model

The stroke model used in this study is a model of permanent middle cerebral artery occlusion which was developed by Tamura et al, *J Cereb Blood Flow and Metab,* Vol. 1, 53-60, 1981. This model is used extensively in stroke studies and is recognized as a gold standard for testing neuroprotective agents. Tamura and coworkers described a subtemporal craniectomy approach to gain access to the proximal regions of the middle cerebral artery. After exposure, the middle cerebral artery is occluded proximal to the lateral lenticulostriate branches by electrocoagulation. The technique used herein is a slight modification of the Tamura technique which allows the zygomatic arch to remain intact and enables the animals to recover better from surgery. With this modification of the model, reproducible infarcts involving cortical and striatal regions of the brain are produced. All of the recovery of function studies are conducted using male Sprague Dawley rats from Charles River's Corporation. The rats are anaesthetized with 3% isofluorane in 70% nitrogen and 30% oxygen and anesthesia is maintained with 1-2% isofluorane. The rats are placed in the lateral position and the anterior portion of the face is held in an anesthesia mask by means of an incisor bar. Anesthesia is maintained for the duration of the procedure. Body temperature is maintained at 37°C using a rectal probe and a small Harvard homeothermic blanket system. Artificial tears from Butler Company are placed in the rat's eyes and the right eyelid is closed and covered with a small piece of elastikon porous tape to reduce the damage to the eye during surgery. The area between the right lateral canthus of the eye and the base of the right ear is shaved and cleansed with a surgical scrub of betadine and 70% alcohol and a curved vertical 2 cm skin incision is made in the midpoint between the left orbit and the external auditory canal. An incision is made around the superior and posterior margins of the temporalis muscle and the muscle is scraped from the lateral aspect of the skull and reflected forwards. With the aid of a surgical microscope, the proximal middle cerebral artery is exposed through a subtemporal craniectomy using a hand-held drill with a 1.4 mm steel burr. Then a curved Fredman-Peason micro-rongeurs is used to remove pieces of the skull without removing the zygomatic arch, orbital contents, and without transecting the facial nerve. This is a part of the procedure that differs somewhat from the procedure originally reported by Tamura et al. Their procedure removed part of the zygomatic arch, whereas the present procedure leaves it intact. After the middle cerebral artery is exposed, the dura that covers the artery is removed and the artery is occluded by micropolar coagulation from just proximal to the olfactory tract to the inferior cerebral vein. The artery is then transected with extra delicate mini-vannus scissors. A bipolar unit setting ranges between 15-20 during the coagulation period. Surgical 3/0 nylon suture is used to close

the incision site. Antibiotic ointment is topically applied to the incision site. Every rat receives 40mg/kg of cefazolin 1 day prior to surgery and the day of surgery to prevent infection. The initial success of the surgery is assessed by examining the abnormal posture of the rat after recovery from anesthesia. Abnormal posture is assessed by suspending the rats by the tail and observing twisting of the thorax and extension of forelimbs. The presence of thorax twisting and the absence of contralateral forepaw extension is the initial evidence that the surgery had been successful. In order to determine if the animals are suitable to be included in the experiment for drug testing, they are exposed to a neurological battery of tests 24h after the stroke. This neurological battery of tests was developed so that the animals could be examined 24h after the stroke before any drug testing was begun. The purpose of using this neurological battery is to eliminate animals with minimal neurological deficits and select only the animals with significant neurological deficits to be included in the drug testing experiments. This use of the neurological battery testing significantly reduces the variability in the experimental paradigms. In order to determine if a drug is causing true recovery rather than acute neuroprotection, drugs are not administered until 48h after the stroke.

The drug is typically administered intravenously at two doses. For example, one group of rats is dosed intravenously with 100 μg/kg and the other group is dosed with 3 μg/kg and a third group is a vehicle control. Intravenous injections are administered daily for 10 days beginning 48h after the stroke.

Staircase Test

The staircase test is the most difficult test for an animal to perform after middle cerebral artery occlusion. The staircase test which was originally developed by Montoya et al, *Journal of Neuroscience Methods,* Vol. 36, 219-228, 1991, is a test that measures the skilled reaching and grasping with fine motor function. The animals need to be pretrained for this test. The animals are trained to perform the task before the stroke surgery and the test is repeated at intervals after the stroke. The test requires food deprivation and the animals are given 12-15 g of regular rat chow per day. The rats are food deprived by feeding them just enough food to maintain body weight at 95% of the free-feeding body weight. The rat is placed in an apparatus with a series of steps where 2 food pellets are placed on each step. The rat can only reach the steps on the left side with its left front paw and the steps on the right side with its right front paw. The food pellets are in small wells on each step. As the height of the steps decrease it becomes more difficult for the rat to reach the food pellet on the step. A total of 14 food pellets are placed on each staircase. The rats are allowed 10 minutes each to grasp and retrieve the reward food pellets. After 10 minutes the double staircase is removed and the number of food pellets that remained on each staircase were counted. The total number of missing and displaced food pellets for both ipsilateral and contralateral forepaws between the vehicle group of animals and drug-treated group of animals are counted. The number of missing food pellets is directly related to the ability of the animal to recover fine motor function.

Sticky Tape Test

The sticky tape test is used to measure sensory neglect. Sensory neglect is a common feature of stroke patients. Sensory neglect involves neglect of one part of the body. The sticky tape test for sensory neglect was developed by Schallert and Whishaw, *Behavioral Neuroscience,* Vol. 98, 518-540, 1984. This test had been variously called the tape test or bilateral asymmetry test and is based on observations of behavior of humans with unilateral brain damage. If two stimuli are presented simultaneously, one on each side of the body, the contralateral stimulus appears to be masked and either remains undetected until the ipsilateral stimulus is removed or feels subjectively weaker. In rats, the test consists of placing adhesive tapes (Avery adhesive labels, 1 cm circles) on the distal radial region of each wrist of the forelimb. A placement of each test is randomized between the contralateral and ipsilateral forelimbs. Tape on both forepaws is touched simultaneously prior to placing the animal in a Plexiglas cage and measuring both the latency to touch and a latency to remove each stimulus from the contralateral and ipsilateral forepaws with a stopwatch. The test is terminated at 5 min. if the tapes have not already been removed. The most important detail to note is the side that is contacted first. A drug that would improve recovery of function would decrease the latency with which the animal touches the tape placed on the contralateral forepaw.

The pharmaceutical compositions are prepared by known procedures using well-known and readily available ingredients. In making the compositions of the present invention, the active ingredient will usually be mixed with a carrier, or diluted by a carrier, or enclosed within a carrier, and may be in the form of a capsule, sachet, paper, or other container. When the carrier serves as a diluent, it may be a solid, semi-solid, or liquid material which acts as a vehicle, excipient, or medium for the active ingredient. The compositions can be in the form of tablets, pills, powders, lozenges, sachets, cachets, elixirs, suspensions, emulsions, solutions, syrups, aerosols, ointments containing, for example, up to 10% by weight of active compound, soft and hard gelatin capsules, suppositories, sterile injectable solutions, and sterile packaged powders.

Some examples of suitable carriers, excipients, and diluents include lactose, dextrose, sucrose, sorbitol, mannitol, starches, gum, acacia, calcium phosphate, alginates, tragcanth, gelatin, calcium silicate, micro-crystalline cellulose, polyvinylpyrrolidone, cellulose, water syrup, methyl cellulose, methyl and propyl hydroxybenzoates, talc, magnesium stearate, and mineral oil. The formulations can additionally include lubricating agents, wetting agents, emulsifying and suspending agents, preserving agents, sweetening agents, or flavoring agents. Compositions of the invention may be formulated so as to provide quick, sustained, or delayed release of the active ingredient after administration to the patient by employing procedures well known in the art.

The compositions are preferably formulated in a unit dosage form, each dosage containing from about 1 mg to about 500 mg, more preferably about 5 mg to about 300 mg (for example 25 mg) of the active ingredient. The term "unit dosage form" refers to a physically discrete unit suitable as unitary dosages for human subjects and other mammals, each unit containing a predetermined quantity of active material calculated to produce the desired therapeutic effect, in association with a suitable pharmaceutical carrier, diluent, or excipient. The following formulation examples are illustrative only and are not intended to limit the scope of the invention in any way.

| **Formulation 1** | |
|---|---|
| Hard gelatin capsules are prepared using the following ingredients: | |
| | Quantify (mg/capsule) |
| Active Ingredient | 250 |
| Starch, dried | 200 |
| Magnesium Stearate | 10 |
| **Total** | **460** |

The above ingredients are mixed and filled into hard gelatin capsules in 460 mg quantities.

| **Formulation 2** | |
|---|---|
| Tablets each containing 60 mg of active ingredient are made as follows: | |
| | Quantify (mg/tablet) |
| Active Ingredient | 60 |
| Starch | 45 |
| Microcrystalline Cellulose | 35 |
| Polyvinylpyrrolidone | 4 |
| Sodium Carboxymethyl Starch | 4.5 |
| Magnesium Stearate | 0.5 |
| Talc | 1 |
| **Total** | **150** |

The active ingredient, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50°C and passed through a No. 18 mesh U.S. sieve. The sodium carboxymethyl starch, magnesium stearate, and talc, previously passed through a No. 60 mesh U.S. sieve, are then added to the granules which, after mixing, are compressed on a tablet machine to yield tablets each weighing 150 mg.

As used herein the term "patient" refers to a mammal, such as a mouse, guinea pig, rat, dog or human. It is understood that the preferred patient is a human.

As used herein, the terms "treating" or "to treat" each mean to alleviate symptoms, eliminate the causation either on a temporary or permanent basis, to prevent or slow the appearance of symptoms of stroke in a patient, or to enhance recovery following stroke in a patient.

As used herein, the term "effective amount" refers to the amount of a compound of formula I which is effective, upon single or multiple dose administration to a patient, in treating the patient suffering from stroke.

An effective amount can be readily determined by the attending diagnostician, as one skilled in the art, by the use of known techniques and by observing results obtained under analogous circumstances. In determining the effective amount or dose, a number of factors are considered by the attending diagnostician, including, but not limited to: the species of mammal; its size, age, and general health; the degree of or involvement or the severity of the stroke; the response of the individual patient; the particular compound administered; the mode of administration; the bioavailability characteristics of the preparation administered; the dose regimen selected; the use of concomitant medication; and other relevant circumstances.

The compounds can be administered by a variety of routes including oral, rectal, transdermal, subcutaneous, intravenous, intramuscular, bucal or intranasal routes. Alternatively, the compound may be administered by continuous infusion. A typical daily dose will contain from about 0.01 mg/kg to about 100 mg/kg of the active compound of this invention. Preferably, daily doses will be about 0.05 mg/kg to about 50 mg/kg, more preferably from about 0.1 mg/kg to about 25 mg/kg.

The following examples and preparations represent typical syntheses of the compounds of formula I as described generally above. These examples are illustrative only and are not intended to limit the invention in any way. The reagents and starting materials are readily available to one of ordinary skill in the art. As used herein, the following terms have the meanings indicated: "eq" refers to equivalents; "g" refers to grams; "mg" refers to milligrams; "L" refers to liters; "mL" refers to milliliters; "µL" refers to microliters; "mol" refers to moles; "mmol" refers to millimoles; "psi" refers to pounds per square inch; "min" refers to minutes; "h" or "hr" refers to hours; "°C" refers to degrees Celsius; "TLC" refers to thin layer chromatography; "HPLC" refers to high performance liquid chromatography; "$R_f$" refers to retention factor; "$R_t$" refers to retention time; "δ"refers to part per million down-field from tetramethylsilane; "THF" refers to tetrahydrofuran; "DMF" refers to N,N-dimethylformamide; "DMSO" refers to methyl sulfoxide; "LDA" refers to lithium diisopropylamide; "EtOAc" refers to ethyl acetate; "aq" refers to aqueous; "iPrOAc" refers to isopropyl acetate; "methyl DAST" refers to dimethylaminosulfur trifluoride, "$PdCl_2$(dppf)" refers to [1,1' bis(diphenylphosphino)-ferrocene] dichloropalladium (II); "DAST" refers to diethylaminosulfur trifluoride, "DBU" refers to 1,8-diazabicyclo[5.4.0]undec-7-ene; and "RT" refers to room temperature.

Example 1

Preparation of [2-fluoro-2-(4-iodophenyl)propyl][(methylethyl)sulfonyl]amine.

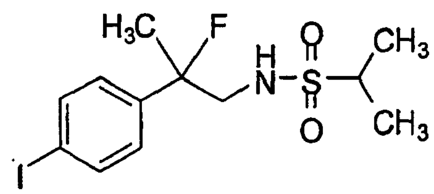

Preparation of 1-amino-2-(4-iodophenyl)propan-2-ol.

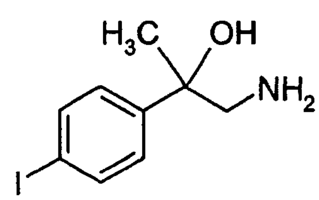

Scheme IV, Step A: The trimethylsilyl-protected cyanohydrin derivative of 4-iodoacetophenone was prepared *in situ* following generally the method disclosed by Greenlee and Hangauer, *Tetrahedron Lett.,* 24(42), 4559 (1983). Accordingly, cyanotrimethylsilane (21.4 g, 0.216 mol) was added dropwise over 5 minutes to a dry, room temperature solution containing 4-iodoacetophenone (44.3 g, 0.180 mol), 18-crown-6 (1.6 g, 6.1 mmoles) and KCN (1.17g, 0.018 mol) in THF (100 mL). The resulting solution was allowed to stir for 2.5 h. TLC analysis (3:7 EtOAc / Hexanes) showed consumption of starting acetophenone.

Scheme IV, step B: A 10M solution of borane in dimethylsulfide (25 mL, 0.25 mol) was added rapidly to the reaction solution and the resulting mixture was heated at reflux for 16 h. The mixture was cooled to room temperature

and anhydrous 10% (by wt) HCl in methanol was added slowly over 1 h (GAS EVOLUTION). The solution was allowed to stir for an additional hour, and was concentrated under reduced pressure to give the crude title compound as white solid and as the hydrochloride salt. This salt was triturated with methyl *t*-butyl ether and filtered. The free base was prepared by adding 1N NaOH to a suspension of the HCl salt in $CH_2Cl_2$ (150 mL) and THF (350 mL) until pH 12.3 was reached. The phases were separated and the organic phase was washed with brine (25 mL). The organic phase containing the free amine was concentrated under reduced pressure and the resulting solids were triturated with diethyl ether (30mL) to afford the intermediate title compound (35.6 g, 71.3%) as an off-white powder after vacuum drying. $^1H$ NMR ($CD_3OD$, 300 MHz): δ 7.68 (d, 2H, *J* = 8.4), 7.24 (d, 2H, *J* = 8.7), 2.78 (m, 2H), 1.46 (s, 3H).

Preparation of [2-hydroxy-2-(4-iodophenyl)propyl][(methylethyl)sulfonyl]amine.

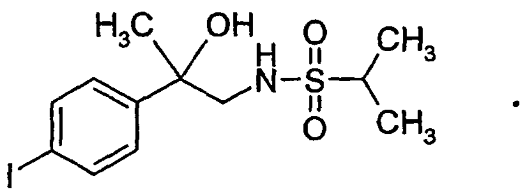

Scheme I, Step A: Into a 250 mL 3 necked flask fitted with a stirrer and thermometer, was added dropwise 2-propanesulfonyl chloride (1.60 g, 0.011 mol) to 1-amino-2-(4-iodophenyl)propan-2-ol (2.77 gm, 0.01 mol) in 125 mL $CH_2Cl_2$ while stirring at 0°C under nitrogen. The reaction was then allowed to warm to room temperature and stirred overnight at this temperature. In the morning, the mixture was poured into $H_2O$ and the layers were separated. The organic layer was washed once with $H_2O$, dried over anhydrous $Na_2SO_4$, filtered, and concentrated under reduced vacuum . The resulting semi-solid was purified via silica gel chromatography employing the Prep. LC-2000 and eluting with a solvent of Hexane/EtOAc 3:1 to provide the intermediate title compound (744 mg, 19%) as a solid material. FDMS 382 (M*).

| Analysis for $C_{12}H_{18}NO_3$ S I: | | | |
|---|---|---|---|
| Theory | C, 37.61 | H, 4.73 | N, 3.65 |
| Found | C, 38.08 | H, 4.26 | N, 3.55 |

Alternative preparation of title compound.

Scheme I, step A: In a 250 mL-3 neck flask fitted with a stirrer and thermometer, 2.10 g. of propanesulfonyl chloride was added dropwise to 2.77 g. of 1-amino-2-(4-iodophenyl)propan-2-ol and 2.30 g. of DBU in $CH_2Cl_2$ (150 mL) while stirring at 0°C under a nitrogen atmosphere. The reaction was allowed to warm to room temperature and stirred overnight at this temperature. In the morning, the reaction was diluted with $CH_2Cl_2$ (100 mL) and the organic layer was washed two times with $H_2O$, dried over anhydrous $Na_2SO_4$, filtered, and concentrated under reduced vacuum to yield a viscous oil. This material was purified via silica gel chromatography employing the Chromatotron, using a 4000 micron rotor and eluting with a solvent of methylene chloride/methanol 19:1 to yield the intermediate title compound (1.0 g, 31%) as a viscous oil. Ion spray M.S. 382 (M*-1).

Preparation of final title compound.

Scheme I, Step B: Into a 10 mL single neck flask, a solution of [2-hydroxy-2-(4-iodophenyl)propyl][(methylethyl)sulfonyl]amine (158 mg, 0.41 mmol) in 1.7 mL $CH_2Cl_2$ was added syringe wise slowly to a solution of DAST (66 mg, 0.41 mmol) in 0.3 mL $CH_2Cl_2$ while stirring at -78°C under nitrogen. The reaction was then allowed to warm to room temperature and the mixture was diluted with $H_2O$ and $CH_2Cl_2$. The layers were separated and the organic layer was washed twice with $H_2O$, dried over anhydrous $Na_2SO_4$, filtered, and concentrated under reduced vacuum to provide the final title compound (113 mg) as a solid. Ion spray M.S. 384 (M*-1).

Additional preparation of final title compound.

Scheme I, step B: Into a 100 mL 3-neck flask fitted with a stirrer and thermometer, 1.0 g. of [2-hydroxy-2-(4-iodophenyl)propyl][(methylethyl)sulfonyl]amine in $CH_2Cl_2$ (15 mL) was added dropwise to 0.3 mL DAST in $CH_2CL_2$ (10 mL) while stirring at -78°C under a nitrogen atmosphere. Reaction was allowed to warm to room temperature and diluted with $CH_2Cl_2$ (50 mL). This organic layer was washed with $H_2O$, dried over anhydrous $Na_2SO_4$, filtered, and concentrated under reduced vacuum to yield an oil. This material was purified via silica gel chromatography employing the Chromatotron and using a 4000 micron rotor while eluting with a gradient solvent of hexane/ethyl acetate 9:1 to hexane/ethyl acetate 3:1 to yield the final title compound (0.906 g) as a white solid. Ion spray M.S. 384 (M* - 1 ).

| Analysis for $C_{12}H_{17}NO_2SFI$: | | | |
|---|---|---|---|
| Theory | C, 37.42 | H, 4.44 | N, 3.64 |
| Found | C, 37.27 | H, 4.33 | N, 3.61 |

Example 1a

Preparation of (+)-[2-fluoro-2-(4-iodophenyl)propyl][(methylethyl)sulfonyl]amine and (-)-[2-fluoro-2-(4-iodophenyl)propyl][(methylethyl)sulfonyl]amine.

[2-fluoro-2-(4-iodophenyl)propyl][(methylethyl)sulfonyl]amine (2.0 g, prepared in example 1) was dissolved into 3A ethanol (30 mL) and was further diluted with heptane (20 mL). [As used herein the term "3A ethanol" refers to ethanol containing 5% methanol.] The mixture was agitated via ultrasound to form a clear, colorless solution. This lot was loaded upon a 8 x 28 cm preparative Chiralpak AD chromatographic column that was pre-equilibrated with 60% 3A ethanol/40% heptane. Eluent flow was 300 mUmin and detection wavelength was 240 nm. The first eluting substance was (+)-[2-fluoro-2-(4-iodophenyl)propyl][(methylethyl)sulfonyl]amine, $[\alpha]_D$= +18.5 (c=1.08, MeOH), and the subsequent eluting substance was (-)-[2-fluoro-2-(4-iodophenyl)propyl][(methylethyl)sulfonyl]amine, $[\alpha]_D$ = -23.5 (c=1.02, MeOH). The above procedure was repeated twice in an analogous manner with [2-fluoro-2-(4-iodophenyl)propyl](methylethyl)sulfonyl]amine (second run, 3.0 g dissolved in 50 mL 3A ethanol/heptane, 3:2 and a third run, 2.0 g dissolved in 0.8 g dissolved in 40 mL 3A ethanol/heptane, 3:2). Thus, in three runs, a total of 5.8 g of [2-fluoro-2-(4-iodophenyl)propyl)[(methylethyl)sulfonyl]amine was resolved into its component enantiomers in the following yields after concentration (in vacuo) of fractions:

(+)-[2-fluoro-2-(4-iodophenyl)propyl][(methylethyl)sulfonyl]amine (2.38 g, 41.0%);
(-)-[2-fluoro-2-(4-iodophenyl)propyl][(methylethyl)sulfonyl]amine (1.2 g, 20.7%).

Analysis conditions: 0.46 x 35 cm Chiralpak AD 60% ethanol (5% methanol)/40% Heptane; Flow: 1.0 mL/min, detection wavelength: 240 nm.

For (+)-[2-fluoro-2-(4-iodophenyl)propyl][(methylethyl)sulfonyl]amine: $R_t$=5.4 min, MS (ES+) 384 (M-1). $^1H$ NMR ($CDCl_3$, 300 MHz): δ 7.73 (d, 2H, J=8.1), 7.09 (d, 2H, J=8.4), 4.27 (t, 1H, J=6.2), 3.50 (m, 2H), 3.03 (m, 1 H), 1.69 (d, 3H, J=22), 1.30 (d, 3H, J=7), 1.27 (d, 3H. J=7).

| Analysis for $C_{12}H_{17}FINO_2S$: | | | |
|---|---|---|---|
| Theory | C 37.41, | H 4.45, | N 3.64. |
| Found | C 37.54, | H 4.43, | N 3.64. |

For (-)-[2-fluoro-2-(4-iodophenyl)propyl][(methylethyl)sulfonyl]amine: $R_t$ = 10.1 min. MS (ES+) 384 (M-1). $^1H$ NMR spectrum identical to that of (+)-[2-fluoro-2-(4-iodophenyl)propyl][(methylethyl)sulfonyl]amine.

| Analysis for $C_{12}H_{17}FINO_2S$: | | | |
|---|---|---|---|
| Theory | C 37.41, | H 4.45, | N 3.64. |
| Found | C 37.56, | H 4.43, | N 3.59. |

Example 2

Preparation of [2-fluoro-2-(4-phenylphenyl)propyl][(methylethyl)sulfonyl]amine.

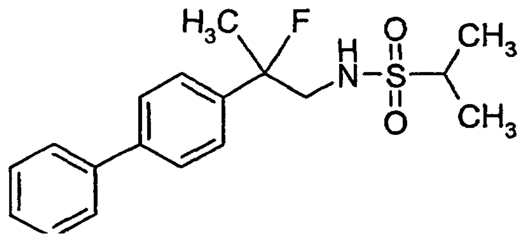

Scheme VI, Step A': Into a 10 mL single neck flask was placed [2-fluoro-2-(4-iodophenyl)propyl][(methylethyl) sulfonyl]amine (113 mg, 0.29 mmol, prepared in example 1), phenyl boric acid (54 mg, 0.44 mmol), potassium carbonate (61 mg, 0.44 mmol), and tetrakis(triphenyl phosphine)Pd(0) (17 mg, 0.02 mmol) in a mixture of dioxane/water (3:1, 7 mL). The mixture was then heated at 100 °C with stirring for 18 hours. The reaction was then cooled to room temperature and poured into $H_2O$. The mixture was extracted with EtOAc and the organic layer was washed twice with $H_2O$, dried over anhydrous $Na_2SO_4$, filtered, and concentrated under reduced vacuum. The resulting semi-solid was purified via radial chromatography (Chromatotron, Harrison Research Inc., 840 Moana Court, Palo Alto CA 94306) using a 2000 micron rotor (silica gel) and eluting with a solvent of Hexane/EtOAc 4:1 to provide the title compound (33 mg, 34%) as a slowly crystallizing tan oil. Ion spray M.S. 335 (M*+1).

| Analysis for $C_{18}H_{22}NO_2S$: | | | |
|---|---|---|---|
| Theory | C, 64.45 | H, 6.61 | N, 4.18 |
| Found | C, 65.50 | H, 6.46 | N, 4.05 |

Example 3

Preparation of 4-[4-(1-fluoro-1-methyl-2 {[(methylethy)sulfonyl]amino}ethyl)phenyl]benzenecarbonitrile.

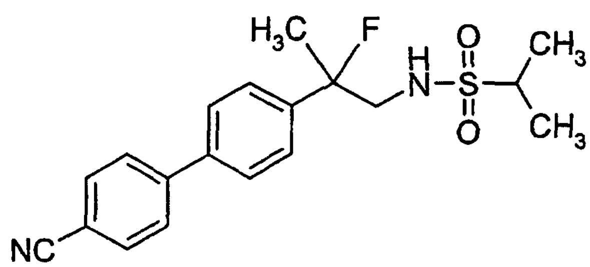

Preparation of 4-cyanobenzene boronic acid.

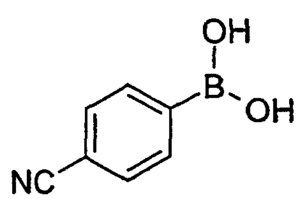

A solution of 4-bromobenzonitrile (91 g, 0.50 mole) in THF (1.1 L) was dried in the presence of activated 3 Å molecular sieves at room temp. This solution was filtered and cooled to -100°C. A 1.6 M solution of *n*-butyllithium in hexanes (355 mL. 0.567 mol) was added to the cold solution over 15 min while maintaining the internal temperature between -105 and -93°C. To the resulting orange reaction mixture was added trimethylborate (81 g, 0.78 mol) over 3 min, briefly increasing the reaction temperature to -72°C. The reaction mixture was recooled to -100°C over 5 minutes and then was allowed to warm slowly to room temperature over 2.3 h. The reaction mixture was acidified with 4N HCl to pH 2.2, and was diluted with $CH_2Cl_2$ (200 mL). The aqueous layer was separated and the organic layer was washed with brine (2 x 200 mL), dried over anhydrous $MgSO_4$, filtered, and the solvent removed under reduced pressure to a pale yellow solid. This solid was additionally purified by dissolution in 1N NaOH and extraction into $CH_2Cl_2$ / THF (1: 1, 2 x 200 mL). The aqueous phase was acidified with 4N HCl to pH 2.2 and was extracted into $CH_2Cl_2$ / THF (1:1, 500 mL). The combined organic extracts were concentrated to a crude solid (64.6 g) that was triturated with diethyl ether (160 mL) and dried under vacuum to afford the intermediate title compound (44.0 g, 59.9%) as a white powder. $^1H$ NMR ($d_6$-acetone, 300 MHz): δ 8.03 (d, 2H, *J* = 8.1), 7.75 (d, 2H, *J* = 8.4), 7.54 (s, 2H).

Preparation of 4-[4-(1-hydroxy-1-methyl-2 {[(methylethyl)sulfonyl]amino}ethyl)phenyl]benzenecarbonitrile.

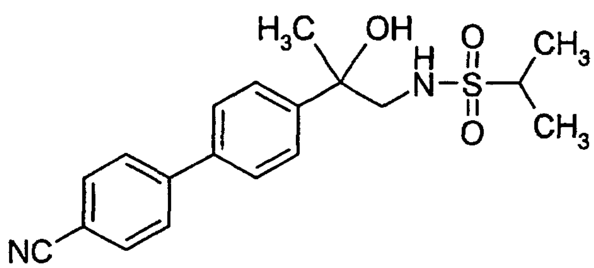

Scheme VI, Step A: Into a 50 mL single neck flask was placed 2-hydroxy-2-(4-iodophenyl)propyl][(methylethyl) sulfonyl]amine (350 mg, 0.90 mmol, intermediate prepared in example 1), 4-cyanobenzene boronic acid (206 mg, 1.40 mmol), potassium carbonate (193 mg, 1.40 mmol), and tetrakis(triphenyl phosphine)palladium(0) (52 mg, 0.045 mmol) in dioxane/water (3:1, 25 mL), and the mixture was heated at 100°C with stirring for 18 hours. The reaction was cooled to room temperature and poured into $H_2O$. The reaction mixture was extracted with EtOAc and the organic layer was separated and washed twice with $H_2O$, dried over anhydrous $Na_2SO_4$, filtered, and concentrated under reduced vacuum. The resulting semi-solid was purified via radial chromatography (Chromatotron) using a 4000 micron rotor (silica gel) and eluting with a solvent of Hexane/EtOAc 3:1 to provide the intermediate title compound (265 mg, 82%) as a solid. Ion spray M.S. 357 (M*-1).

| Analysis for $C_{19}H_{22}N_2O_3S$: | | | |
|---|---|---|---|
| Theory | C, 63.66 | H, 6.18 | N, 7.81 |
| Found | C, 63.26 | H, 6.49 | N, 7.67 |

Preparation of final title compound.

Scheme VI, Step B: Into a 25 mL single neck flask, a solution of 4-[4-(1-hydroxy-1-methyl-2-{[(methylethyl) sulfonyl]amino}ethyl)phenyl]benzenecarbonitrile (253 mg, 0.70 mmol) in 8 mL $CH_2Cl_2$ was added syringe wise slowly to a solution of DAST (114 mg, 0.71 mmol) in 2 mL $CH_2Cl_2$ while stirring at -78°C under an atmosphere of nitrogen. The reaction was then allowed to warm to room temperature and the mixture was diluted with $H_2O$ and $CH_2Cl_2$. The layers were separated and the organic layer was washed twice with $H_2O$, dried over anhydrous $Na_2SO_4$, filtered, and concentrated under reduced vacuum to provide the crude title compound as a solid. This crude material was then purified via radial chromatography using a 2000 micron rotor (silica gel) and eluting with a solvent of Hexane/EtOAc 3:1 to provide the title compound (174 mg, 69%) as a white solid. m.p. 123°-126°C Ion spray M.S. 359 (M*-1 ).

| Analysis for $C_{19}H_{21}N_2O_2SF$: | | | |
|---|---|---|---|
| Theory | C, 63.31 | H, 5.87 | N, 7.77 |
| Found | C, 62.72 | H, 5.76 | N, 7.72 |

Example 4

Preparation of 4-[4-(1-fluoro-1-methyl-2-{[(methylethyl)sulfonyl]amino}ethyl)phenyl]benzoic acid.

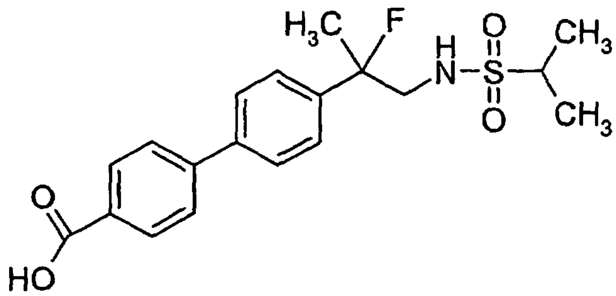

Scheme VI, Step A': Into a 50 mL single neck flask [2-fluoro-2-(4-iodophenyl)propyl][(methylethyl)sulfonyl] amine (300 mg, 0.78 mmol, prepared in example 1), 4-carboxybenzene boronic acid (188 mg, 1.13 mmol), potassium carbonate (156 mg, 1.13 mmol) and tetrakis(triphenyl phosphine)palladium(0) (52 mg, 0.05 mmol) were combined in dioxane/water (30 mL, 3:1). The mixture was then heated at 100 °C with stirring for 18 hours. The reaction was cooled to room temperature and poured into $H_2O$. The desired product was extracted with ethyl acetate and the organic layer was separated, washed twice with $H_2O$, dried over $K_2CO_3$ , and concentrated under reduced vacuum to yield the crude material (350 mg) as a tan solid. The crude material was purified via silica gel chromatography employing the Chromatotron using a 4000 micron rotor and eluting with a solvent of methylene chloride/methanol 9:1 to yield the title compound (91 mg, 31%) as a white solid.
Ion spray M.S. 378 (M*-1)

Example 4a

Preparation of 4-[4-(1-Fluoro-1-methyl-2-{[(methylethyl)sulfonyl]amino}ethyl)phenyl]benzoic acid (enantiomer 1).

(+)-[2-Fluoro-2-(4-iodophenyl)propyl][(methylethyl)sulfonyl]amine (1.00 g, 2.6 mmol, prepared in example 1a), 4-carboxybenzene boronic acid (627 mg, 3.8 mmol), potassium carbonate (520 mg, 3.8 mmol), tetrakis(triphenyl phosphine)palladium(0) (206 mg, 0.2 mmol) and dioxane/water (112 mL, 3:1) were mixed together in a 250 mL single neck flask and stirred at 80°C for 4 hours. The reaction was cooled to room temperature and poured into 1N HCl and the desired product was extracted with ethyl acetate. The organic layer was dried over $MgSO_4$, filtered, and concentrated under reduced pressure to yield 1.43 g as a dark oil. This material was purified via silica gel chromatography employing the chromatotron and using a 4000 micron rotor while eluting with a solvent of methylene chloride/methanol 9:1 to yield the title compound (355 mg, 36%) as a tan solid. Ion spray M.S. 378.3 (M*-1).

Example 4b

Preparation of 4-(4-(1-Fluoro-1-methyl-2-{[(methylethyl)sulfonyl]amino}ethyl)phenyl]benzoic acid (enantiomer 2).

(-)-[2-Fluoro-2-(4-iodophenyl)propyl][(methylethyl)sulfonyl]amine (1.00 g, 2.6 mmol, prepared in example 1a), 4-carboxybenzene boronic acid (485 mg, 2.9 mmol), $Na_2CO_3/H_2O$ (4.4 mL, excess), tetrakis(triphenyl phosphine)palladium(0) (206 mg, 0.2 mmol) and dioxane (20 mL) were mixed together in a 50 mL single neck flask and stirred at 80°C for 4 hours. The reaction was cooled to room temperature and poured into 1N HCl and the desired product was extracted with ethyl acetate. The organic layer was dried over $MgSO_4$, filtered, and concentrated under reduced pressure to yield 871 mg as a foam. This material was purified via silica gel chromatography employing the chromatotron and using a 4000 micron rotor while eluting with a solvent of methylene chloride/methanol 9:1 to yield the title compound (500 mg, 51%) as a tan solid. Ion spray M.S. 378.1 (M*-1).

| Calculated for $C_{19}H_{22}NO_4S$ F- $H_2O$ : | | | |
|---|---|---|---|
| Theory | C 57.41, | H 6.09, | N 3.52. |

(continued)

| Calculated for $C_{19}H_{22}NO_4S$ F- $H_2O$ : | | | |
|---|---|---|---|
| Found | C 57.20, | H 5.82, | N 3.52. |

Example 5

Preparation of {2-[4-(3-aminophenyl)phenyl]-2-fluoropropyl}[(methylethyl)sulfonyl]amine.

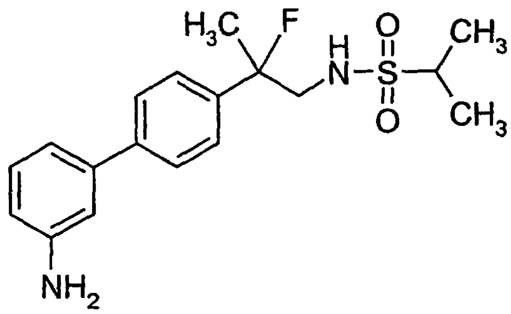

Scheme VI, Step A': Into a 50 mL single neck flask [2-fluoro-2-(4-iodophenyl)propyl][(methylethyl)sulfonyl] amine (200 mg, 0.53 mmol, prepared in example 1), 3-aminobenzene boronic acid (188 mg, 0.76 mmol), potassium carbonate (104 mg, 0.76 mmol) and tetrakis(triphenyl phosphine)palladium(0) (41 mg, 0.036 mmol) were combined in dioxane/water (20 mL, 3:1). The mixture was heated at 100 °C under stirring for 18 hours. The reaction was cooled to room temperature and poured into $H_2O$. The desired product was extracted with ethyl acetate and the organic layer was separated and washed twice with $H_2O$, dried over $K_2CO_3$ , and concentrated under reduced vacuum to yield the crude material (276 mg) as a dark oil. The resulting oil was purified via silica gel chromatography employing the Chromatotron using a 4000 micron rotor and eluting with a solvent of Hexane/Ethyl Acetate 1:1 to yield the title compound (164 mg, 90%) as a viscous oil. Ion spray M.S. 351.4 (M*+1 ).

| Analysis calculated for: $C_{18}$ $H_{23}$ $N_2$ $O_2$ S F: | | | |
|---|---|---|---|
| Theory | C, 61.69 | H, 6.62 | N, 7.99 |
| Found | C, 61.53 | H, 6.55 | N, 8.13 |

Example 6

Preparation of [2-fluoro-2-(4{3-[(methylsulfonyl)amino]phenyl}phenyl)propyl][(methylethyl)sulfonyl]amine.

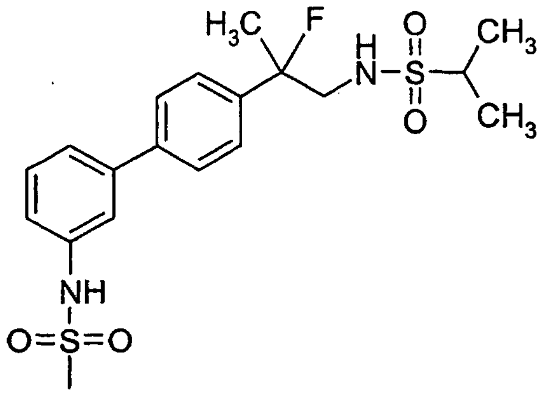

A 50 mL flask fitted with a stirrer and thermometer was charged with DBU (67 mg, 1.1 eq), {2-[4-(3-aminophenyl)phenyl]-2-fluoropropyl}[(methylethyl)sulfonyl]amine (140 mg, 0.44 mmol, prepared in example 5) and methylene chloride (10 mL) under an atmosphere of nitrogen, and cooled to 0°C. To this stirring solution was added dropwise chloro-methane sulfonyl chloride (69 mg, 1.5 eq). The reaction was allowed to warm to room temperature and stirred overnight at this temperature. In the morning, the mixture was poured into $H_2O$ and the layers were separated. The organic layer was washed once with $H_2O$, dried over anhydrous $Na_2SO_4$, filtered, and concentrated under reduced vacuum to yield the crude material (192 mg) as a yellow oil. This crude material was purified via silica gel chromatography employing the Chromatotron using a 4000 micron rotor and eluting with a solvent of Methylene Chloride/ethyl acetate 9:1 to yield the title compound (50 mg, 29%) as a white foam. Ion spray mass spectra 427.1 (M*-1).

| Analysis for $C_{19}$ $H_{25}$ $N_2$ $O_4$ $S_2$ F: | | | |
|---|---|---|---|
| Theory | C, 53.25 | H, 5.88 | N, 6.54 |
| Found | C, 53.56 | H, 6.11 | N, 6.29 |

Example 6a

Preparation of [2-Fluoro-2-(4-{3-[(methylsulfonyl)amino]phenyl}phenyl)propyl][(methylethyl)sulfonyl]amine (enantiomer 1).

(+)-[2-Fluoro-2-(4-iodophenyl)propyl][(methylethyl)sulfonyl]amine (300 mg, 0.78 mmol, prepared in example 1a), the borate of formula:

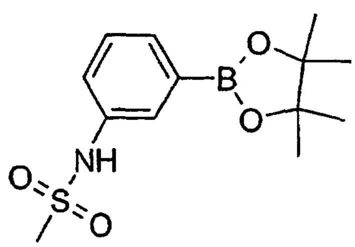

(347 mg, 1.5 eq.), potassium carbonate (156 mg, 1.5 eq), tetrakis(triphenyl phosphine)palladium(0) (75 mg, 0.06 mmol) and dioxane/water (36 mL, 3:1) were mixed together in a 100 mL single neck flask and stirred at 80°C for 4 hours. The reaction was cooled to room temperature and poured into $H_2O$ and the desired product was extracted with ethyl acetate. The organic layer was backwashed once with $H_2O$, dried over $K_2CO_3$, filtered, and concentrated under reduced pressure to yield 191 mg as a viscous oil. This material was purified via silica gel chromatography employing the chromatotron and using a 2000 micron rotor while eluting with a solvent of hexane/ethyl acetate 1:1 to yield the title compound (86 mg, 26%) as a white solid. Ion spray M.S. 427.1 (M*-1).

| Calculated for: $C_{19}H_{25}N_2O_4S_2$ F- $H_2O$ : | | | |
|---|---|---|---|
| Theory | C 51.08, | H 6.09, | N 6.27. |
| Found | C 51.29, | H 5.63, | N 6.29. |

Example 6b

Preparation of [2-Fluoro-2-(4-{3-[(methylsulfonyl)amino]phenyl}phenyl)propyl][methylethyl)sulfonyl]amine (enantiomer 2).

(-)-[2-Fluoro-2-(4-iodophenyl)propyl][(methylethyl)sulfonyl)amine (493 mg, 1.28 mmol, prepared in example 1a), the borate of formula:

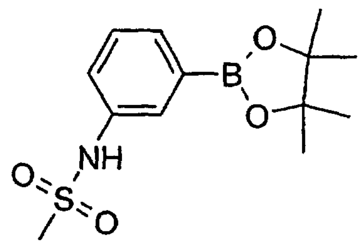

(385 mg, 1.30 mmol), 2.0 M $Na_2CO_3/H_2O$ (2.2 mL, excess), tetrakis(triphenyl phosphine)palladium(0) (100 mg, 0.09 mmol) and dioxane (15 mL) were mixed together in a 50 mL single neck flask and stirred at 80°C overnight. In the morning the reaction was cooled to room temperature and poured into $H_2O$ and the desired product was extracted with ethyl acetate. The organic layer was backwashed once with $H_2O$, dried over $K_2CO_3$, filtered, and concentrated under reduced pressure to yield 571 mg as a foam. This material was purified via silica gel chromatography employing the chromatotron and using a 4000 micron rotor while eluting with a solvent of hexane/ethyl acetate 1:1 to yield the title compound (294 mg, 56%) as a brown solid. Ion spray M.S. 427.3 (M*-1).

| Calculated for: $C_{19}H_{25}N_2O_4S_2$ F- $H_2O$: | | | |
|---|---|---|---|
| Theory | C 51.08, | H 6.09, | N 6.27. |
| Found | C 51.29, | H 5.63, | N 6.29. |

Example 7

Preparation of [2-fluoro-2-(4-(3-thienyl)phenyl)propyl][(methylethyl)sulfonyl]amine.

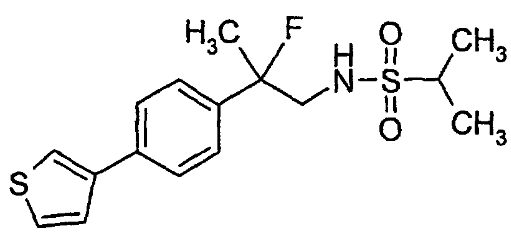

Scheme VI, Step A': Into a 50 mL single neck [2-fluoro-2-(4-iodophenyl)propyl][(methylethyl)sulfonyl]amine (150 mg, 0.39 mmol, prepared in example 1), thiophene -3-benzene boronic acid (74 mg, 0.56 mmol), potassium

carbonate (80 mg, 0.56 mmol) and tetrakis(triphenyl phosphine)palladium(0) (31 mg, 0.027 mmol) were combined in dioxane/water (15 mL, 3:1). The mixture was heated at 100 °C under stirring for 18 hours. The reaction was cooled to room temperature and poured into $H_2O$. The desired product was extracted with ethyl acetate and the organic layer was separated and washed twice with $H_2O$, dried over $K_2CO_3$, and concentrated under reduced vacuum to yield the crude material (236 mg) as a dark oil. The resulting oil was purified via silica gel chromatography employing the Chromatotron using a 4000 micron rotor and eluting with a solvent of hexane/ethyl acetate 7:3 to yield the title compound (107 mg, 81%) as a white solid. Ion spray M.S. 340.0 (M*-1).

| Analysis calculated for: $C_{16}H_{20}NO_2S_2F$: | | | |
|---|---|---|---|
| Theory | C, 56.28 | H, 5.90 | N, 4.10 |
| Found | C, 56.24 | H, 5.86 | N, 3.79. |

Example 8

Preparation of [2-fluoro-2-(4-(3-pyridyl)phenyl)propyl][(methylethyl)sulfonyl]amine.

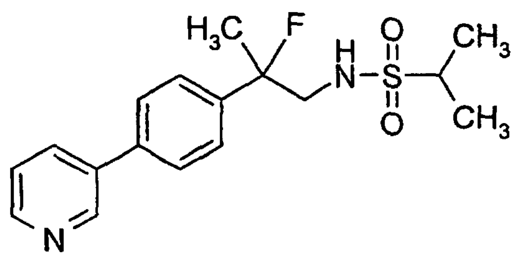

Scheme VI, Step A': [2-fluoro-2-(4-iodophenyl)propyl][(methylethyl)sulfonyl]amine (200 mg, 0.52 mmol, prepared in example 1), pyridyl boronic acid (93 mg, 0.76 mmol), potassium carbonate (104 mg, 0.76 mmol), tetrakis (triphenyl phosphine)palladium(0) (41 mg, 0.036 mmol), and dioxane/water (20 mL, 3: 1) were mixed together in a 100 mL single neck flask and stirred at 90°C over night. In the morning, the reaction was cooled to room temperature and poured into $H_2O$, and the desired product was extracted with ethyl acetate. The organic layer was backwashed once with $H_2O$, dried over $K_2CO_3$, filtered, and concentrated under reduced pressure to yield 235 mg. of a viscous oil. This material was purified via silica gel chromatography employing the Chromatotron and using a 4000 micron rotor while eluting with a solvent of methylene chloride/ethyl acetate 1:1 to yield the title compound (126 mg, 72%) as a semi-solid. Ion spray M.S. 337.2 (M* +1).

| Calculated for $C_{17}H_{21}N_2O_2SF$: | | | |
|---|---|---|---|
| Theory | C 60.69, | H 6.29, | N 8.33. |
| Found | C 60.86, | H 6.17, | N 7.99. |

Example 9

Preparation of 2-{4-[4-(1-fluoro-1-methyl-2-{[methylethyl)sulfony]amino}ethyl)phenyl]phenyl}ethanenitrile.

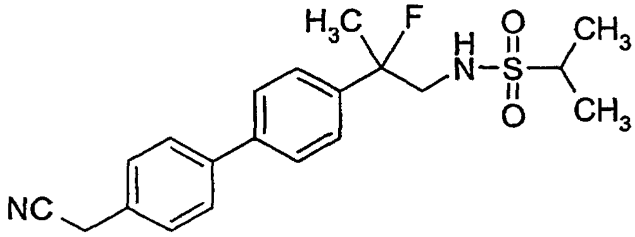

Scheme VI, step A': [2-fluoro-2-(4-iodophenyl)propyl][(methylethyl)sulfonyl]amine (500 mg, 1.3 mmol, prepared in example 1), 4-phenylacetonitrile boronic acid (193 mg, 1.4 mmol), potassium carbonate (193 mg, 1.4 mmol), tetrakis(triphenyl phosphine)palladium(0) (75 mg, 0.65 mmol), dioxane/water (30 mL, 3:1) were mixed together in a 100 mL single neck flask and stirred at 90°C over night. In the morning, the reaction was cooled to room temperature and poured into $H_2O$ and the desired product was extracted with ethyl acetate. The organic layer was backwashed once with $H_2O$, dried over $K_2CO_3$, and concentrated under reduced pressure to yield a viscous oil. This material was purified via silica gel chromatography employing the Chromatotron and using a 2000 micron rotor while eluting with a solvent of hexane/ethyl acetate 7:3 to yield the title compound (143 mg, 30%) as a yellow solid. Material was recrystallized from ethyl acetate-hexane 1:1. M.P. 155°-157°C. Ion spray M.S. 373 (M*-1).

| Calculated for $C_{20}H_{23}N_2O_2SF$: | | | |
|---|---|---|---|
| Theory | C 64.15, | H 6.19, | N 7.48. |
| Found | C 63.91, | H 5.96, | N 7.37. |

Example 10

Preparation of 4-[4-(1-fluoro-1-methyl-2-{[(methylethyl)sulfonyl]amino}ethyl)phenyl]benzaldehyde.

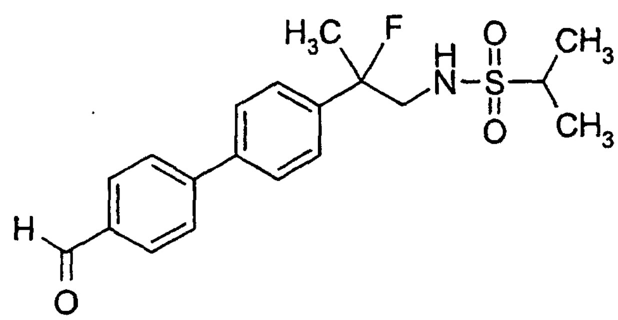

Preparation of 4-[4-(1-hydroxy-1-methyl-2-{[(methylethyl)sulfonyl]amino}ethyl)phenyl]benzaldehyde.

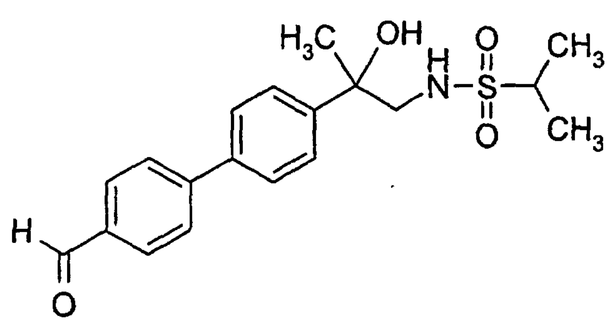

Scheme VI, step A: [2-hydroxy-2-(4-iodophenyl)propyl][(methylethyl)sulfonyl]amine (1.05 g, 2.7 mmol, intermediate prepared in example 1), 4-formylphenyl boronic acid (564 mg, 3.5 mmol), potassium carbonate (483 mg, 3.5 mmol), tetrakis(triphenyl phosphine)palladium(0) (162 mg, 1.4 mmol), and dioxane/water (60 mL, 3:1) were mixed together in a 250 mL single neck flask and stirred at 90°C over night. In the morning, the reaction was cooled to room temperature and poured into $H_2O$ and the desired product was extracted with ethyl acetate. The organic layer was backwashed once with $H_2O$, dried over $K_2CO_3$, and concentrated under reduced pressure to yield a viscous oil. This material was purified via silica gel chromatography employing the Chromatotron and using a 2000 micron rotor while eluting with a solvent of hexane/ethyl acetate 1:1 to yield the title compound as a pale yellow solid. This material was

recrystallized from ethyl acetate-hexane 1:1 to yield the intermediate title compound (519 mg) as a solid. Ion spray M. S. 360 (M* - 1).

| Calculated for $C_{19}H_{23}NO_4S$: | | | |
|---|---|---|---|
| Theory | C 63.13, | H 6.41, | N 3.87. |
| Found | C 62.94, | H 6.29, | N 3.82. |

Preparation of final title compound.

Scheme VI, step B: Into a 250, 3 neck flask fitted with a stirrer and thermometer, 4-[4-(1-hydroxy-1-methyl-2-{[(methylethyl)sulfonyl]amino}ethyl)phenyl]benzaldehyde (519 mg) in $CH_2Cl_2$ (25 mL) was added dropwise to 0.19 mL DAST in $CH_2Cl_2$ (35 mL) while stirring at -78°C under a nitrogen atmosphere. Reaction was allowed to warm to room temperature and diluted with $CH_2Cl_2$ (75 mL). This organic layer was washed with $H_2O$, dried over $Na_2SO_4$, filtered, and concentrated under reduced vacuum to yield an oil. This two spot material was purified via silica gel chromatography employing the Chromatotron and using a 2000 micron rotor while eluting with a solvent of hexane/ ethyl acetate 3:1 to provide the final title compound (0.337 g, 66%, bottom spot) as a white solid. Ion spray M.S. 362 (M* - 1).

| Calculated for $C_{19}H_{22}NO_3SF$ : | | | |
|---|---|---|---|
| Theory | C 62.79, | H 6.10, | N 3.85. |
| Found | C 65.22, | H 6.13, | N 3.21. |

Example 11

Preparation of {4-[4-(1-fluoro-1-methyl-2-{[(methylethyl)sulfonyl]amino}ethyl)phenyl]phenyl}-N-methylcarboxamide.

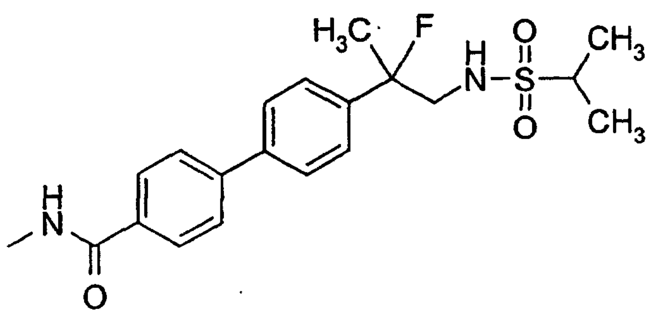

Into a 50 mL single neck flask, 1 mL oxalyl chloride was added syringe wise to 4-[4-(1-fluoro-1-methyl-2-{[(methylethyl)sulfonyl]amino}ethyl)phenyl]benzoic acid (150 mg, 0.40 mmol, prepared in example 4) in $CH_2Cl_2$ (10 mL) while stirring under nitrogen at room temperature. Immediately, 1 drop of DMF was added by pipette initiating a foaming of the mixture. The reaction was stirred one hour at this temperature and then concentrated under reduced vacuum to yield a white semi-solid. This material was placed into THF (10 mL) and added dropwise to a stirring solution of 40% methylamine in water (5 mL) at room temperature and the mixture was stirred overnight. In the morning, the solution was concentrated under reduced vacuum and the resulting oil was taken into $CH_2Cl_2$ and the organic layer was washed once with $H_2O$, dried over $K_2CO_3$, and concentrated under reduced vacuum to yield 159 mg as a semi-solid. This material was purified via silica gel chromatography employing the Chromatotron and using a 2000 micron rotor while eluting with a solvent of methylene chloride/ethyl acetate 1:1 to yield the title compound (51 mg, 32%) as a white solid. Ion spray M.S. 393.1 (M* + 1).

| Calculated for $C_{20}H_{25}N_2O_3SF$: | | | |
|---|---|---|---|
| Theory | C 61.21, | H 6.42, | N 7.14. |
| Found | C 59.92, | H 5.86, | N 6.84. |

Example 11a

Preparation of {4-[4-(1-Fluoro-1-methyl-2-{[(methylethyl)sulfonyl]amino}ethyl)phenyl]phenyl}-N-methylcarboxamide (enantiomer 1).

Into a 50 mL single neck flask, 1 mL oxalyl chloride was added syringe wise to (+)4-[4-(1-fluoro-1-methyl-2-{[(methylethyl)sulfonyl]amino}ethyl)phenyl]benzoic acid (300 mg, 0.79 mmol, prepared in example 1a) in $CH_2Cl_2$ (20 mL) while stirring under nitrogen at room temperature. Immediately, 1 drop of DMF was added by pipette initiating a foaming of the mixture. The reaction was stirred one hour at this temperature and then concentrated under reduced vacuum to yield a white semi-solid. This material was placed into dioxane (20 mL) and added dropwise to a stirring solution of 40% methylamine (5 mL) at room temperature and the mixture was stirred overnight. In the morning, the solution was concentrated under reduced vacuum and the resulting oil was taken into methylene chloride and the organic layer was washed once with $H_2O$, dried over $K_2CO_3$, filtered, and concentrated under reduced vacuum to yield 271 mg as a semi-solid. This material was purified via silica gel chromatography employing the Chromatotron and using a 2000 micron rotor while eluting with a solvent of methylene chloride/ethyl acetate 1:1 to yield the title compound (100 mg, 32%) as a white solid. Ion spray M.S. 391.2 (M*-1).

| Calculated for $C_{20}H_{25}N_2O_3SF$-1/2 $H_2O$: | | | |
|---|---|---|---|
| Theory | C 59.82, | H 6.52, | N 6.98. |
| Found | C 59.69, | H 6.29, | N 6.81. |

Example 11b

Preparation of {4-[4-(1-Fluoro-1-methyl-2-{[(methylethyl)sulfonyl]amino}ethyl)phenyl]phenyl}-N-methylcarboxamide (enantiomer 2).

Into a 50 mL single neck flask, 1 mL oxalyl chloride was added syringe wise to (-)-4-[4-(1-fluoro-1-methyl-2-{[(methylethyl)sulfonyl]amino}ethyl)phenyl]benzoic acid (300 mg, 0.79 mmol, prepared in example 1a) in methylene chloride (15 mL) while stirring under nitrogen at room temperature. immediately, 1 drop of DMF was added by pipette initiating a foaming of the mixture. The reaction was stirred one hour at this temperature and then concentrated under reduced vacuum to yield a white semi-solid. This material was placed into dioxane (20 mL) and added dropwise to a stirring solution of 40% methylamine (5 mL) at room temperature and the mixture was stirred overnight. In the morning, the solution was concentrated under reduced vacuum and the resulting oil was taken into methylene chloride and the organic layer was washed once with $H_2O$, dried over $K_2CO_3$, filtered, and concentrated under reduced vacuum to yield 391 mg as a solid. This material was purified via recrystallization from hexane/ethyl acetate 1:1 to yield the title compound (231 mg, 49%) as a white solid. Ion spray M.S. 391.4 (M*-1).

| Calculated for $C_{20}H_{25}N_2O_3SF$-1/2 $H_2O$: | | | |
|---|---|---|---|
| Theory | C 59.82, | H 6.52, | N 6.98. |
| Found | C 59.78, | H 6.53, | N 6.89. |

Example 12

Preparation of {4-[4-(1-fluoro-1-methyl-2-{[(methylethyl)sulfonyl]amino}ethyl)phenyl]phenyl}-N,N-dimethylcarboxamide.

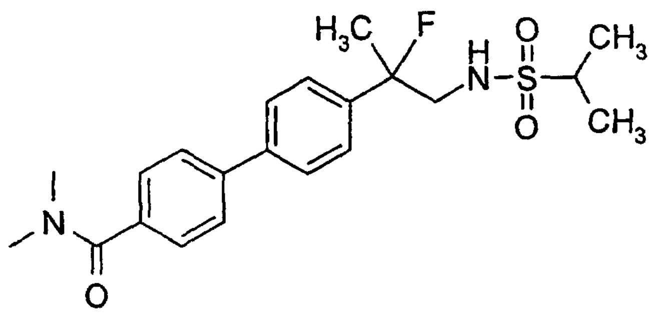

Into a 50 mL single neck flask, 1 mL oxalyl chloride was added syringe wise to 4-[4-(1-fluoro-1-methyl-2-{[(methylethyl)sulfonyl]amino}ethyl)phenyl]benzoic acid (150 mg, 0.40 mmol, prepared in example 4) in $CH_2Cl_2$ (10 mL) while stirring under nitrogen at room temperature. Immediately, 1 drop of DMF was added by pipette initiating a foaming of the mixture. The reaction was stirred one hour at this temperature and then concentrated under reduced vacuum to yield a white semi-solid. This material was placed into THF (10 mL) and added dropwise to a stirring solution of 40% dimethylamine in water (5mL) at room temperature and the mixture was stirred overnight. In the morning, the solution was concentrated under reduced vacuum and the resulting oil was taken into $CH_2Cl_2$ and the organic layer was washed once with $H_2O$, dried over $K_2CO_3$, and concentrated under reduced vacuum to yield 167 mg as an oil. This material was purified via silica gel chromatography employing the Chromatotron and using a 2000 micron rotor while eluting with a solvent of methylene chloride/ethyl acetate 1:1 to yield the title compound (97 mg, 60%) as a viscous oil. Ion spray M.S. 407.4 (M*+1).

| Calculated for $C_{21}H_{27}N_2O_3SF$: | | | |
|---|---|---|---|
| Theory | C 62.05, | H 6.70, | N 6.89. |
| Found | C 61.32, | H 6.69, | N 6.85. |

Example 13

Preparation of N-ethyl{4-[4-(1-fluoro-1-methyl-2-{[(methylethyl)sulfonyl]amino}ethyl)phenyl]phenyl}carboxamide.

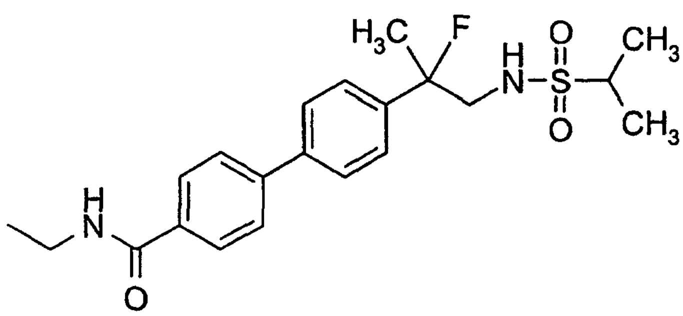

Into a 50 mL single neck flask, 1 mL oxalyl chloride was added syringe wise to 4-[4-(1-fluoro-1-methyl-2-{[(methylethyl)sulfonyl]amino}ethyl)phenyl]benzoic acid (140 mg, 0.37 mmol, prepared in example 4) in $CH_2Cl_2$ (10 mL) while stirring under nitrogen at room temperature. Immediately, 1 drop of DMF was added by pipette initiating a foaming of the mixture. The reaction was stirred one hour at this temperature and then concentrated under reduced vacuum to yield a white semi-solid. This material was placed into THF (10 mL) and added dropwise to a stirring solution of 2.0

M ethylamine in THF (0.5mL) at room temperature and the mixture was stirred overnight. In the morning, the solution was concentrated under reduced vacuum and the resulting oil was taken into $CH_2Cl_2$ and the organic layer was washed once with $H_2O$, dried over $K_2CO_3$, and concentrated under reduced vacuum to yield 151 mg as an oil. This material was purified via silica gel chromatography employing the Chromatotron and using a 2000 micron rotor while eluting with a solvent of methylene chloride/ethyl acetate 1:1 to yield the title compound (127 mg, 85%) as a viscous oil. Ion spray M.S. 407.4 (M* +1).

Example 14

Preparation of 4-[4-(1-fluoro-1-methyl-2-{[(methylethyl)sulfonyl]amino}ethyl)phenyl]phenyl pyrrolidinyl ketone.

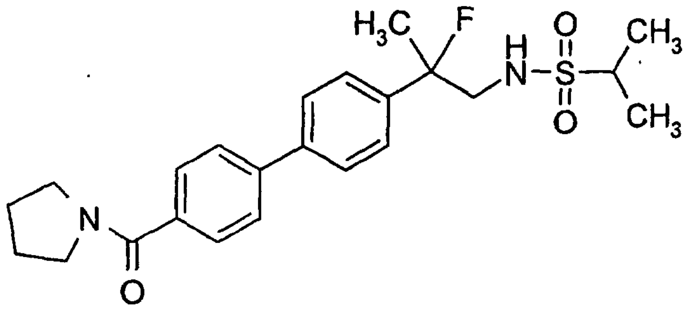

Into a 50 mL single neck flask, 1 mL oxalyl chloride was added syringe wise to 4-[4-(1-fluoro-1-methyl-2-{[(methylethyl)sulfonyl]amino}ethyl)phenyl]benzoic acid (200 mg, 0.53 mmol, prepared in example 4) in $CH_2Cl_2$ (15 mL) while stirring under nitrogen at room temperature. Immediately, 1 drop of DMF was added by pipette initiating a foaming of the mixture. The reaction was stirred one hour at this temperature and then concentrated under reduced vacuum to yield a white semi-solid. This material was placed into THF (10 mL) and added dropwise to a stirring solution of 94 mg pyrolline in THF (10mL) at room temperature and the mixture was stirred overnight. In the moming, the solution was concentrated under reduced vacuum and the resulting oil was taken into $CH_2Cl_2$ and the organic layer was washed once with $H_2O$, dried over $K_2CO_3$, and concentrated under reduced vacuum to yield 271 mg as an oil. This material was purified via silica gel chromatography employing the Chromatotron and using a 4000 micron rotor while eluting with a solvent of hexane/ethyl acetate 7:3 to yield the title compound (141 mg, 62%) as a viscous oil. Ion spray M.S. 433.3 (M* + 1 ).

| Calculated for $C_{23}H_{29}N_2O_3SF$: | | | |
|---|---|---|---|
| Theory | C 63.86, | H 6.76, | N 6.48. |
| Found | C 62.93, | H 6.40, | N 5.95. |

Example 15

Preparation of N-{3-[4-(1-fluoro-1-methyl-2-{[(methylethyl)sulfonyl]amino}ethyl)phenyl]phenyl}acetamide.

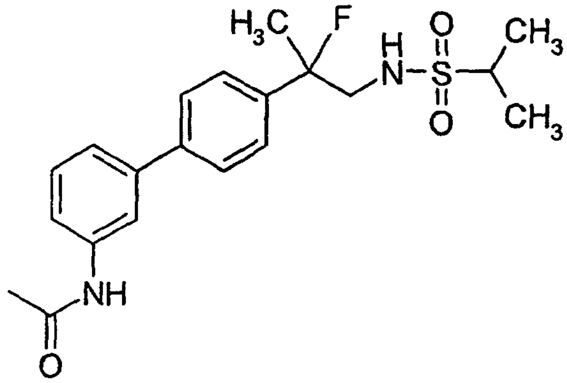

Into a 100 mL single-neck flask, 49 mg of acetyl chloride was added dropwise to 200 mg of {2-[4-(3-aminophenyl)phenyl]-2-fluoropropyl}[(methylethyl)sulfonyl]amine (prepared in example 5) and 63 mg of triethylamine in THF (25 mL) while stirring under a nitrogen atmosphere at room temperature. The reaction was allowed to stir at this temperature for 2 h. The mixture was then poured into $H_2O$ and the desired product was extracted into ethyl acetate. The organic layer was backwashed once with $H_2O$, dried over $K_2CO_3$, and concentrated under reduced vacuum to yield 193 mg as a foam. This material was purified via silica gel chromatography employing the Chromatotron, using a 4000 micron rotor and eluting with a solvent of hexane/ethyl acetate 1:1 to yield the title compound (121 mg, 54%) as a white foam. Ion spray M.S. 391.2 (M*-1).

| Calculated for: $C_{20}H_{25}N_2O_3SF$: | | | |
|---|---|---|---|
| Theory | C 61.20, | H 6.42, | N 7.13. |
| Found | C 60.28, | H 6.40, | N 6.76. |

Example 16

Preparation of N-{3-[4-(1-fluoro-1-methyl-2-{[(methylethyl)sulfonyl]amino}ethyl)phenyl]phenyl}propanamide.

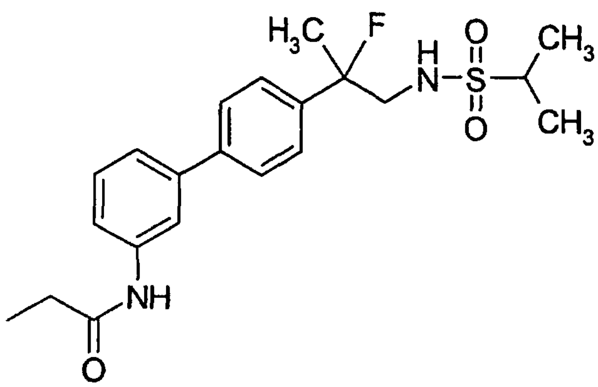

Into a 100 mL single-neck flask, 58 mg of propynyl chloride was added dropwise to 200 mg of {2-[4-(3-aminophenyl)phenyl]-2-fluoropropyl}[(methylethyl)sulfonyl]amine (prepared in example 5) and 63 mg of triethylamine in THF (25 mL) while stirring under a nitrogen atmosphere at room temperature. The reaction was allowed to stir at this temperature for 2 h. The mixture was then poured into $H_2O$ and the desired product was extracted into ethyl acetate.

The organic layer was backwashed once with $H_2O$, dried over $K_2CO_3$, and concentrated under reduced vacuum to yield 256 mg as a foam. This material was purified via silica gel chromatography employing the Chromatotron, using a 4000 micron rotor and eluting with a solvent of hexane/ethyl acetate 1:1 to yield the title compound (130 mg, 56%) as a white foam. Ion spray M.S. 405.5 (M*-1).

| Calculated for: $C_{21}H_{27}N_2O_3SF$ -1/2$H_2O$: | | | |
|---|---|---|---|
| Theory | C 60.69, | H 6.79, | N 6.74. |
| Found | C 60.95, | H 6.49, | N 6.77. |

Example 17

Preparation of N-{3-[4-(1-fluoro-1-methyl-2-{[(methylethyl)sulfonyl]amino}ethyl)phenyl]phenyl}butanamide.

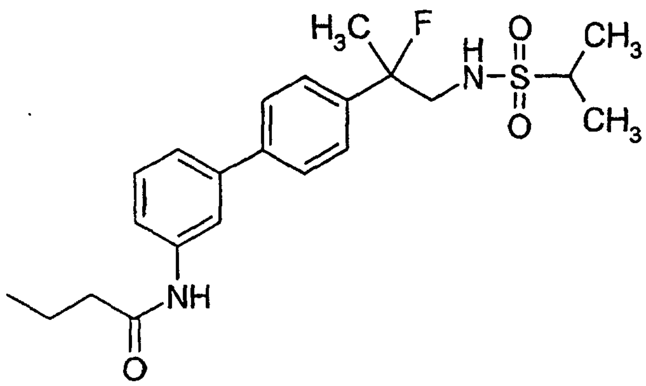

Into a 100 mL single-neck flask, 71 mg of butyryl chloride was added dropwise to 200 mg {2-[4-(3-aminophenyl) phenyl]-2-fluoropropyl}[(methylethyl)sulfonyl]amine (prepared in example 5) and 63 mg of triethylamine in THF (25 mL) while stirring under a nitrogen atmosphere at room temperature. The reaction was allowed to stir at this temperature for 2 h. The mixture was then poured into $H_2O$ and the desired product was extracted into ethyl acetate. The organic layer was backwashed once with $H_2O$, dried over $K_2CO_3$, and concentrated under reduced vacuum to yield 211 mg as an oil. This material was purified via silica gel chromatography employing the Chromatotron, using a 4000 micron rotor and eluting with a solvent of hexane/ethyl acetate 1:1 to yield the title compound (130 mg, 54%) as a white foam. Ion spray M.S. 419.2 (M*-1).

| Calculated for: $C_{22}H_{29}N_2O_3SF$: | | | |
|---|---|---|---|
| Theory | C 62.83, | H 6.95, | N 6.66. |
| Found | C 62.49, | H 6.84, | N 6.60. |

Example 18

Preparation of amino-N-{3-[4-(1-fluoro-1-methyl-2-{[(methylethyl)sulfonyl]amino}ethyl)phenyl]phenyl}amide.

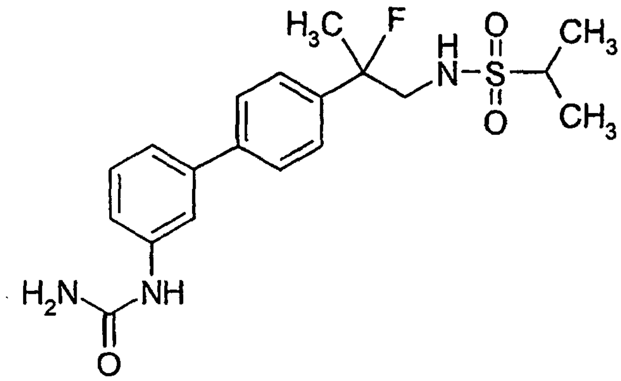

Into a 100-single neck flask, 0.1 mL of TFA in toluene (5 mL) was added dropwise to 200 mg {2-[4-(3-aminophenyl)phenyl]-2-fluoropropyl}[(methylethyl)sulfonyl]amine (prepared in example 5) and 56 mg of sodium cyanate in toluene (15 mL) while stirring under a nitrogen atmosphere at 45°-50°C. The reaction was then heated to 80°C for 1 h. The solution was cooled to room temperature and concentrated under reduced vacuum to yield a solid. This TFA salt was liberated with 1N NaOH and the desired product was extracted into methylene chloride. The organic layer was backwashed once with $H_2O$, dried over $K_2CO_3$, and concentrated under reduced vacuum to yield 168 mg as a foam. This material was purified via silica gel chromatography employing the Chromatotron, using a 4000 micron rotor and eluting with a solvent of hexane/ethyl acetate 1:9 to yield the title compound (77 mg, 34%) as a yellow foam. Ion spray M.S. 392.2 (M*-1).

| Calculated for: $C_{19}H_{24}N_3O_3SF-H_2O$: | | | |
|---|---|---|---|
| Theory | C 55.45, | H 6.37, | N 10.21. |
| Found | C 55.82, | H 6.02, | N 9.91. |

Example 19

Preparation of N-[4-(1-fluoro-1-methyl-2-{[methylethyl)sulfonyl]amino}ethyl)phenyl]benzamide.

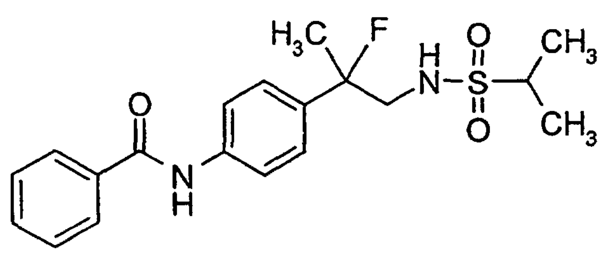

Preparation of [2-hydroxy-2-(4-nitrophenyl)propyl][(methylethyl)sulfonyl]amine.

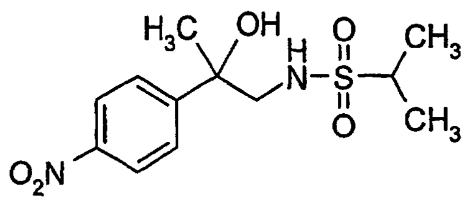

In a 500 mL, 3 neck flask fitted with a stirrer and thermometer, 2.98 g. of propanesulfonyl chloride was added dropwise to 3.92 g. of 2-hydroxy-2-(4-nitrophenyl)propylamine and 3.19 g. of DBU in $CH_2Cl_2$ (200 mL) while stirring at 0°C under a nitrogen atmosphere. The reaction was allowed to warm to room temperature and stirred overnight at this temperature. In the morning, reaction was diluted with $CH_2Cl_2$ (100 mL) and the organic layer was washed two times with $H_2O$ dried over $Na_2SO_4$, and concentrated under reduced vacuum to yield a viscous oil. This material was purified via silica gel chromatography employing the Water's Prep 2000 and eluting with a solvent of hexane/ethyl acetate 1: 1 to yield the intermediate title compound (940 mg, 16%) as a viscous oil. Ion spray M.S. 302.1 (M*- 1).

Preparation of [2-4-aminophenyl)-2-hydroxypropyl][(methylethyl)sulfonyl]amine.

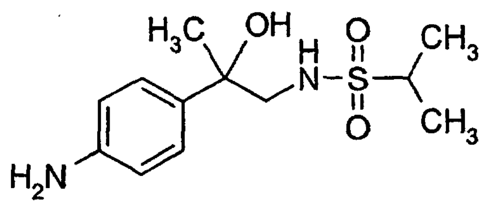

900 mg of [2-hydroxy-2-(4-nitrophenyl)propyl][(methylethyl)sulfonyl]amine and 1.2 equivalence of 10% Pd/C in ethyl acetate (50 mL) were subjected to a hydrogen atmosphere while shaking at 60 psi for 3 hours. Solution was filtered through a Celite® mat and the resulting filtrate was concentrated under reduced vacuum to yield 720 mg as an oil. This material was purified via silica gel chromatography employing the Chromatotron and using a 4000 micron rotor while eluting with a solvent of hexane/ethyl acetate 1:1 to yield the intermediate title compound (340 mg, 42%) as a tan solid. Ion spray M.S. 271.4 (M* - 1).

| Calculated for $C_{12}H_{20}N_2O_3S$: | | | |
|---|---|---|---|
| Theory | C 52.92, | H 7.40, | N 10.29 |
| Found | C 53.26, | H 7.40, | N 10.11 |

Preparation of N-[4-(1-hydroxy-1-methyl-2-{[(methylethyl)sulfonyl]amino}ethyl)phenyl]benzamide.

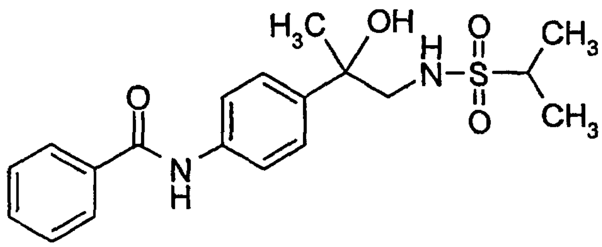

Scheme XII, Step A: Into a 100 mL single-neck flask, 77 mg of benzoyl chloride was added dropwise to 135 mg of [2-(4-aminophenyl)-2-hydroxypropyl][(methylethyl)sulfonyl]amine and 56 mg of triethylamine in THF (20 mL) while stirring under a nitrogen atmosphere at room temperature. The reaction was allowed to stir at this temperature for 2 h. The mixture was then poured into $H_2O$ and the desired product was extracted into ethyl acetate. The organic layer was backwashed once with $H_2O$, dried over $K_2CO_3$, and concentrated under reduced vacuum to yield 233 mg as a solid. This material was purified via silica gel chromatography employing the Chromatotron, using a 2000 micron rotor and eluting with a solvent of ethyl acetate to yield the intermediate title compound (151 mg, 80%) as a white solid. Ion spray M.S. 375.2 (M*-1).

Preparation of final title compound.

Scheme XII, Step B: Into a 50 mL 3 neck flask fitted with a stirrer and thermometer, 130 mg of N-[4-(1-hydroxy-1-methyl-2-{[(methylethyl)sulfonyl]amino}ethyl)phenyl]benzamide in methylene chloride (5 mL) was added dropwise to 62 mg of DAST in methylene chloride (5 mL) while stirring at -78°C under a nitrogen atmosphere. Reaction was allowed to warm to room temperature and diluted with methylene chloride (25 mL). This organic layer was washed with $H_2O$, dried over $Na_2SO_4$, and concentrated under reduced vacuum to yield 136 mg as a white foam.. This material was purified via silica gel chromatography employing the Chromatotron and using a 2000 micron rotor while eluting with a solvent of hexane/ethyl acetate 1:1 to yield the final title compound (79 mg, 60%) as a white solid. Yield = 60%. Ion spray M.S. 377.4 (M*-1).

| Calculated for $C_{19}H_{23}N_2O_3SF$ : | | | |
|---|---|---|---|
| Theory | C 60.30, | H 6.13, | N 7.31. |
| Found | C 60.20, | H 6.05, | N 7.30. |

Example 20

Preparation of (3-cyanophenyl)-N-[4-(1-fluoro-1-methyl-2-{[(methylethyl)sulfonyl]amino}ethyl)phenyl]carboxamide.

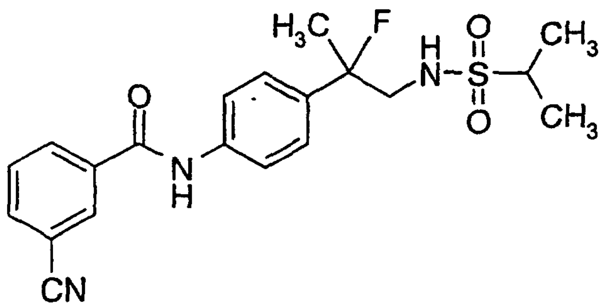

Preparation of (3-cyanophenyl)-N-[4-(1-hydroxy-1-methyl-2-{[(methylethyl)sulfonyl]amino}ethyl)phenyl]carboxamide.

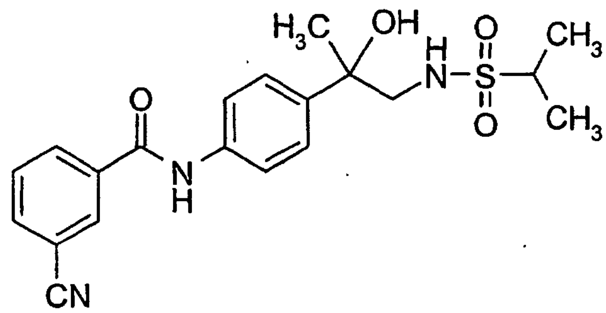

Scheme XII, Step A: Into a 100 mL single-neck flask, 91 mg of 3-cyanobenzoyl chloride was added dropwise to 135 mg of [2-(4-aminophenyl)-2-hydroxypropyl][(methylethyl)sulfonyl]amine (prepared in example 19) and 56 mg of triethylamine in THF (20 mL) while stirring under a nitrogen atmosphere at room temperature. The reaction was allowed to stir at this temperature for 2 h. The mixture was then poured into $H_2O$ and the desired product was extracted into ethyl acetate. The organic layer was backwashed once with $H_2O$, dried over $K_2CO_3$, and concentrated under reduced vacuum to yield 254 mg as a solid. This material was purified via silica gel chromatography employing the Chromatotron, using a 2000 micron rotor and eluting with a solvent of ethyl acetate to yield the intermediate title compound (131 mg, 66%) as an oil. Ion spray M.S. 400.1 (M*-1).

| Calculated for: $C_{20}H_{23}N_3O_4S$: | | | |
|---|---|---|---|
| Theory | C 59.83, | H 5.77, | N 10.47. |
| Found | C 61.33, | H 5.64, | N 10.19. |

Preparation of final title compound.

Scheme XII, Step B: Into a 50 mL 3 neck flask fitted with a stirrer and thermometer, 110 mg of (3-cyanophenyl) -N-[4-(1-hydroxy-1-methyl-2-{[(methylethyl)sulfonyl]amino}ethyl)phenyl]carboxamide in methylene chloride (5 mL) was added dropwise to 48 mg of DAST in methylene chloride (5 mL) while stirring at -78°C under a nitrogen atmosphere. Reaction was allowed to warm to room temperature and diluted with methylene chloride (25 mL). This organic layer was washed with $H_2O$, dried over $Na_2SO_4$, and concentrated under reduced vacuum to yield 110 mg as a tan solid. This material was purified via silica gel chromatography employing the Chromatotron and using a 2000 micron rotor while eluting with a solvent of hexane/ethyl acetate 1:1 to yield the final title compound (62 mg, 62%) as a yellow solid. Ion spray M.S. 402.2 (M* - 1).

| Calculated for $C_{20}H_{22}N_3O_3SF$ : | | | |
|---|---|---|---|
| Theory | C 59.54, | H 5.49, | N 10.41. |
| Found | C 58.74 | H 5.29, | N 10.03. |

Example 21

Preparation of N-[4-(1-fluoro-1-methyl-2-{[(methylethyl)sulfonyl]amino}ethyl)phenyl]-4-pyridylcarboxamide.

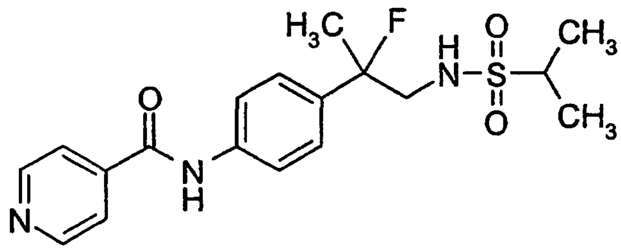

Preparation of N-[4-(1-hydroxy-1-methyl-2-{[(methylethyl)sulfonyl]amino}ethyl)phenyl]-4-pyridylcarboxamide.

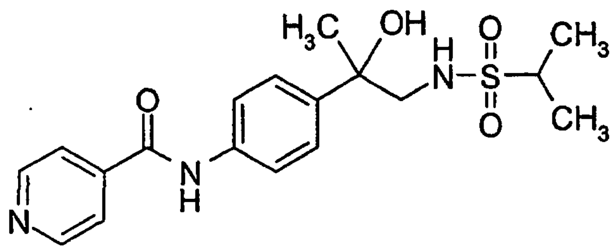

Scheme XII, Step A: [2-(4-aminophenyl)-2-hydroxypropyl][(methylethyl)sulfonyl]amine (350 mg, 1.3 mmol, prepared in example 19), isonicotinic acid (176 mg, 1 mmol), triethylamine (333 mg, 3.3 mmol), 4-dimethylamino pyridine (12.2 mg, DMAP), and 1-[3-(dimethylamino)propyl]-3-ethylcarbodiimide HCl (298 mg, 1.6 mmol, DCC) were mixed together with methylene chloride (15 mL) in a 50 mL single neck flask and stirred under a nitrogen atmosphere at room temperature for 64 hours. The solution was then poured into $H_2O$, and the desired product was extracted with ethyl acetate. The organic layer was backwashed once with $H_2O$, dried over $K_2CO_3$, and concentrated under reduced vacuum to yield 471 mg as an oil. This material was purified via silica gel chromatography employing the Chromatotron, using a 4000 micron rotor and eluting with a solvent of methylene chloride/methanol 19:1 to yield the intermediate title compound (131 mg, 27%) as an oil. Ion spray M.S. 378.5 (M*+1).

| Calculated for: $C_{18}H_{23}N_3O_4S$: | | | |
|---|---|---|---|
| Theory | C 57.27, | H 6.14, | N 11.13. |
| Found | C 57.75, | H 5.97, | N 11.05. |

Preparation of final title compound.

Scheme XII, Step B: Into a 50 mL 3 neck flask fitted with a stirrer and thermometer, 80 mg of N-[4-(1-hydroxy-1-methyl-2-{[(methylethyl)sulfonyl]amino}ethyl)phenyl]-4-pyridylcarboxamide in methylene chloride (5 mL) was added dropwise to 0.1 mL of DAST in methylene chloride (5 mL) while stirring at -78°C under a nitrogen atmosphere. Reaction was allowed to warm to room temperature and diluted with methylene chloride (25 mL). This organic layer was washed with $H_2O$, dried over $Na_2SO_4$, and concentrated under reduced vacuum to yield 62.2 mg as a yellow solid. This material was purified via silica gel chromatography employing the Chromatotron and using a 1000 micron rotor while eluting with a solvent of ethyl acetate to yield the final title compound (32 mg, 40%) as a pale yellow solid. Ion spray M.S. 380.4 (M* + 1).

| Calculated for $C_{18}H_{22}N_3O_3SF$-1/2 $H_2O$ : | | | |
|---|---|---|---|
| Theory | C 55.56, | H 5.96, | N 10.81. |
| Found | C 55.40, | H 5.73, | N 10.41. |

Example 22

Preparation of N-[4-(1-fluoro-1-methyl-2-{[(methylethyl)sulfonyl]amino}ethyl)phenyl]{4-[2-(methoxycarbonylamino) ethyl]phenyl}carboxamide.

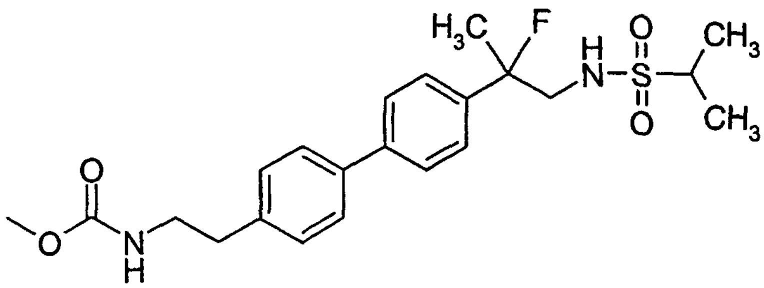

Preparation of N-[2-(4-bromophenyl)ethyl]methoxycarboxamide.

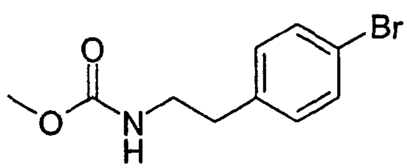

Into a 50 mL-single neck flask, 260 mg of methyl chloroformate in acetone (5mL) was added dropwise to 500 mg of 4-bromophenethyl amine and 291 mg of $Na_2CO_3$ in acetone (20 mL) while stirring at room temperature under a nitrogen atmosphere. The reaction was then stirred overnight at this temperature. In the morning, the mixture was poured into $H_2O$ and the desired product was extracted with ethyl acetate. This organic layer was washed with $H_2O$, dried over $Na_2SO_4$, and concentrated under reduced vacuum to yield the intermediate title compound (714 mg) as a white solid. This material was taken to the next reaction without further purification. Ion spray M.S. 259.2 (M*+1).

Preparation of final title compound.

Into a 100 mL- single neck flask, 285 mg of [2-fluoro-2-(4-iodophenyl)propyl][(methylethyl)sulfonyl]amine (prepared in example 1), 200 mg of 4,4,5,5-tetramethyl-1,2,3-dioxaborolane (Dibron), and 215 mg of potassium acetate in DMF (15 mL) was stirred and degassed with argon for 10 minutes. Then, 15 mg of $PdCl_2$(dppf) was added portion wise and the reaction was stirred at 80°C under a nitrogen atmosphere for 2 hours. The reaction was then cooled to room temperature and 3.40 mg of N-[2-(4-bromophenyl)ethyl]methoxycarboxamide, 1.80 mL $Na_2SO_3/H_2O$, and an additional 15 mg of $PdCl_2$(dppf) was added all portion wise and the mixture was then heated to 80°C and stirred overnight. In the morning, the mixture was cooled to room temperature and poured into $H_2O$ and the desired product was extracted with ethyl acetate. This organic layer was washed with $H_2O$, dried over $Na_2SO_4$, and concentrated under reduced vacuum to yield 616 mg of a dark oil. This two spot material was purified via silica gel chromatography employing the Chromatotron and using a 4000 micron rotor while eluting with a solvent of methylene chloride/ethyl acetate 9:1. Collection of the bottom spot provided the final title compound (55 mg, 17%) as a slowly crystallizing oil. Ion spray M.S. 435.3 (M*-1).

| Calculated for: $C_{22}H_{29}N_2O_4SF$: | | | |
|---|---|---|---|
| Theory | C 60.53, | H 6.70, | N 6.42. |
| Found | C 60.52, | H 6.77, | N 6.06. |

Example 23

Preparation of (2-{4-[(3,5-difluorophenyl)methoxy]phenyl}-2-fluoropropyl)[(methylethyl)sulfonyl]amine.

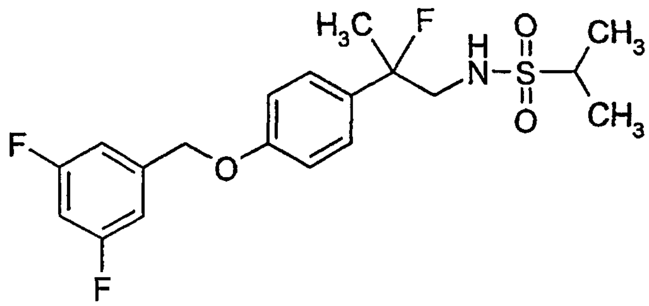

Preparation of [2-hydroxy-2-(4-hvdroxyphenyl)propyl][(methylethyl)sulfonyl]amine.

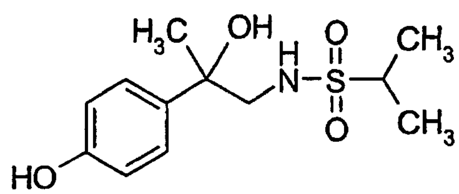

Into a 50 mL-3 neck flask fitted with a stirrer and thermometer was placed 1.0 gm. Of [2-(4-aminophenyl)-2-hydroxypropyl][(methylethyl)sulfonyl]amine (prepared in example 19) and 4.06 gm of 48% $HBF_4$ in $H_2O$ (20 mL) at 0°C. The reaction was then stirred at this temperature for 30 minutes. 0.26 gm of sodium nitrite was then added portion wise to the mixture and stirring at this temperature continued for an additional 30 minutes. The reaction was then slowly heated to 70°C and stirred at this temperature for 3 hours. The mixture was cooled to room temperature and the solution was taken to pH 12 with 1 N NaOH and the solution was filtered. The filtrate was the taken to pH 3.0 with 1 N HCl and the desired product was extracted into ethyl acetate. The organic layer was dried over $Na_2SO_4$, and concentrated under reduced vacuum to yield 1.20 gm of a dark oil. This two spot material was purified via silica gel chromatography employing the Chromatotron and using a 4000 micron rotor while eluting with a solvent of methylene chloride/ethyl acetate 9:1. Collection of the bottom spot provided the intermediate title compound (200 mg, 20%) as a slowly crystallizing oil. Ion spray M.S. 272.4 (M*-9).

Preparation of (2-{4-[(3,5-difluorophenyl)methoxy]phenyl}-2-hydroxypropyl)[(methylethyl)sulfonyl]amine.

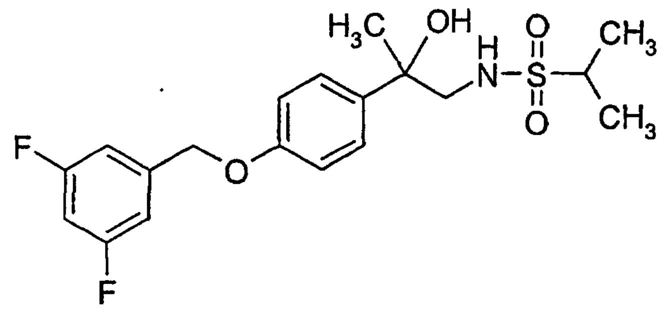

Scheme XI, Step A: Into a 100 mL-3 neck flask fitted with a stirrer and thermometer, 200 mg of [2-hydroxy-2-(4-hydroxyphenyl)propyl][(methylethyl)sulfonyl]amine in DMF (5 mL) was added dropwise to 35 mg of NaH in DMF (20 mL) while stirring at room temperature under a nitrogen atmosphere. After stirring at this temperature for 30 minutes, 153 mg of 3,5-difluorobenzyl bromide in DMF (5 mL) was added dropwise followed by the addition of 92 mg of NaI. portion wise. The reaction was then heated at 100°C for 2 hours. The mixture was cooled to room temperature and poured into $H_2O$ and the desired product was extracted with ethyl acetate. This organic layer was washed with $H_2O$, dried over $K_2CO_3$, and concentrated under reduced vacuum to yield 410 mg of a brown oil. This two spot material was purified via silica gel chromatography employing the Chromatotron and using a 4000 micron rotor while eluting with a solvent of hexane/ethyl acetate 1:1. Collection of the bottom spot provided the intermediate title compound (70 mg, 24%) as an oil. Ion spray M.S. 398.1 (M*-1).

Preparation of final title compound.

Scheme XI, Step B: Into a 25-3 neck flask fitted with a stirrer and thermometer, 70 mg of (2-{4-[(3,5-difluorophenyl)methoxy]phenyl}-2-hydroxypropyl)[(methylethyl)sulfonyl]amine in methylene chloride (5 mL) was added dropwise to 0.1 mL DAST in methylene chloride (5 mL) while stirring at -78°C under a nitrogen atmosphere. The reaction was allowed to warm to room temperature and diluted with methylene chloride (25 mL). This organic layer was washed with $H_2O$, dried over $Na_2SO_4$, and concentrated under reduced vacuum to yield 64 mg of an oil. This material was purified via silica gel chromatography employing the Chromatotron and using a 1000 micron rotor while eluting with a solvent of hexane/ethyl acetate 1:1 to provide the final title compound (45 mg, 63%) as an orange oil. Ion spray M.S. 401.3 (M*).

Example 24

Preparation of {2-fluoro-2-[4-(4-{2-[(methylsulfonyl)amino]ethyl}phenyl)phenyl]propyl}[(methylethyl)sulfonyl]amine.

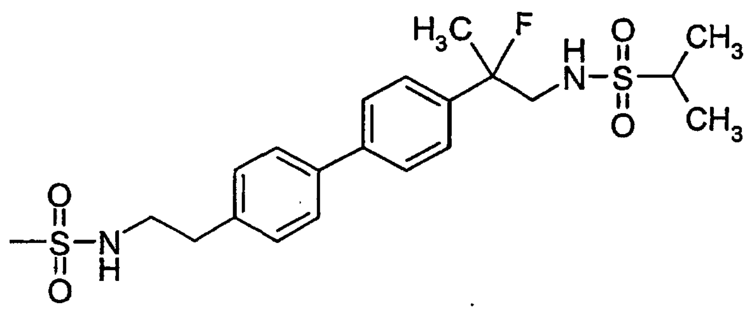

Preparation of {2-hydroxy-2-[4-(4-{2-[(methylsulfonyl)amino]ethyl}phenyl)phenyl]propyl}[(methylethyl)sulfonyl]amine.

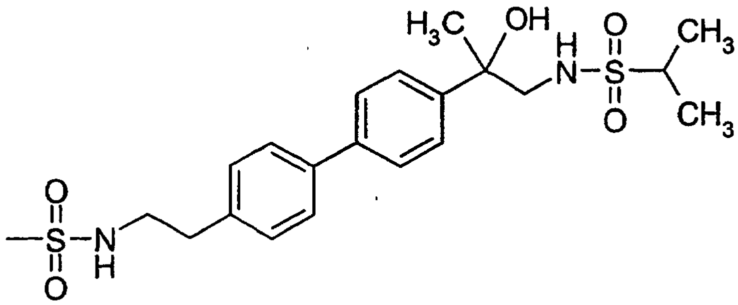

Scheme VI, Step A: [2-hydroxy-2-(4-iodophenyl)propyl][(methylethyl)sulfonyl]amine (383 mg, 100 mmol, intermediate prepared in example 1), BOC-4-(ethyl)methyl-phenyl boronic acid (688 mg, 200 mmol), potassium carbonate (276 mg, 200 mmol), tetrakis(triphenyl phosphine)palladium(0) (58 mg, 0.05 mmol), and dioxane/water (60 mL, 3:1) were mixed together in a 250 mL single neck flask and stirred at 90°C over night. In the moming, the reaction was cooled to room temperature and poured into $H_2O$ and the desired product was extracted with ethyl acetate. The organic layer was backwashed once with $H_2O$, dried over $K_2CO_3$, and concentrated under reduced pressure to yield a 3 spot material as a viscous oil. This material was purified via silica gel chromatography employing the Chromatotron and using a 4000 micron rotor while eluting with a solvent of hexane/ethyl acetate 7:3 to yield the intermediate title compound (383 mg, 72%, bottom spot) as an oil. Ion spray M.S. 453 (M* - 1 ).

| Calculated for $C_{21}H_{30}N_2O_5S_2$: | | | |
|---|---|---|---|
| Theory | C 55.48, | H 6.65, | N 6.16. |
| Found | C 55.11, | H 6.48, | N 6.04. |

Preparation of final title compound.

Scheme VI, Step B: Into a 50-3 neck flask fitted with a stirrer and thermometer, 383 mg of {2-hydroxy-2-[4-(4-{2-[(methylsulfonyl)amino]ethyl}phenyl)phenyl]propyl}[(methylethyl)sulfonyl]amine in methylene chloride (5 mL) was added dropwise to 0.1 mL DAST in methylene chloride (20 mL) while stirring at -78°C under a nitrogen atmosphere. The reaction was allowed to warm to room temperature and diluted with methylene chloride (50 mL). This organic layer was washed with $H_2O$, dried over $Na_2SO_4$; and concentrated under reduced vacuum to yield an oil. This material was purified via silica gel chromatography employing the Chromatotron and using a 2000 micron rotor while eluting with a solvent of hexane/ethyl acetate 1:1 to yield the final title compound (228 mg, 56%) as an oil. Ion spray M.S. 455 (M*-1).

| Calculated for $C_{21}H_{29}N_2O_4S_2F$: | | | |
|---|---|---|---|
| Theory | C 55.24, | H 6.41, | N 6.13. |
| Found | C 55.05, | H 6.41, | N 6.10. |

Example 25

Preparation of (4-chlorophenyl)-N-[4-(1-fluoro-1-methyl-2-{[(methylethyl)sulfonyl]amino}ethyl)phenyl]carboxamide.

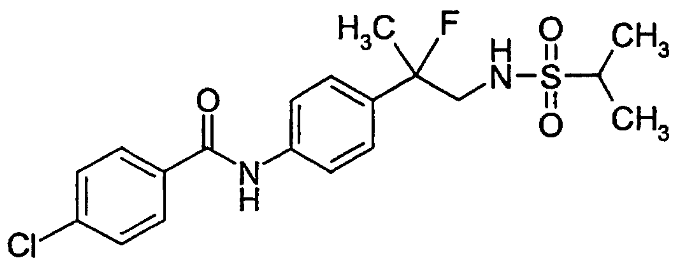

Preparation of (4-chlorophenyl)-N-[4-(1-hydroxy-1-methyl-2-{[(methylethyl)sulfonyl]amino}ethyl)phenyl]carboxamide.

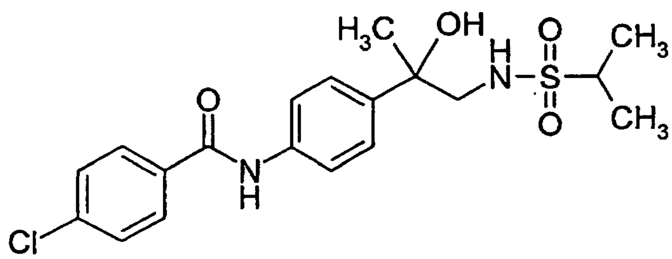

Scheme XII, Step A: Into a 100 mL single-neck flask, 142 mg of 4-chlorobenzoyl chloride was added dropwise to 200 mg of [2-(4-aminophenyl)-2-hydroxypropyl][(methylethyl)sulfonyl]amine (prepared in example 19) and 83 mg of triethylamine in THF (25 mL) while stirring under a nitrogen atmosphere at room temperature. The reaction was allowed to stir at this temperature for 2 h. The mixture was then poured into $H_2O$ and the desired product was extracted into ethyl acetate. The organic layer was backwashed once with $H_2O$, dried over $K_2CO_3$, and concentrated under reduced vacuum to yield 310 mg as a white solid. This material was purified via recrystallization from hexane-ethyl acetate 1: 1 to yield intermediate title compound (165 mg, 54%) as a white solid. Ion spray M.S. 409.2 (M*-1).

| Calculated for $C_{19} H_{23} N_2 O_4 S Cl$: | | | |
|---|---|---|---|
| Theory | C 55.53, | H 5.64, | N 6.82. |
| Found | C 55.33, | H 5.47, | N 6.75. |

Preparation of final title compound.

Scheme XII, Step B: Into a 25 mL 3 neck flask fitted with a stirrer and thermometer, 100 mg of (4-chlorophenyl} -N-[4-(1-hydroxy-1-methyl-2-{[(methylethyl)sulfonyl]amino}ethyl)phenyl]carboxamide in $CH_2Cl_2$ (5 mL) was added dropwise to 0.03 mL DAST in $CH_2CL_2$ (5 mL) while stirring at-78°C under a nitrogen atmosphere. Reaction was allowed to warm to room temperature and diluted with $CH_2Cl_2$ (25 mL). This organic layer was washed with $H_2O$, dried over $Na_2SO_4$, and concentrated under reduced vacuum to yield 108 mg as a foam. This material was triturated in hexane/ ethyl acetate 19:1 for ½ h and then filtered to yield the final title compound (90 mg, 87%) as a tan solid. Ion spray M. S. 413.1 (M*).

| Calculated for: $C_{19}H_{22}N_2O_3SFCl$-1/2 $H_2O$: | | | |
|---|---|---|---|
| Theory | C 54.08, | H 5.49, | N 6.64. |
| Found | C 53.91, | H 5.28, | N 6.75. |

Example 26

Preparation of (6-chloro(3-pyridyl))-N-[4-(1-fluoro-1-methyl-2-{[(methylethyl)sulfonyl]amino}ethyl)phenyl] carboxamide.

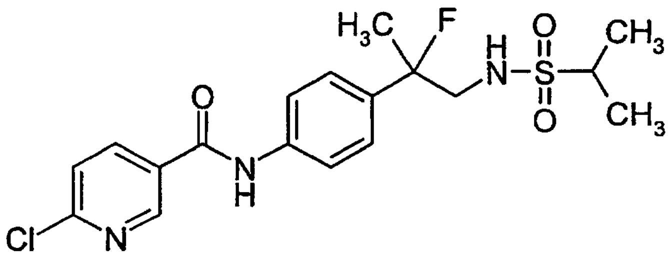

Preparation of (6-chloro(3-pyridyl))-N-[4-(1-hydroxy-1-methyl-2-{[(methylethyl)sulfonyl]amino}ethyl)phenyl] carboxamide.

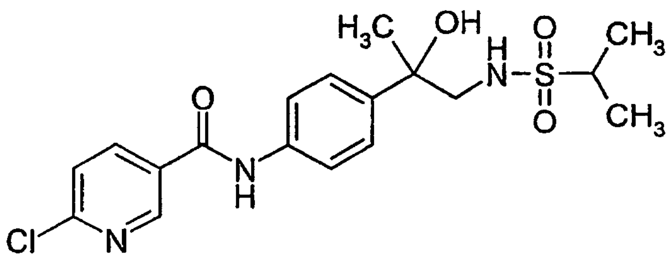

Scheme XII, Step A: Into a 100 mL single-neck flask, 142 mg of 6-chloronicotinoyl chloride was added dropwise to 200 mg of [2-(4-aminophenyl)-2-hydroxypropyl][(methylethyl)sulfonyl]amine (prepared in example 19) and 83 mg of triethylamine in THF (25 mL) while stirring under a nitrogen atmosphere at room temperature. The reaction was allowed to stir at this temperature for 2 h. The mixture was then poured into $H_2O$ and the desired product was extracted into ethyl acetate. The organic layer was backwashed once with $H_2O$, dried over $K_2CO_3$, and concentrated under reduced vacuum to yield 294 mg as a white solid. This material was purified via recrystallization from ethyl acetate to yield the intermediate title compound (161 mg, 53%) as a white solid. Ion spray M.S. 412.1 (M*-1).

| Calculated for $C_{18}$ $H_{22}$ $N_3$ $O_4$ S Cl: | | | |
|---|---|---|---|
| Theory | C 52.49, | H 5.38, | N 10.20. |
| Found | C 51.94, | H 5.20, | N 9.87. |

Preparation of final title compound.

Scheme XII, Step B: Into a 25-3n flask fitted with a stirrer and thermometer, 100 mg of (6-chloro(3-pyridyl)) -N-[4-(1-hydroxy-1-methyl-2-{[(methylethyl)sulfonyl]amino}ethyl)phenyl]carboxamide in $CH_2Cl_2$ (5 mL) was added dropwise to 0.03 mL DAST in $CH_2CL_2$ (5 mL) while stirring at -78°C under a nitrogen atmosphere. Reaction was allowed to warm to room temperature and diluted with $CH_2Cl_2$ (25 mL). This organic layer was washed with $H_2O$, dried over $Na_2SO_4$, and concentrated under reduced vacuum to yield 118 mg as a foam. This material was triturated in hexane/ ethyl acetate 19:1 for ½ h and then filtered to yield the final title compound (80 mg, 81 %) as a white solid. Ion spray M.S. 414.1 (M*).

| Calculated for: $C_{18}H_{21}N_3O_3SFCl$: | | | |
|---|---|---|---|
| Theory | C 52.23, | H 5.11, | N 10.15. |
| Found | C 52.49, | H 5.26, | N 9.75. |

Example 27

Preparation of (4-cyanophenyl)-N-[4-(1-fluoro-1-methyl-2-{[(methylethyl)sulfonyl]amino}ethyl)phenyl]carboxamide.

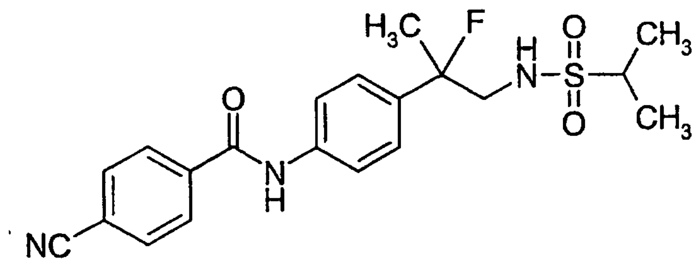

Preparation of (4-cyanophenyl)-N-(4-(1-hydroxy-1-methyl-2-{[(methylethyl)sulfonyl]amino}ethyl)phenyl]carboxamide.

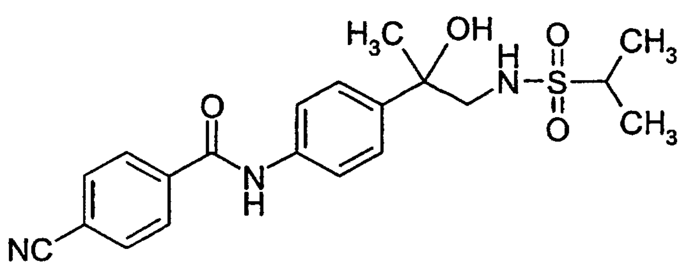

Scheme XII, Step A: Into a 100 mL single-neck flask, 135 mg of 4-cyanobenzoyl chloride was added dropwise to 200 mg of [2-(4-aminophenyl)-2-hydroxypropyl][(methylethyl)sulfonyl]amine (prepared in example 19) and 83 mg of triethylamine in THF (25 mL) while stirring under a nitrogen atmosphere at room temperature. The reaction was allowed to stir at this temperature for 2 h. The mixture was then poured into $H_2O$ and the desired product was extracted into ethyl acetate. The organic layer was backwashed once with $H_2O$, dried over $K_2CO_3$, and concentrated under reduced vacuum to yield 294 mg as a white solid. This material was purified via recrystallization from ethyl acetate/hexane 2: 1 to yield intermediate title compound (131 mg, 55%) as a white solid. Ion spray M.S. 400.2 (M*-1 ).

| Calculated for $C_{20}H_{23}N_3O_4S$ : | | | |
|---|---|---|---|
| Theory | C 59.83, | H 5.77, | N 10.46. |
| Found | C 59.00, | H 5.53, | N 10.27. |

Preparation of final title compound.

Scheme XII, Step B: Into a 25 mL 3 neck flask fitted with a stirrer and thermometer, 100 mg of (4-cyanophenyl) -N-[4-(1-hydroxy-1-methyl-2-{[(methylethyl)sulfonyl]amino}ethyl)phenyl]carboxamide in $CH_2Cl_2$ (5 mL) was added dropwise to 0.03 mL DAST in $CH_2Cl_2$ (5 mL) while stirring at -78°C under a nitrogen atmosphere. The reaction was allowed to warm to room temperature and diluted with $CH_2Cl_2$ (25 mL). This organic layer was washed with $H_2O$, dried

over $Na_2SO_4$, and concentrated under reduced vacuum to yield 107 mg as a yellow solid. This material was triturated in hexane/ethyl acetate 19:1 for ½ h and then filtered to yield the final title compound (65 mg, 65%) as a tan solid. Ion spray M.S. 404.1 (M*+1).

| Calculated for: $C_{20}H_{22}N_3O_3SF$-1/2 $H_2O$: | | | |
|---|---|---|---|
| Theory | C 58.23, | H 5.62, | N 10.18. |
| Found | C 58.36, | H 5.37, | N 10.16. |

Example 28

Preparation of ethoxy-N-[4-(1-fluoro-1-methyl-2-{[(methylethyl)sulfonyl]amino}ethyl)phenyl]carboxamide.

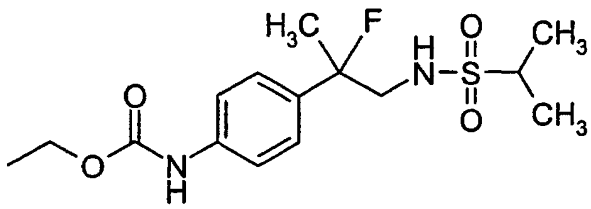

Preparation of ethoxy-N-[4-(1-hydroxy-1-methyl-2-{[(methylethyl)sulfonyl]amino}ethyl)phenyl]carboxamide.

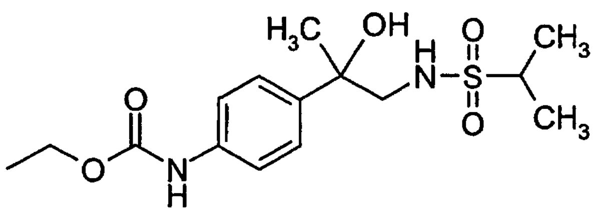

Scheme XII, Step A: Into a 100 mL single-neck flask, 89 mg of ethyl chloroformate was added dropwise to 200 mg of [2-(4-aminophenyl)-2-hydroxypropyl][(methylethyl)sulfonyl]amine (prepared in example 19) and 83 mg of triethylamine in THF (25 mL) while stirring under a nitrogen atmosphere at room temperature. The reaction was allowed to stir at this temperature for 2 h. The mixture was then poured into $H_2O$ and the desired product was extracted into ethyl acetate. The organic layer was backwashed once with $H_2O$, dried over $K_2CO_3$, and concentrated under reduced vacuum to yield 317 mg as an oil. This material was purified via silica gel chromatography employing the Chromatotron while using a 4000 micron rotor and eluting with an isocratic solvent of methylene chloride/ethyl acetate 7:3 to yield intermediate title compound (200 mg, 78%) as a viscous oil. Ion spray M.S. 343.2 (M*-1).

| Calculated for $C_{15}$ $H_{24}$ $N_2$ $O_5$ S : | | | |
|---|---|---|---|
| Theory | C 52.30, | H 7.02, | N 8.13. |
| Found | C 52.01, | H 6.98, | N 7.95. |

Preparation of final title compound.

Scheme XII, Step B: Into a 25 mL, 3 neck flask fitted with a stirrer and thermometer, 175 mg of ethoxy-N-[4-(1-hydroxy-1-methyl-2-{[(methylethyl)sulfonyl]amino}ethyl)phenyl]carboxamide in $CH_2Cl_2$ (5 mL) was added dropwise to 0.04 mL DAST in $CH_2CL_2$ (10 mL) while stirring at -78°C under a nitrogen atmosphere. The reaction was allowed to warm to room temperature and diluted with $CH_2Cl_2$ (25 mL). This organic layer was washed with $H_2O$, dried over $Na_2SO_4$, and concentrated under reduced vacuum to yield 181 mg as an oil. This material was purified via silica gel

chromatography employing the Chromatotron, using a 2000 micron rotor and eluting with a solvent of hexane/ethyl acetate 7:3 to yield the final title compound (101 mg, 57%) as a viscous oil. Ion spray M.S. 345.1 (M*-1).

| Calculated for: $C_{15}H_{23}N_2O_4SF$: | | | |
|---|---|---|---|
| Theory | C 52.01, | H 6.69, | N 8.09. |
| Found | C 51.82, | H 6.64, | N 8.22. |

Example 29

Preparation of N-[4-(1-fluoro-1-methyl-2-{[(methylethyl)sulfonyl]amino}ethyl)phenyl]isoxazol-5-ylcarboxamide.

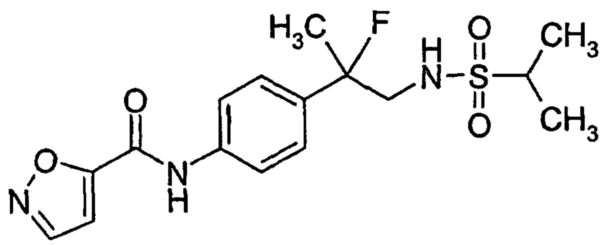

Preparation of N-[4-(1-hydroxy-1-methyl-2-{[(methylethyl)sulfonyl]amino}ethyl)phenyl]isoxazol-5-ylcarboxamide.

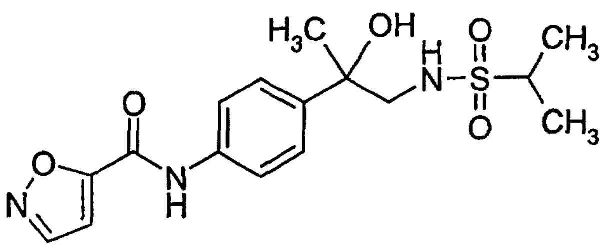

Scheme XII, Step A: Into a 100 mL single-neck flask, 159 mg of isoxazole-5-carbonyl chloride was added dropwise to 300 mg of [2-(4-aminophenyl)-2-hydroxypropyl][(methylethyl)sulfonyl]amine (prepared in example 19) and 122 mg of triethylamine in THF (35 mL) while stirring under a nitrogen atmosphere at room temperature. The reaction was allowed to stir at this temperature for 2 h. The mixture was then poured into $H_2O$ and the desired product was extracted into ethyl acetate. The organic layer was backwashed once with $H_2O$, dried over $K_2CO_3$, and concentrated under reduced vacuum to yield 416 mg as a solid. This material was purified via recrystallization from ethyl acetate/hexane 1:1 to yield intermediate title compound (207 mg, 51%) as a white solid. Ion spray M.S. 366.1 (M*-1).

| Calculated for $C_{16}$ $H_{21}$ $N_3$ $O_5$ S | | | |
|---|---|---|---|
| Theory | C 52.30, | H 5.76, | N 11.43. |
| Found | C 52.25, | H 5.80, | N 11.25. |

Preparation of final title compound.

Scheme XII, Step B: Into a 25 mL 3 neck flask fitted with a stirrer and thermometer, 200 mg of N-[4-(1-hydroxy-1-methyl-2-{[(methylethyl)sulfonyl]amino}ethyl)phenyl]isoxazol-5-ylcarboxamide in $CH_2Cl_2$ (5 mL) was added dropwise to 0.07 mL DAST in $CH_2Cl_2$ (10 mL) while stirring at -78°C under a nitrogen atmosphere. The reaction was allowed to warm to room temperature and diluted with $CH_2Cl_2$ (25 mL). This organic layer was washed with $H_2O$, dried over $Na_2SO_4$, and concentrated under reduced vacuum to yield 321 mg as an oil. This material was purified via silica gel

chromatography employing the Chromatotron, using a 2000 micron rotor and eluting with a solvent of hexane/ethyl acetate 1:1 to yield the final title compound (187 mg, 94%) as a semi-solid. Ion spray M.S. 368.2 (M*-1).

| Calculated for: $C_{16}H_{20}N_3O_4SF$: | | | |
|---|---|---|---|
| Theory | C 52.02, | H 5.46, | N 11.37. |
| Found | C 51.70, | H 5.59, | N 10.88. |

Example 30

Preparation of 4-(dimethylamino)-N-[4-(1-fluoro-1-methyl-2-{[(methylethyl)sulfonyl]amino}ethyl)phenyl]butanamide.

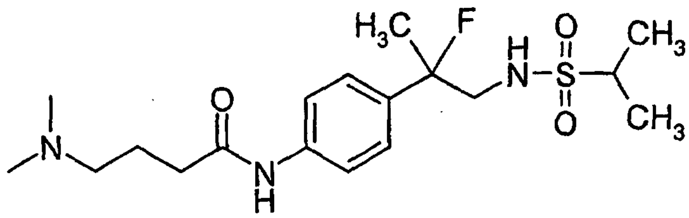

Preparation of 4-(dimethylamino)-N-[4-(1-hydroxy-1-methyl-2-{[(methylethyl)sulfonyl]amino}ethyl)phenyl]butanamide.

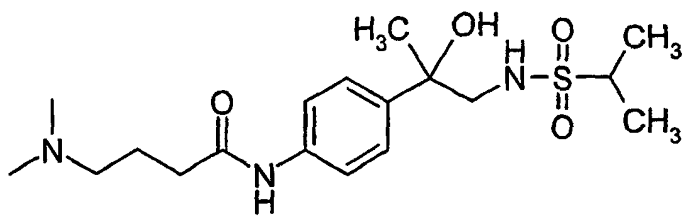

Into a 50 mL single-neck flask, 1 mL of oxalyl chloride was added syringe wise to 306 mg of 4-(dimethylamino) butyric acid hydrochloride in $CH_2Cl_2$ (20 mL) while stirring under a nitrogen atmosphere at room temperature. Then at this temperature, 1 drop of DMF was added by pipette and the mixture began to foam. The reaction was allowed to stir at this temperature for 1 h and was then concentrated under reduced vacuum to yield a yellow solid. This solid was added portion wise to a 100 mL single-neck flask containing 460 mg of [2-(4-aminophenyl)-2-hydroxypropyl][(methylethyl)sulfonyl]amine (prepared in example 19) and 183 mg of triethylamine in THF (25 mL) while stirring at room temperature under a nitrogen atmosphere. After 2 h at this temperature, the mixture was concentrated under reduced vacuum and the resulting solid was taken into ethyl acetate. The organic layer was backwashed once with $H_2O$, dried over $K_2CO_3$, and concentrated under reduced vacuum to yield 794 mg as an oil. This material was purified via silica gel chromatography employing the Chromatotron while using a 4000 micron rotor and eluting with an isocratic solvent of methanol/1% $NH_4OH$ to yield intermediate title compound (350 mg, 51 %) as a viscous oil. Ion spray M.S. 385 (M*).

Preparation of final title compound.

Scheme I, Step B: Into a 25 mL 3 neck flask fitted with a stirrer and thermometer, 335 mg of 4-(dimethylamino) -N-[4-(1-hydroxy-1-methyl-2-{[(methylethyl)sulfonyl]amino}ethyl)phenyl]butanamide in $CH_2Cl_2$ (5 mL) was added dropwise to 0.11 mL DAST in $CH_2CL_2$ (10 mL) while stirring at -78°C under a nitrogen atmosphere. Reaction was allowed to warm to room temperature and diluted with $CH_2Cl_2$ (25 mL). This organic layer was washed with $H_2O$, dried over $Na_2SO_4$, and concentrated under reduced vacuum to yield 160 mg as an oil. This material was purified via silica gel chromatography employing the Chromatotron, using a 2000 micron rotor and eluting with a solvent of methanol/1 % $NH_4OH$ to yield the final title compound (100 mg, 30%) as a semi-solid. Ion spray M.S. 388.0 (M*+1).

Example 31

Preparation of N-[4-(1-fluoro-1-methyl-2-{[(methylethyl)sulfonyl]amino}ethyl)phenyl]-3-thienylcarboxamide.

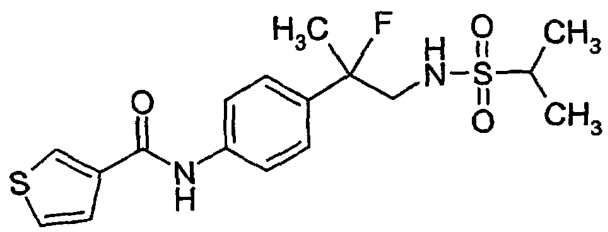

Preparation of N-[4-(1-hydroxy-1-methyl-2-{[(methylethyl)sulfonyl]amino}ethyl)phenyl]-3-thienylcarboxamide.

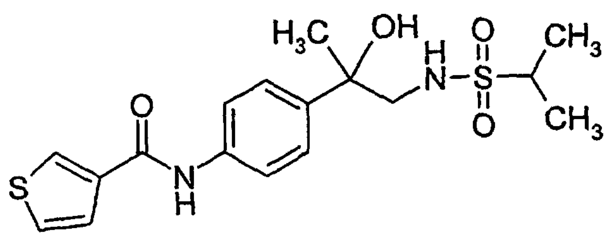

Scheme XII, Step A: [2-(4-aminophenyl)-2-hydroxypropyl][(methylethyl)sulfonyl]amine (300 mg, 1.1 mmol, prepared in example 19), 3-thiophenecarboxylic acid (169 mg, 1.2 mmol), triethylamine (333 mg, 3.0 mmol), 4-dimethylaminopyridine (15 mg, DMAP), 1-[3-(dimethylamino)propyl]-3-ethylcarbodiimide-HCl (260 mg, 1.2 mmol) and methylene chloride (25 mL) were mixed together in a 50 mL single neck flask and stirred under a nitrogen atmosphere at room temperature for 64 hours. The solution was then poured into $H_2O$, and the desired product was extracted with ethyl acetate. The organic layer was backwashed once with $H_2O$, dried over $K_2CO_3$, and concentrated under reduced vacuum to yield 634 mg as an oil. This material was purified via silica gel chromatography employing the Chromatotron, using a 4000 micron rotor and eluting with a solvent of hexane/ethyl acetate 1:1 to yield intermediate title compound (151 mg, 36%) as a white solid. Ion spray M.S. 381.1 (M*+1).

| Calculated for: $C_{17}H_{22}N_2O_4S_2$: | | | |
|---|---|---|---|
| Theory | C 53.38, | H 5.80, | N 7.32. |
| Found | C 53.29, | H 5.79, | N 7.39. |

Preparation of final title compound.

Scheme XII, Step B: Into a 25 mL 3 neck flask fitted with a stirrer and thermometer, 100 mg of N-[4-(1-hydroxy-1-methyl-2-{[(methylethyl)sulfonyl]amino}ethyl)phenyl]-3-thienylcarboxamide in $CH_2Cl_2$ (5 mL) was added dropwise to 0.03 mL DAST in $CH_2CL_2$ (5 mL) while stirring at -78°C under a nitrogen atmosphere. The reaction was allowed to warm to room temperature and diluted with $CH_2Cl_2$ (25 mL). This organic layer was washed with $H_2O$, dried over $Na_2SO_4$, and concentrated under reduced vacuum to yield 111 mg as a foam. This material was triturated in hexane/ethyl acetate 19:1 for ½ h and then filtered to yield the final title compound (80 mg, 70%) as a white solid. Ion spray M.S. 385.1 (M*+1).

| Calculated for: $C_{17}H_{21}N_2O_3S_2F$: | | | |
|---|---|---|---|
| Theory | C 53.11, | H 5.51, | N 7.29. |

(continued)

| Calculated for: $C_{17}H_{21}N_2O_3S_2F$: | | | |
|---|---|---|---|
| Found | C 52.93, | H 5.44, | N 7.15. |

Example 32

Preparation of {2-fluoro-2-[4-(phenylmethoxy)phenyl]propyl}[(methylethyl)sulfonyl]amine.

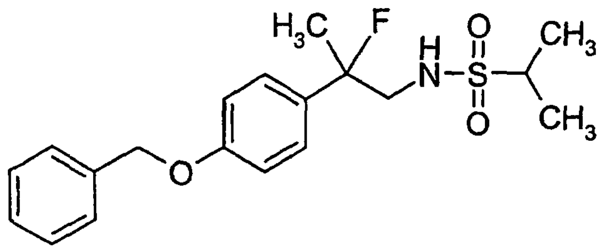

Preparation of 1-amino-2-[4-(phenylmethoxy)phenyl]propan-2-ol.

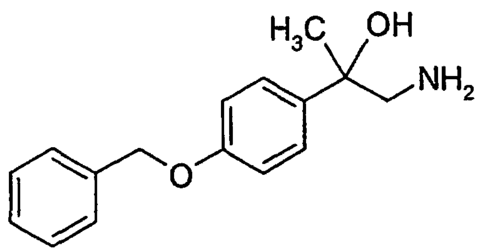

Into a 100 mL single-neck flask, 50 mL of trimethylsilyl cyanide was added dropwise to 26 g of 4'-benzyloxacetophenone and 3.9 g of zinc iodide while stirring under a nitrogen atmosphere at room temperature. The reaction was allowed to stir at this temperature overnight. In the morning, the mixture was diluted with methylene chloride (100 mL) and the organic layer was backwashed once with sat. $Na_2HCO_3$, dried over $MgSO_4$, and concentrated under reduced vacuum to yield 23.25 g. as an orange oil. Because of possible stability problems, this material was used immediately and placed into 300 mL THF in a 1000 mL single-neck flask. While stirring at room temperature under a nitrogen atmosphere, 100 mL of $BH_3$-THF complex was added syringe wise and the reaction was stirred overnight. In the morning, 10 mL of con. hydrochloric acid was added dropwise at room temperature and severe foaming was present. The mixture was then concentrated under reduced vacuum. The resulting HCl salt was liberated while being stirred in 1N NaOH and the free amine was extracted into ethyl acetate. This organic layer was washed with $H_2O$, dried over $Na_2SO_4$, and concentrated under reduced vacuum to yield 24.31 g. as a semi-solid. This material was purified via silica gel chromatography employing the Water's prep. 2000 and eluting with a gradient solvent of methylene chloride/methanol 9:1 to methanol/1 % $NH_4OH$ to yield intermediate title compound (8.61 g, 27%) as a white solid. (FD) M.S. 256.9 (M*).

| Calculated for: $C_{16}H_{19}NO_2$: | | | |
|---|---|---|---|
| Theory | C 74.68, | H 7.44, | N 5.44. |
| Found | C 74.20, | H 7.29, | N 5.44. |

Preparation of {2-hydroxy-2-[4-(phenylmethoxy)phenyl]propyl}[(methylethyl)sulfonyl]amine.

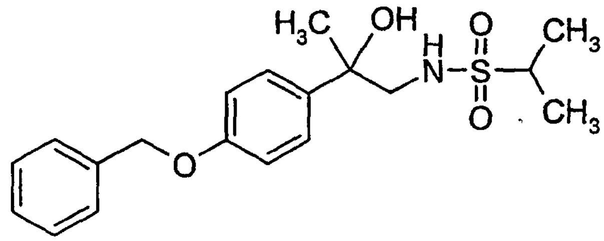

Scheme I, Step A: In a 1000 mL-3n flask fitted with a stirrer and thermometer, 5.69 g. of propanesulfonyl chloride was added dropwise to 8.60 g. of 1-amino-2-[4-(phenylmethoxy)phenyl]propan-2-ol and 6.21 g. of DBU in THF (300 mL) while stirring at 0°C under a nitrogen atmosphere. The reaction was allowed to warm to room temperature and stirred overnight at this temperature. In the morning, reaction was concentrated under reduced vacuum. The resulting oil was taken into ethyl acetate and the organic layer was washed two times with $H_2O$, dried over $Na_2SO_4$, and concentrated under reduced vacuum to yield 10.2 g. as a solid. This material was purified via silica gel chromatography employing the Water's prep. 2000 and eluting with a solvent of hexane/ethyl acetate 9:1 to yield intermediate title compound (6.14 g, 50%) as a white solid. Ion spray M.S. 362.2 (M* - 1 ).

| Calculated for: $C_{19}H_{25}NO_4$ S: | | | |
|---|---|---|---|
| Theory | C 62.79 | H 6.93, | N 3.85. |
| Found | C 62.85, | H 6.89, | N 3.88. |

Preparation of final title compound.

Scheme I, Step B: Into a 25 mL 3 neck flask fitted with a stirrer and thermometer, 400 mg of {2-hydroxy-2-[4-(phenylmethoxy)phenyl]propyl}[(methylethyl)sulfonyl]amine in $CH_2Cl_2$ (5 mL) was added dropwise to 0.1 mL DAST in $CH_2CL_2$ (5 mL) while stirring at -78°C under a nitrogen atmosphere. The reaction was allowed to warm to room temperature and diluted with $CH_2Cl_2$ (25 mL). This organic layer was washed with $H_2O$, dried over $Na_2SO_4$, and concentrated under reduced vacuum to yield 340 mg as a yellow oil. This material was purified via silica gel chromatography employing the Chromatotron and using a 2000 micron rotor while eluting with a solvent of hexane/ethyl acetate 7:3 to yield the final title compound (271 mg, 68%) as a semi- solid. Ion spray M.S. 364.2 (M*-1).

| Calculated for $C_{19}H_{24}NO_3SF$: | | | |
|---|---|---|---|
| Theory | C 62.44, | H 6.62, | N 3.83. |
| Found | C 62.39, | H 6.58, | N 3.92. |

Example 33

Preparation of 2-{[4-(1-fluoro-1-methyl-2-{[(methylethyl)sulfonyl]amino}ethyl)phenoxy]methyl}benzenecarbonitrile.

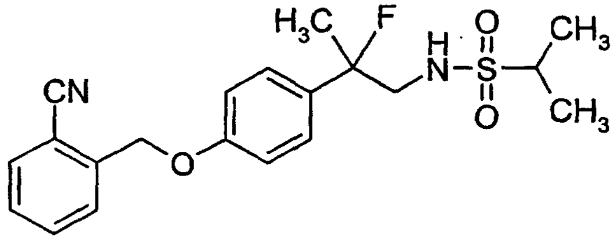

Preparation of [2-hydroxy-2-(4-hydroxyphenyl)propyl][(methylethyl)sulfonyl]amine.

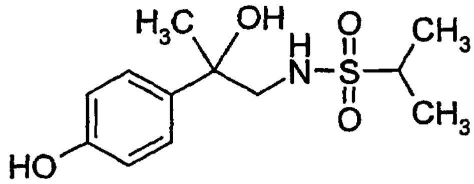

3.00 g of {2-hydroxy-2-[4-(phenylmethoxy)phenyl]propyl}[(methylethyl)sulfonyl]amine (prepared in example 32) and 3.10 g. of 10% Pd/C in ethyl acetate (300 mL) were subjected to a hydrogen atmosphere while shaking at 60 psi's for 4 hours. Solution was filtered through a Celite mat and the resulting filtrate was concentrated under reduced vacuum to yield intermediate title compound (2.48 g, 100%) as a foam. This material slowly crystallized and was used without further purification. Ion spray M.S. 272.1 (M*-1).

| Calculated for $C_{12}H_{19}NO_4S$-1/2 $H_2O$: | | | |
|---|---|---|---|
| Theory | C 51.03, | H 7.14, | N 4.96 |
| Found | C 51.20, | H 7.21, | N 4.71 |

Preparation of 2-{[4-(1-hydroxy-1-methyl-2-{[(methylethyl)sulfonyl]amino}ethyl)phenoxy]methyl}benzenecarbonitrile.

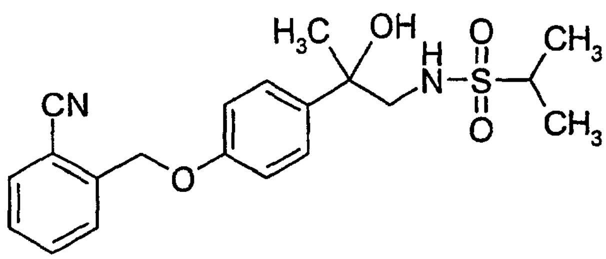

Scheme XI, Step A: Into a 250 mL-3 neck flask fitted with a stirrer and thermometer, 400 mg of [2-hydroxy-2-(4-hydroxyphenyl)propyl][(methylethyl)sulfonyl]amine in DMF (10 mL) was added dropwise to 40 mg of NaH in DMF (30 mL) while stirring at room temperature under a nitrogen atmosphere. After stirring at this temperature for 30 minutes, 290 mg of alpha-bromo 2-cyanotoluene in DMF (10 mL) was added dropwise followed by the addition of 184 mg of Nal portion wise. The reaction was then heated at 100°C for 2 hours. The mixture was cooled to room temperature and poured into $H_2O$ and the desired product was extracted with ethyl acetate. This organic layer was washed with $H_2O$, dried over $K_2CO_3$, and concentrated under reduced vacuum to yield 612 mg of a brown oil. This material was purified via silica gel chromatography employing the Chromatotron and using a 4000 micron rotor while eluting with a solvent of hexane/ethyl acetate 1:1 to yield intermediate title compound (161 mg, 28%) as a viscous oil.. Ion spray M. S. 387.9 (M*-1).

| Calculated for $C_{20}H_{24}N_2O_4S$: | | | |
|---|---|---|---|
| Theory | C 61.84, | H 6.23, | N 7.21 |
| Found | C 59.83, | H 5.65, | N 7.36 |

Preparation of final title compound.

Scheme XI, Step B: Into a 25 mL 3 neck flask fitted with a stirrer and thermometer, 150 mg of 2-{[4-(1-hydroxy-1-methyl-2-{[(methylethyl)sulfonyl]amino}ethyl)phenoxy]methyl}benzenecarbonitrile in $CH_2Cl_2$ (5 mL) was added dropwise to 0.06 mL DAST in $CH_2CL_2$ (5 mL) while stirring at -78°C under a nitrogen atmosphere. The reaction was allowed

to warm to room temperature and diluted with $CH_2Cl_2$ (25 mL). This organic layer was washed with $H_2O$, dried over $Na_2SO_4$, and concentrated under reduced vacuum to yield 157 mg as a yellow oil. This material was purified via silica gel chromatography employing the Chromatotron and using a 2000 micron rotor while eluting with a solvent of hexane/ethyl acetate 9:1 to yield the final title compound (91 mg, 60%) as a solid. Ion spray M.S. 389.2 (M*-1 ).

| Calculated for $C_{20}H_{23}N_2O_3SF$: | | | |
|---|---|---|---|
| Theory | C 61.52, | H 5.94, | N 7.17. |
| Found | C 61.16, | H 5.93, | N 7.14. |

Example 34

Preparation of [2-fluoro-2-(4-methoxyphenyl)propyl][methylethyl)sulfonyl]amine.

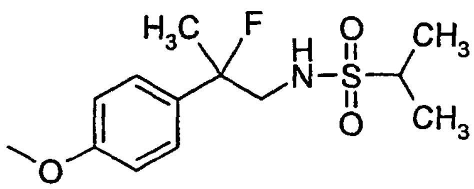

Preparation of 1-amino-2-(4-methoxyphenyl)propan-2-ol.

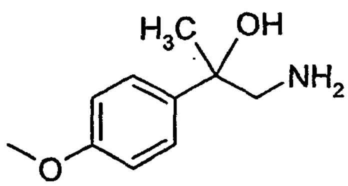

Into a 100 mL single-neck flask, 28.5 mL of trimethylsilyl cyanide was added dropwise to 10 g of 4'-methoxyacetophenone and 2.28 g of zinc iodide while stirring under a nitrogen atmosphere at room temperature. The reaction was allowed to stir at this temperature overnight. In the morning, the mixture was diluted with methylene chloride (100 mL) and the organic layer was backwashed once with sat. $Na_2HCO_3$, dried over $MgSO_4$, and concentrated under reduced vacuum to yield 13 g. as a tan oil. Because of possible stability problems, this material was used immediately and placed into 200 mL THF in a 500 mL single-neck flask. While stirring at room temperature under a nitrogen atmosphere, 70 mL of $BH_3$-THF complex was added syringe wise and the reaction was stirred overnight. In the morning, 10 mL of concentrated hydrochloric acid was added dropwise at room temperature and severe foaming was present. The mixture was then concentrated under reduced vacuum. The resulting HCl salt was liberated while being stirred in 1N NaOH and the free amine was extracted into ethyl acetate. This organic layer was washed with $H_2O$, dried over $Na_2SO_4$, and concentrated under reduced vacuum to yield 7.31 g. of a 2 spot material. This material was purified via silica gel chromatography employing the Water's prep. 2000 and eluting with a solvent of methylene chloride/methanol 9:1 to yield intermediate title compound (3.01 g, 24%) as an oil as the top spot. (FD) M.S. 180.3 (M*-1).

| Calculated for: $C_{10}H_{15}NO_2$ | | | |
|---|---|---|---|
| Theory | C 66.27, | H 8.34, | N 7.73. |
| Found | C 64.46, | H 7.94, | N 7.50. |

Preparation of [2-hydroxy-2-(4-methoxyphenyl)propyl][(methylethyl)sulfonyl]amine.

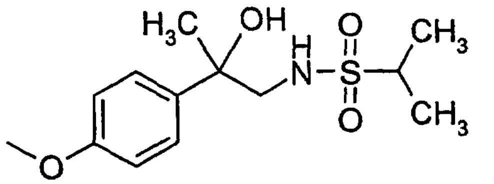

Scheme I, Step A: In a 500 mL-3n flask fitted with a stirrer and thermometer, 2.53 g. of propanesulfonyl chloride was added dropwise to 3.00 g. of 1-amino-2-(4-methoxyphenyl)propan-2-ol and 2.70 g. of DBU in THF (200 mL) while stirring at 0°C under a nitrogen atmosphere. The reaction was allowed to warm to room temperature and stirred overnight at this temperature. In the morning, reaction was concentrated under reduced vacuum. The resulting oil was taken into ethyl acetate and the organic layer was washed two times with $H_2O$, dried over $Na_2SO_4$, and concentrated under reduced vacuum to yield 3.61 g. as a solid. This material was purified via silica gel chromatography employing the Water's prep. 2000 and eluting with a solvent of hexane/ethyl acetate 9:1 to yield intermediate title compound (2.71 g, 57%) as a white solid. Ion spray M.S. 286.2 (M* - 1).

Preparation of final title compound.

Scheme I, Step B: Into a 50 mL 3 neck flask fitted with a stirrer and thermometer, 1.00 gm. of [2-hydroxy-2-(4-methoxyphenyl)propyl][(methylethyl)sulfonyl]amine in $CH_2Cl_2$ (5 mL) was added dropwise to 0.41 mL DAST in $CH_2CL_2$ (10 mL) while stirring at -78°C under a nitrogen atmosphere. The reaction was allowed to warm to room temperature and diluted with $CH_2Cl_2$ (25 mL). This organic layer was washed with $H_2O$, dried over $Na_2SO_4$, and concentrated under reduced vacuum to yield 1.07 g. as a yellow oil. This material was purified via silica gel chromatography employing the Chromatotron and using a 4000 micron rotor while eluting with a solvent of hexane/ethyl acetate 7:3 to yield the final title compound (851 mg, 85%) as a viscous oil. Ion spray M.S. 288.1 (M*-1).

| Calculated for $C_{13}H_{20}NO_3SF$: | | | |
|---|---|---|---|
| Theory | C 53.96, | H 6.97, | N 4.84. |
| Found | C 53.16, | H 6.76, | N 4.81. |

Example 35

Preparation of {2-[4-(3,5-difluorophenyl)phenyl]-2-fluoropropyl}[(methylethyl)sulfonyl]amine.

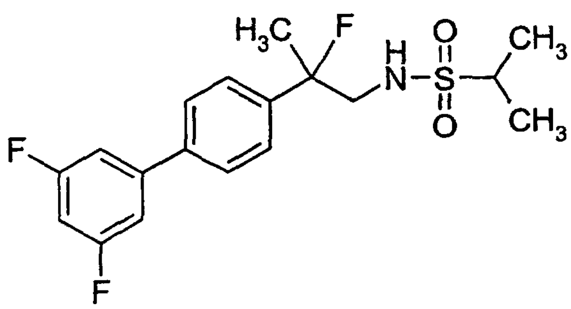

Preparation of:

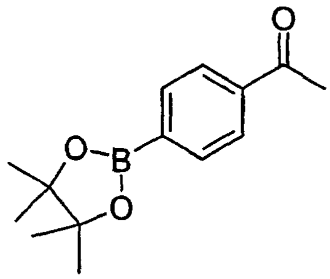

Into a 500 mL single-neck flask fitted with a stirrer and thermometer, 10.0 g. of 4'-iodoacetophenone, 1.0 g. of $PdCl_2$(dppf), and 17 mL triethylamine were placed in Dioxane (160 mL) and stirred at room temperature under a nitrogen atmosphere. This solution was then degassed with nitrogen for 15 minutes, followed by the addition of 8.8 mL pinacol borane syringe wise. Addition was exothermic and the solution turned dark immediately. The reaction was then heated at 85°C for 6 h. The mixture was then cooled to room temperature and poured into $H_2O$ and the desired product was extracted with ether. This organic layer was washed with $H_2O$, dried over $MgSO_4$, and concentrated under reduced vacuum to yield a white solid. This material was purified via silica gel chromatography employing the Water's prep. 2000 and eluting with hexane/ethyl acetate 19:1 to yield intermediate title compound, acetophenone derivative, (6.4 g, 64%) as a white powder.

Preparation of:

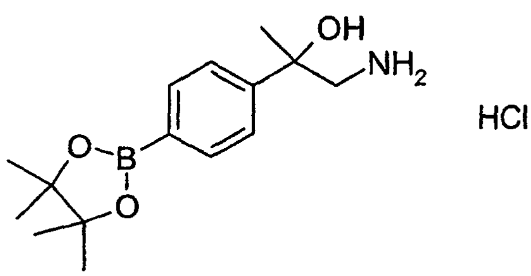

Into a 25 mL single-neck flask, 1.6 mL of trimethylsilyl cyanide was added dropwise to 1.0 g. of the acetophenone derivative prepared directly above and 131 mg of zinc iodide while stirring under a nitrogen atmosphere at room temperature. The reaction was then stirred overnight at this temperature. In the morning the mixture was diluted with methylene chloride (25 mL) and the organic layer was backwashed once with sat. $Na_2HCO_3$, dried over $MgSO_4$, and concentrated under reduced vacuum to yield 910 mg as a semi-solid. Because of possible stability problems, this material was used immediately and placed into 20 mL THF in a 100 mL single-neck flask. While stirring at room temperature under a nitrogen atmosphere, 4 mL of $BH_3$-DMS complex was added syringe wise and the reaction was refluxed for 4 hours. The reaction was cooled to room temperature and 2 mL of con. hydrochloric acid was added dropwise at room temperature and severe foaming was present. The mixture was diluted with ether (50mL) and the reaction was stirred for an additional hour. The precipitate that formed was collected by filtration to yield intermediate title compound, amine derivative, (910 mg, 72%) as a white powder as the HCl salt.

Preparation of:

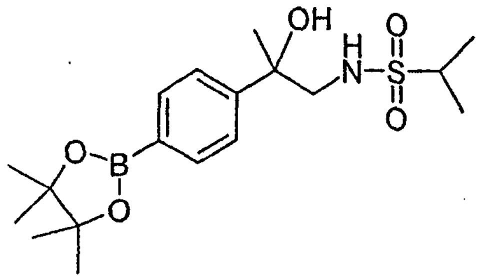

In a 250 mL 3 neck flask fitted with a stirrer and thermometer, 1.05 g. of propanesulfonyl chloride was added dropwise to 2.10 g. of the amine derivative prepared directly above, and 2.14 g. of DBU in THF (60 mL) while stirring at 0°C under a nitrogen atmosphere. The reaction was allowed to warm to room temperature and stirred overnight at this temperature. In the morning, reaction was concentrated under reduced vacuum. The resulting oil was taken into ethyl acetate and the organic layer was washed two times with $H_2O$, dried over $Na_2SO_4$, and concentrated under reduced vacuum to yield 600 mg as an oil. This material was purified via silica gel chromatography employing the Chromatotron and using a 4000 micron rotor while eluting with a solvent of hexane/ethyl acetate 3:2 to yield intermediate title compound, sulfonyl derivative, (250 mg, 10%) as an oil. Ion spray M.S. 382.2 (M*-1).

Preparation of {2-[4-(3,5-difluorophenyl)phenyl]-2-hydroxypropyl}[(methylethyl)sulfonyl]amine.

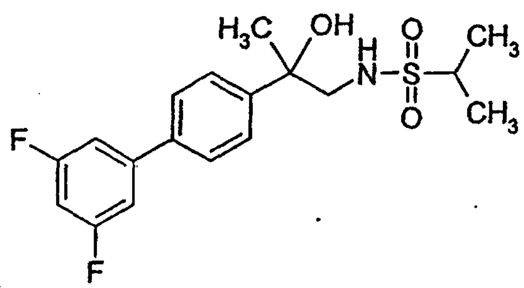

The sulfonyl derivative prepared directly above (250 mg, 0.65 mmol), 1-bromo-3,5-difluorobenzene (138 mg, 0.71 mmol), sodium carbonate (1.1 mL of a 2.0 M solution, 1.4 mmol), tetrakis(triphenyl phosphine)palladium(0) (50 mg, 0.65 mmol), and dioxane (10 mL) were mixed together in a 50 mL single neck flask and stirred at 70°C over night. In the morning, the reaction was cooled to room temperature and poured into $H_2O$ and the desired product was extracted with ethyl acetate. The organic layer was backwashed once with $H_2O$, dried over $K_2CO_3$, and concentrated under reduced pressure to yield 286 mg of a viscous oil. This two spot material was purified via silica gel chromatography employing the Chromatotron and using a 2000 micron rotor while eluting with a solvent of hexane/ethyl acetate 1:1 to yield intermediate title compound (73 mg, 31 %, top spot) as a clear oil. Ion spray M.S. 368.2 (M* - 1).

Preparation of final title compound.

Scheme I, Step B: Into a 25 mL 3 neck flask fitted with a stirrer and thermometer, 22 mg of {2-[4-(3,5-difluorophenyl)phenyl]-2-hydroxypropyl}[(methylethyl)sulfonyl]amine in $CH_2Cl_2$ (2 mL) was added dropwise to 0.01 mL DAST in $CH_2CL_2$ (3 mL) while stirring at -78°C under a nitrogen atmosphere. The reaction was allowed to warm to room temperature and diluted with $CH_2Cl_2$ (25 mL). This organic layer was washed with $H_2O$, dried over $Na_2SO_4$, and concentrated under reduced vacuum to yield 18.4 mg as a yellow oil. This material was purified via silica gel chromatography employing the Chromatotron and using a 1000 micron rotor while eluting with a solvent of hexane/ethyl acetate 7:3 to yield the final title compound (12 mg, 52%) as a viscous oil. Ion spray M.S. 370.2 (M*-1).

Example 36

Preparation of [(dimethylamino)sulfonyl][2-fluoro-2-(4-iodophenyl)propyl]amine.

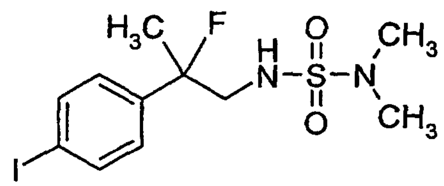

Preparation of [(dimethylamino)sulfonyl][2-hydroxy-2-(4-iodophenyl)propyl]amine.

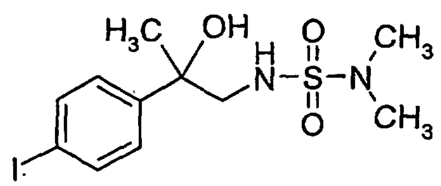

Scheme X, Step A: In a 500 mL-3 neck flask fitted with a stirrer and thermometer, 358 mg of dimethylsulfamoyl chloride was added dropwise to 554 mg of 1-amino-2-(4-iodophenyl)propan-2-ol (intermediate prepared in example 1) and 380 mg of DBU in THF (125 mL) while stirring at 0°C under a nitrogen atmosphere. The reaction was allowed to warm to room temperature and stirred overnight at this temperature. In the morning, reaction was concentrated under reduced vacuum. The resulting oil was taken into ethyl acetate and the organic layer was washed two times with $H_2O$, dried over $Na_2SO_4$, and concentrated under reduced vacuum to yield an oil. This material was purified via silica gel chromatography employing the Chromatotron and using a 2000 micron rotor while eluting with a solvent of hexane/ethyl acetate 3:1 to yield intermediate title compound (257 mg, 33%) as a white solid. (Fd) M.S. 384 (M*).

| Calculated for: $C_{11}H_{17}N_2O_3SI$: | | | |
|---|---|---|---|
| Theory | C 34.39, | H 4.46, | N 7.29. |
| Found | C 33.25, | H 4.24, | N 6.80. |

Preparation of final title compound.

Scheme X, Step B: Into a 50 mL 3 neck flask fitted with a stirrer and thermometer, 257 mg of [(dimethylamino) sulfonyl][2-hydroxy-2-(4-iodophenyl)propyl]amine in $CH_2Cl_2$ (10 mL) was added dropwise to 0.08 mL DAST in $CH_2CL_2$ (10 mL) while stirring at -78°C under a nitrogen atmosphere. The reaction was allowed to warm to room temperature and diluted with $CH_2Cl_2$ (25 mL). This organic layer was washed with $H_2O$, dried over $Na_2SO_4$, and concentrated under reduced vacuum to yield a yellow oil. This material was purified via silica gel chromatography employing the Chromatotron and using a 2000 micron rotor while eluting with a solvent of hexane/ethyl acetate 3:1 to yield the final title compound (147 mg, 57%) as a white solid; m.p. 115°-117°C. Ion spray M.S. 385 (M*+1).

| Calculated for: $C_{11}H_{16}N_2O_2SI$: | | | |
|---|---|---|---|
| Theory | C 34.21, | H 4.18, | N 7.25. |
| Found | C 34.04, | H 4.41, | N 7.59. |

Example 37

Preparation of 4-[4-(2-{[(dimethylamino)sulfonyl]amino}-1-fluoroisopropyl)phenyl]benzenecarbonitrile.

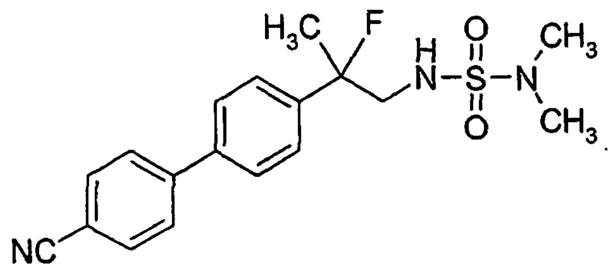

Preparation of 4-[4-(2-{[(dimethylamino)sulfonyl]amino}-1-hydroxyisopropyl)phenyl]benzenecarbonitrile.

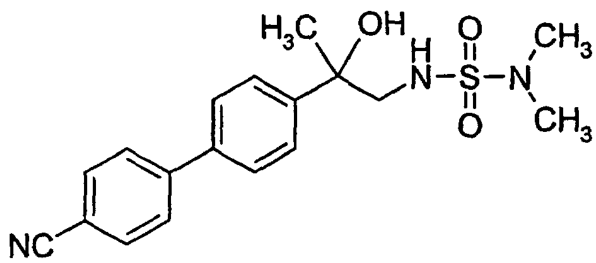

Scheme VI, Step A: [(Dimethylamino)sulfonyl][2-hydroxy-2-(4-iodophenyl)propyl]amine (384 mg, 1.0 mmol, prepared in example 36), 4-cyanobenzene boronic acid (191 mg, 1.3 mmol), potassium carbonate (179 mg, 1.3 mmol), tetrakis(triphenyl phosphine)palladium(0) (58 mg, 0.05 mmol), and dioxane/water (60 mL, 3:1) were mixed together in a 100 mL single neck flask and stirred at 70°C over night. In the morning, the reaction was cooled to room temperature and poured into $H_2O$ and the desired product was extracted with ethyl acetate. The organic layer was backwashed once with $H_2O$, dried over $K_2CO_3$, and concentrated under reduced pressure to yield a viscous oil. This two spot material was purified via silica gel chromatography employing the Chromatotron and using a 2000 micron rotor while eluting with a solvent of methylene chloride/ethyl acetate 9:1 to yield intermediate title compound (255 mg, 71 %, bottom spot) as a white solid. (FD) M.S. 359 (M*).

| Calculated for: $C_{18}H_{21}N_3O_3S$- ¼ $H_2O$ : | | | |
|---|---|---|---|
| Theoryé | C 59.34, | H 5.77, | N 11.54. |
| Found | C 58.89, | H 5.63, | N 11.26. |

Preparation of final title compound.

Scheme VI, Step B: Into a 25 mL 3 neck flask fitted with a stirrer and thermometer, 209 mg of 4-[4-(2-{[(dimethylamino)sulfonyl]amino}-1-hydroxyisopropyl)phenyl]benzenecarbonitrile in $CH_2Cl_2$ (2 mL) was added dropwise to 0.07 mL DAST in $CH_2CL_2$ (3 mL) while stirring at -78°C under a nitrogen atmosphere. The reaction was allowed to warm to room temperature and diluted with $CH_2Cl_2$ (25 mL). This organic layer was washed with $H_2O$, dried over $Na_2SO_4$, and concentrated under reduced vacuum to yield a solid. This material was purified via silica gel chromatography employing the Chromatotron and using a 2000 micron rotor while eluting with a solvent of hexane/ethyl acetate 3:1 to yield the final title compound (132 mg, 63%) as a white solid. (FD) M.S. 361 (M*).

Example 38

Preparation of {2-[4-(3-aminophenyl)phenyl]-2-fluoropropyl)}[(dimethylamino)sulfonyl]amine.

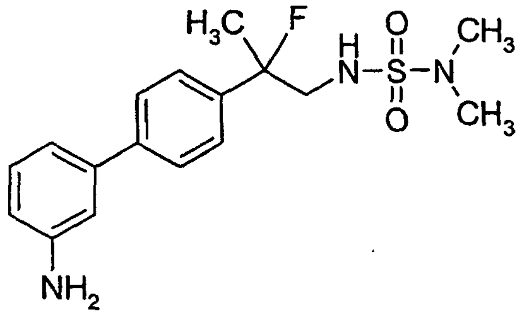

[(Dimethylamino)sulfonyl][2-fluoro-2-(4-iodophenyl)propyl]amine (386, 1.0 mmol, prepared in example 36), 3-aminobenzeneboronic acid (201 mg, 1.3 mmol), potassium carbonate (179 mg, 1.3 mmol), tetrakis(triphenyl phosphine)palladium(0) (58 mg, 0.05 mmol), and dioxane/water (60 mL, 3:1) are mixed together in a 100 mL single neck flask and stirred at 70°C over night. In the morning, the reaction is cooled to room temperature and poured into $H_2O$, and the desired product is extracted with ethyl acetate. The organic layer is backwashed once with $H_2O$, dried over $K_2CO_3$, filtered, and concentrated under reduced pressure. The crude material is purified via silica gel chromatography employing the Chromatotron and using a 2000 micron rotor with hexane/ethyl acetate eluent to provide the title compound.

Example 39

Preparation of [(dimethylamino)sulfonyl][2-fluoro-2-(4-{3-[(methylsulfonyl)amino]phenyl}phenyl)propyl]amine.

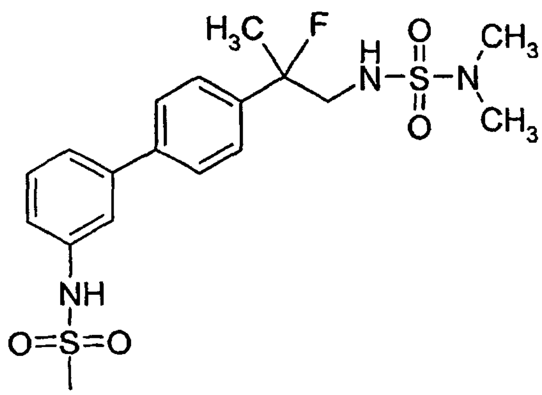

In a 250 mL 3 neck flask fitted with a stirrer and thermometer, 285 mg of methanesulfonyl chloride is added dropwise to 350 mg of {2-[4-(3-aminophenyl)pheny)]-2-fluoropropyl}[(dimethylamino)sulfonyl]amine (prepared in example 38) and 380 mg of DBU in THF (125 mL) while stirring at 0°C under a nitrogen atmosphere. The reaction is allowed to warm to room temperature and then stirred overnight at this temperature. In the morning, the reaction is concentrated under reduced vacuum. The resulting material is taken into ethyl acetate and the organic layer is washed two times with $H_2O$, dried over $Na_2SO_4$, filtered, and concentrated under reduced vacuum. This crude material is purified via silica gel chromatography employing the Chromatotron and using a 4000 micron rotor with hexane/ethyl acetate eluent to provide the title compound.

Example 40

Preparation of [(dimethylamino)sulfonyl]{2-fluoro-2-[4-(3-{[methylethyl)sulfonyl]amino}phenyl)phenyl]propyl}amine.

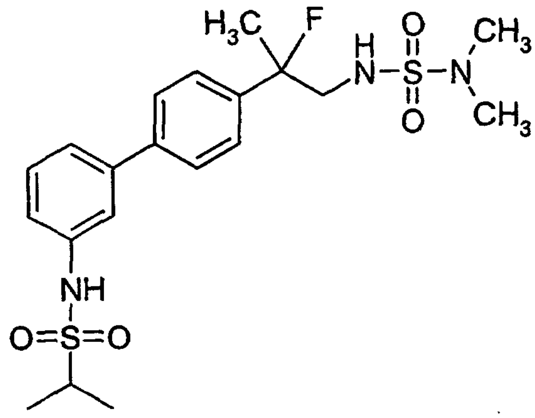

In a 250 mL 3 neck flask fitted with a stirrer and thermometer, 356 mg of 2-propanesulfonyl chloride is added dropwise to 350 mg of {2-[4-(3-aminophenyl)phenyl]-2-fluoropropyl}[(dimethylamino)sulfonyl]amine (prepared in example 38) and 380 mg of DBU in THF (125 mL) while stirring at 0°C under a nitrogen atmosphere. The reaction is allowed to warm to room temperature and then stirred overnight at this temperature. In the morning, the reaction is concentrated under reduced vacuum. The resulting material is taken into ethyl acetate and the organic layer is washed two times with $H_2O$, dried over $Na_2SO_4$, filtered, and concentrated under reduced vacuum. This crude material is purified via silica gel chromatography employing the Chromatotron and using a 4000 micron rotor with hexane/ethyl acetate eluent to provide the title compound.

Example 41

Preparation of 4-[4-(1-fluoro-1-methyl-2-{[(methylethyl)sulfonyl]amino}ethyl)phenyl]benzenesulfonamide.

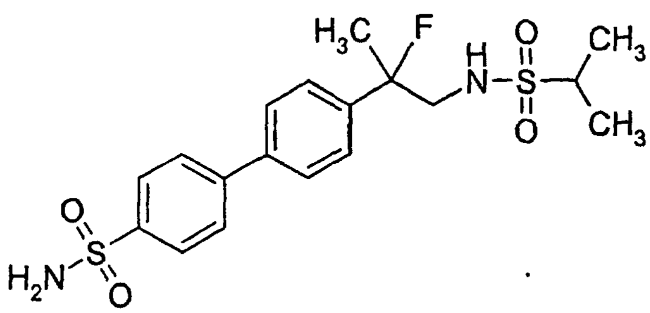

Preparation of 4-bromobenzenesulfonamide.

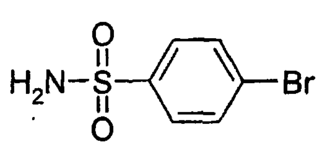

4-Bromobenzenesulfonyl chloride (1.0 g, 3.9 mmol), methanol/ammonia solution (5 mL, excess), and methanol (5 mL) were mixed together in a 25 mL-single neck flask and stirred overnight under a nitrogen atmosphere. In the morning, the reaction was concentrated under reduced vacuum to yield intermediate title compound (1.13 g, 100%) as a white solid. This material was used without further purification. Ion spray M.S. 235.9 (M*).

Preparation of 4-[4-(1-hydroxy-1-methyl-2 {[(methylethyl)sulfonyl]amino}ethyl)phenyl]benzenesulfonamide.

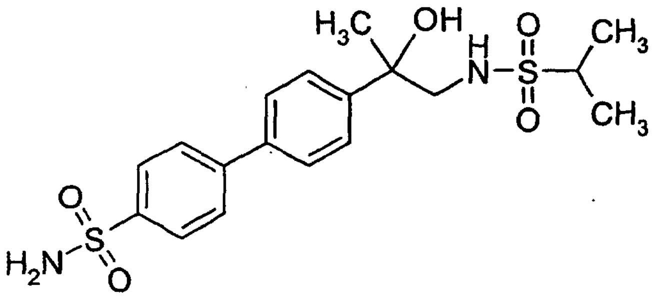

4-bromobenzenesulfonamide (236 mg, 1.0 mmol), the intermediate sulfonyl derivative prepared in example 35 of structure:

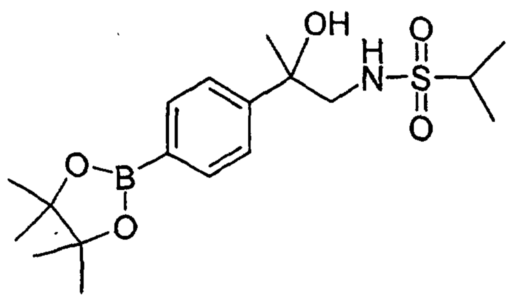

(575 mg, 1.5 mmol), sodium carbonate (1.2 mL of a 2.0 M solution, 1.5 mmol), tetrakis (triphenyl phosphine) palladium(0) (50 mg, 0.65 mmol), and dioxane (20 mL) are mixed together in a 50 mL single neck flask and stirred at 70°C over night. In the morning, the reaction is cooled to room temperature and poured into $H_2O$ and the desired product is extracted with ethyl acetate. The organic layer is backwashed once with $H_2O$, dried over $K_2CO_3$, filtered, and concentrated under reduced pressure. This crude material is purified via silica gel chromatography employing the Chromatotron and using a 4000 micron rotor to provide the title compound.

Preparation of final title compound.

Scheme I, Step B: Into a 50 mL 3 neck flask fitted with a stirrer and thermometer, 412 mg of 4-[4-(1-hydroxy-1-methyl-2-{[(methylethyl)sulfonyl]amino}ethyl)phenyl]benzenesulfonamide in $CH_2Cl_2$ (10 mL) is added dropwise to 0.14 mL DAST in $CH_2CL_2$ (10 mL) while stirring at -78°C under a nitrogen atmosphere. The reaction is allowed to warm to room temperature and diluted with $CH_2Cl_2$ (25 mL). This organic layer is washed with $H_2O$, dried over $Na_2SO_4$, filtered, and concentrated under reduced vacuum. This crude material is purified via silica gel chromatography employing the Chromatotron and using a 4000 micron rotor with hexane/ethyl acetate eluent to provide the title compound.

Example 41A

Preparation of 4-[4-((1S)-1-fluoro-1-methyl-2-{[(methylethyl)sulfonyl]amino}ethyl)phenyl]benzenesulfonamide.

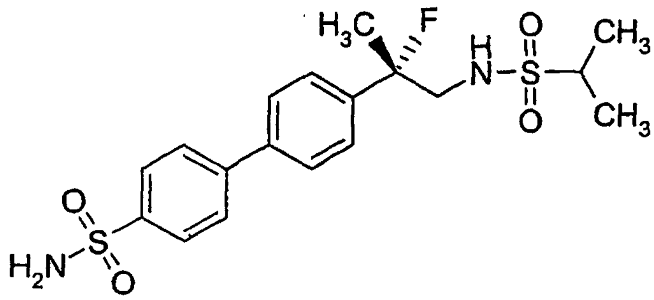

Into a 500 mL single neck flask fitted with a stirrer was placed (+)-[2-fluoro-2-(4-iodophenyl)propyl][(methylethyl)sulfonyl]amine (3.42 g, 8.87 mmol, see example 1a), pinacolato diboron (2.40 g, 9.45 mmol), and potassium acetate (2.58 g, 3 equivalents) in DMF (180 mL). This mixture was stirred at room temperature and de-gassed with argon for 10 minutes. Then, $PdCl_2$(dppf) (180 mg) was added portion wise and the reaction was stirred at 80°C under a nitrogen atmosphere for 2 hours. The dark mixture was allowed to cool to room temperature and 4-bromo-phenylsulfonamide (3.97 g, 16.8 mmol) and an additional amount of $PdCl_2$(dppf) (180 mg) was added portion wise. This was followed by the addition of 21.6 mL of 2.0 M sodium carbonate/water syringe wise. The mixture was then heated to 80°C and stirred overnight. In the morning, the reaction was allowed to cool to room temperature and poured into water. The desired material was extracted into ethyl acetate. The organic layer was washed once with water, dried over potassium carbonate, filtered, and concentrated under reduced vacuum to yield 6.41 g of crude material (2 spots by thin layer chromatography, TLC) as a dark oil. This material was separated and purified via silica gel chromatography employing the Water's Prep. 2000 and eluting with an isocratic solvent of methylene chloride/ethyl acetate (9:1) to provide the title compound (2.47 g, 67%, lower spot by TLC) as a white solid. Melting point was 119.5-121°C; Ion spray M.S. 413 (M* - 1):
Rotation = +18.69 in methanol at room temperature at Sodium D.

| Calculated for: $C_{18}H_{23}N_2O_4S_2F$: | | | |
|---|---|---|---|
| Theory | C 52.16, | H 5.59, | N 6.76 |
| Found | C 52.37, | H 5.96, | N 6.19 |

Example 41B

Preparation of 4-[4-((1R)-1-fluoro-1-methyl-2-{[(methylethyl)sulfonyl]amino}ethyl)phenyl]benzenesulfonamide.

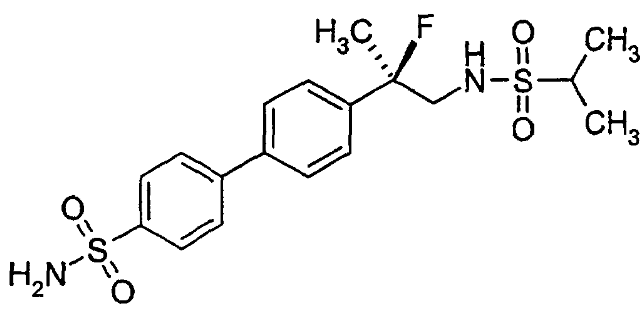

The title compound can be prepared in a manner analogous to the procedure set forth in example 41A from

(-)-[2-fluoro-2-(4-iodophenyl)propyl][(methylethyl)sulfonyl]amine and 4-bromo-phenylsulfonamide.

Example 42

Preparation of N-(2-{3-Fluoro-4'-[1-fluoro-1-methyl)-2-(propane-2 sulfonylamino)-ethyl]-biphenyl-4-yl}-ethyl)-isobutyramide.

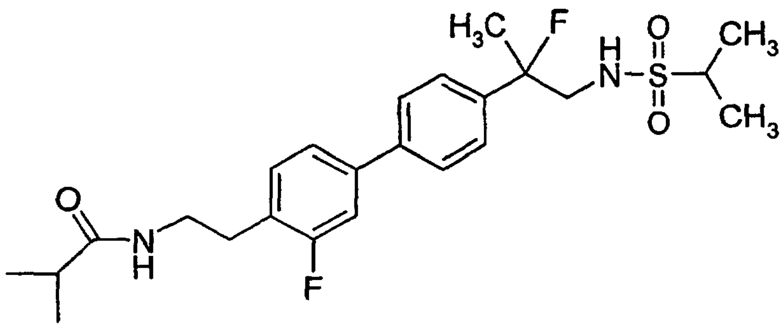

Preparation of (4-Bromo-2-fluoro-phenyl)-acetonitrile.

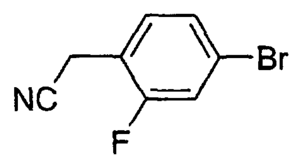

Sodium cyanide (1.37g, 28.00 mmol) was added to a stirred solution of 2-fluoro-4-bromo benzyl bromide (5.0 g, 18.66 mmol) in dry DMSO (60 mL) at ambient temperature under nitrogen. The reaction was stirred for 4 h at ambient temperature then poured into $H_2O$ (150 mL) and extracted with EtOAc. The organic layer was washed with brine, dried ($MgSO_4$), filtered, and concentrated *in vacuo* to give the intermediate title compound as a brown oil (3.85 g, 96%). Electrospray mass spectrum: M = 213, M+2 = 215

Preparation of 1-[4-(4,4,5,5-Tetramethyl-[1,3,2]dioxaborolan-2yl)phenylethanone.

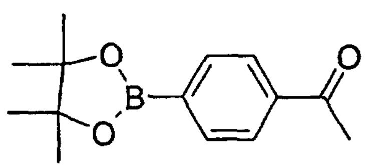

4-Iodo acetophenone (10.00 g, 40.64 mmol), $PdCl_2$(dppf).$CH_2Cl_2$ (1.00 g , 1.22 mmol) dry dioxane (160 mL) and $Et_3N$ (17.0 mL, 121.96 mmol) were combined in a dry flask which had been purged with $N_2$ several times. Pinacol borane (8.80 mL, 60.96 mmol) was added and the reaction mixture was heated at reflux for 4 h then cooled to ambient temperature, poured into Diethyl ether (250 mL) and extracted with $H_2O$ and washed with brine. The Diethyl ether was dried ($MgSO_4$), filtered and the filtrate concentrated *in vacuo.* Purification by gradient chromatography on silica (hexane, 5% EtOAc/hexane) followed by recrystallization (hexane) gave 6.4 g (64%) of the intermediate title compound as a white powder.

Preparation of 2-[4(4,4,5,5-Tetramethyl-[1,3,2] dioxaborolan-2-yl)-phenyl]-2-trimethylsilanyloxy-propionitrile.

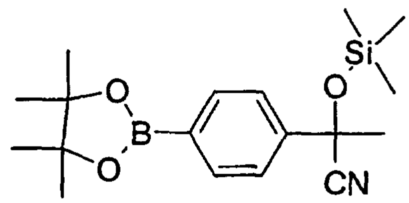

Scheme Va, Step A: 1-[4-(4,4,5,5-Tetramethyl-[1,3,2] dioxaborolan-2yl)phenyl-ethanone (1.0 g, 4.06 mmol) dissolved in THF(1.0 mL) was added to $ZnI_2$ (0.131 g,0.41 mmol). To this stirred mixture under $N_2$ was added dropwise TMSCN (1.6 mL, 12.18 mmol) and the reaction mixture was stirred at ambient temperature overnight. The resulting mixture was extracted with $NaHCO_3$, $H_2O$, washed with brine, dried ($MgSO_4$), filtered, and concentrated *in vacuo* to give the intermediate title compound as a yellow oil 0.91g (65%).

Preparation of 1-Amino-2-[4( 4,4,5,5-tetramethyl-[1,3,2]dioxaborolan-2-yl)-phenyl]-propan-2-ol hydrochloride.

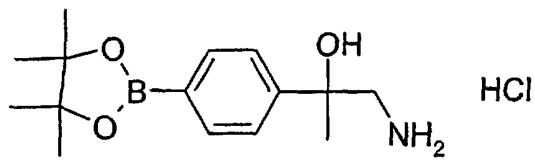

Scheme Va, Step B: Borane dimethyl sulfide 2.0 M (4.0 mL, 8.0 mmol) was added dropwise to 2-[4(4,4,5,5-tetramethyl-[1,3,2] dioxaborolan-2-yl)-phenyl]-2-trimethylsilanyloxy- propionitrile (0.910 g, 2.64 mmol) in dry THF (20.0mL) at ambient temperature. After the initial exotherm, the reaction mixture was heated and stirred at reflux for 4 h cooled to ambient temperature and quenched by a slow addition of concentrated HCl. The reaction was diluted with Diethyl ether and stirred for approximately 1 h. The resulting white precipitate was collected by filtration and used in the next reaction.

Preparation of N-{2-Hydroxy-2-[4(4,4,5,5-tetramethyl-[1,3,2] dioxaboralan-2-yl)phenyl]propyl}-2-propanesulfonamide.

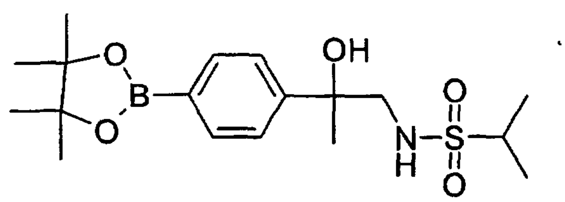

Isopropyl sulfonamide (0.313g, 2.2 mmol) is added to 1-Amino-2-[4(4,4,5,5-tetramethyl-[1,3,2]dioxaborolan-2-yl)-phenyl]-propan-2-ol hydrochloride (0.627 g, 2.0 mmol) in $CH_2Cl_2$ (20 mL) at 0°C. Triethylamine (0.505 g, 5.0 mmol) is then added and the resulting mixture is stirred for 4 h at 0°C. The reaction mixture is extracted with $H_2O$, washed with brine, dried over magnesium sulfate, filtered, and concentrated *in vacuo.* The residue is purified by chromatography on silica gel with EtOAc/hexane to provide the intermediate title compound.

Preparation of N-[2-(4'-cyanomethyl-3'-fluoro-biphenyl-4yl)-2-hydroxy-propyl]-2-propanesulfonamide.

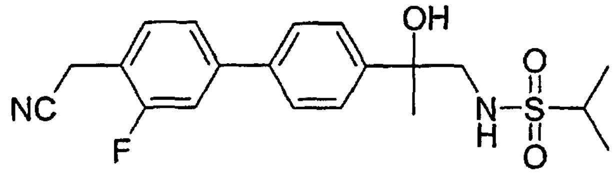

(4-Bromo-2-fluoro-phenyl)-acetonitrile (0.255 g, 1.19 mmol), $PdCl_2(dppf).CH_2Cl_2$ (.039 g, .048 mmol) and N-{2-hydroxy-2-[4(4,4,5,5-tetramethyl-[1,3,2] dioxaboralan-2-yl)phenyl]propyl}-2-propanesulfonamide (0.383g, 1.0 mmol) are mixed together in dry DMF (30 mL) under $N_2$ at ambient temperature. To this stirred mixture is added 2M $Na_2CO_3$ (1.25 mL, 2.5 mmol) and the resulting mixture is heated and stirred at 80° C for 6h. The mixture is cooled and poured into EtOAc. The EtOAc is extracted several times with $H_2O$, washed with brine, dried ($MgSO_4$), filtered, and the filtrate evaporated *in vacuo.* Chromatography on silica gel eluting with EtOAc/hexane gives the intermediate title compound.

Preparation of N-2-[4-(4'-Amino ethyl-3'-fluorophenyl)phenyl]2-hydroxy-propyl-2-propanesulfonamide.

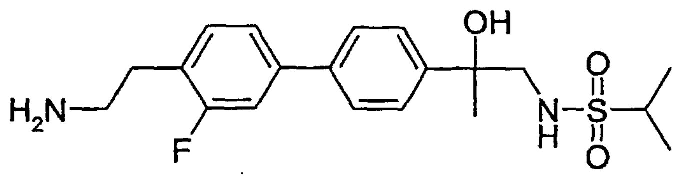

Borane dimethyl sulfide (3.0 mL, 6.0 mmol) is added to N-[2-(4'-cyanomethyl-3'-fluoro-biphenyl-4yl)-2-hydroxy-propyl]-2-propanesulfonamide (0.78 g, 2.0 mmol) in THF (20mL) and the resulting solution is heated and stirred at reflux for 4h. The resulting mixture is cooled and acidified cautiously with concentrated HCl. Collection of the precipitated solid by filtration yields the intermediate title compound.

Preparation of N-(2-{3-Fluoro-4'-[1-hydroxy-1-methyl-2-(propane-2-sulfonylamino)-ethyl]-biphenyl-4yl}-ethyl)-isobutyramide.

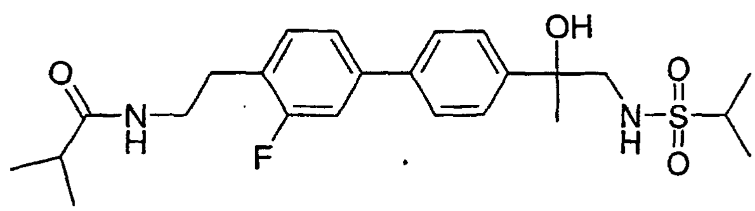

To N-2-[4-(4'-amino ethyl-3'-fluorophenyl)phenyl]2-hydroxy-propyl-2-propanesulfonamide (0.104 g, 0.26 mmol) in dry $CH_2Cl_2$ in a 12 mL vial is added $Et_3N$ (81 μL, 0.50 mmol), and isobutyryl chloride (34 μL, 0.32 mmol) respectively. The reaction vial is stirred overnight at ambient temperature. The resulting mixture is diluted with $CH_2Cl_2$, washed successively with 1M HCl, saturated $NaHCO_3$, brine, dried ($MgSO_4$), filtered, and concentrated to give the intermediate title compound.

Preparation of final title compound.

Scheme I, Step B: To DAST (26μL, 0.20mmol) in $CH_2Cl_2$(.3 mL) under $N_2$ at -78° C is added dropwise N-(2-{3-fluoro-4'-[1-hydroxy-1-methyl-2-(propane-2-sulfonylamino)-ethyl]-biphenyl-4yl}-ethyl)-isobutyramide (0.0928 g, 0.20 mmol) in $CH_2Cl_2$ (1.0 mL). Following addition the dry ice acetone-cooling bath is replaced with an ice water bath and the reaction mixture allowed to warm to ambient temperature. The reaction mixture is diluted with $CH_2Cl_2$, extracted with $H_2O$, dried ($Na_2SO_4$), filtered, and the filtrate is evaporated to dryness to give the title compound.

Example 43

Preparation of N-(2-{3-Fluoro-4'-[1-fluoro-1-methyl-2-(propane-2-sulfonylamino)-ethyl]-biphenyl-4-yl}-ethyl)-acetamide.

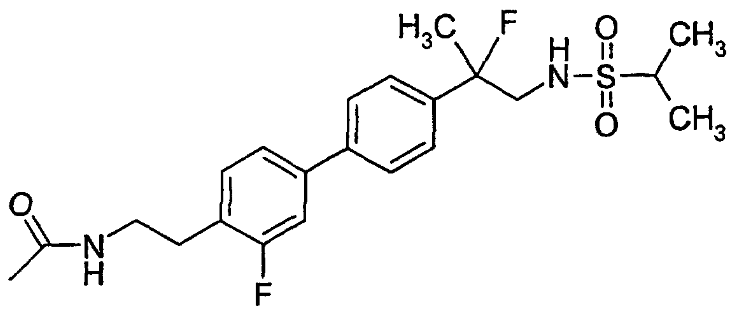

Preparation of N-(2-{3-Fluoro-4'-[1-hydroxy-1-methyl-2-(propane-2-sulfonylamino)ethyl]-biphenyl-4yl}-ethyl)-acetamide.

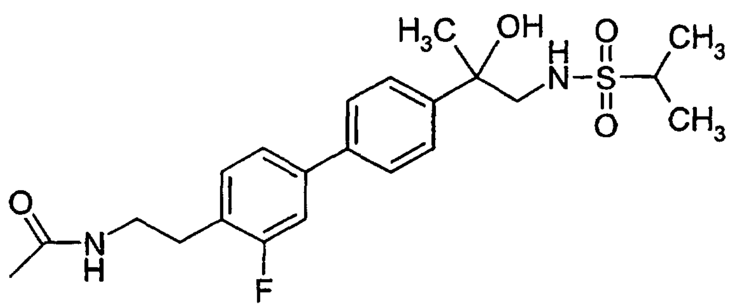

To N-2-[4-(4'-Amino ethyl-3'-fluorophenyl)phenyl]2-hydroxy-propyl-2-propanesulfonamide (0.104g, 0.26 mmol, intermediate prepared in example 42), in dry $CH_2Cl_2$ in a 12 mL vial is added $Et_3N$ (81 µL, 0.50 mmol) and acetyl chloride (23 µL, 0.32 mmol) respectively. The reaction vial is stirred overnight at ambient temperature. The resulting mixture is diluted with $CH_2Cl_2$, washed successively with 1M HCl, saturated $NaHCO_3$, brine, dried ($MgSO_4$), filtered, and concentrated to provide the intermediate title compound.

Preparation of final title compound.

Scheme I, Step B: To DAST (26 µL, 0.20 mmol) in $CH_2Cl_2$(0.3 mL) under $N_2$ at-78°C is added dropwise N-(2-{3-fluoro-4'-[1-hydroxy-1-methyl-2-(propane-2-sulfonylamino)ethyl]-biphenyl-4yl}-ethyl)acetamide (.087 g. 0.20 mmol) in $CH_2Cl_2$(1.0 mL). Following addition the dry ice acetone-cooling bath is replaced with an ice water bath and the reaction mixture allowed to warm to ambient temperature. The reaction mixture is diluted with $CH_2Cl_2$, extracted with $H_2O$, dried ($Na_2SO_4$), filtered, and the filtrate is evaporated to dryness to give the final title compound.

Example 44

Preparation of N-{2-(2-Fluoro-2-[3'-fluoro-4'-(2-methanesulfonylamino)-ethyl)-biphenyl-4-yl]propyl}-2-propane sulfonamide.

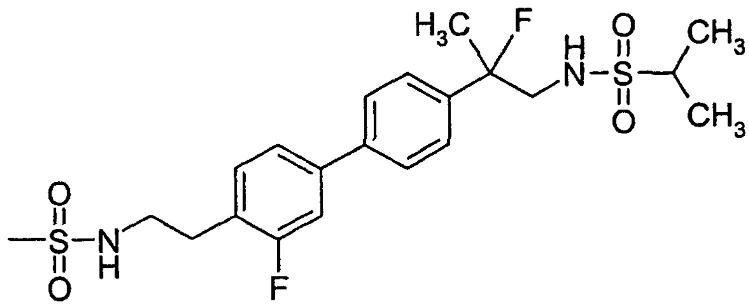

Preparation of N-(2-{3'-Fluoro-4'-[2-methanesulfonylamino)-ethyl]-biphenyl}-2-hydroxy-propyl)-2-propanesulfonamide.

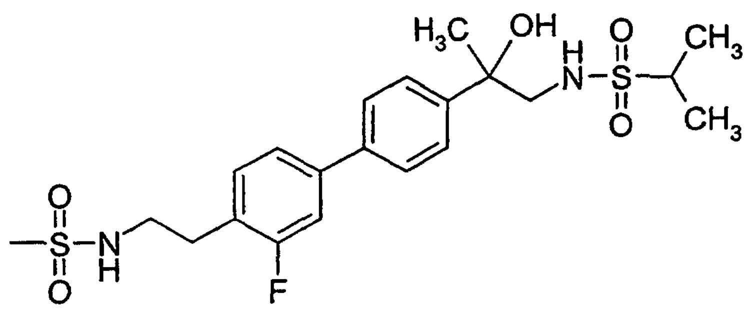

N-2-[4-(4'-Amino ethyl-3'-fluorophenyl)phenyl]2-hydroxy-propyl-2-propanesulfonamide (0.104 g, 0.26 mmol, prepared in example 42), $Et_3N$ (81 µL, 0.50 mmol) and methane sulfonyl chloride (23 µL, 0.30 mmol) are combined with methylene chloride in a 12 mL vial. The reaction vial is stirred overnight at ambient temperature. The resulting mixture is diluted with $CH_2Cl_2$, washed successively with 1M HCl, saturated $NaHCO_3$, brine, dried ($MgSO_4$), filtered, and concentrated to provide the intermediate title compound.

Preparation of final title compound.

Scheme I, Step B: To a solution of DAST (26 µL, 0.20 mmol) in $CH_2Cl_2$ (0.3 mL) under $N_2$ is added dropwise N-(2-{3'-fluoro-4'-[2-methanesulfonylamino)-ethyl]-biphenyl}-2-hydroxy-propyl)-2-propanesulfonamide (0.094g, 0.20 mmol) in $CH_2Cl_2$ (1.0mL) at -78° C. Following addition the dry ice acetone-cooling bath is replaced with an ice water bath and the reaction mixture allowed to warm to ambient temperature. The reaction mixture is diluted with $CH_2Cl_2$, extracted with $H_2O$, dried ($Na_2SO_4$), filtered, and the filtrate is evaporated to dryness to give the final title compound.

Example 45

Preparation of N-{2-(2-Fluoro-2-[3'-fluoro-4'-(2-(propane-2-sulfonylamino)-ethyl)-biphenyl-4-yl]propyl}-2-propane sulfonamide.

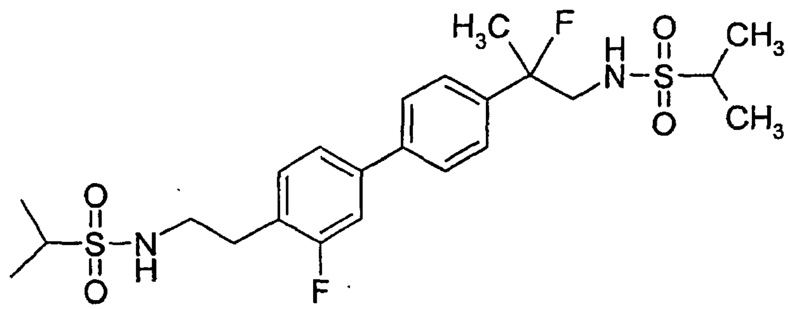

Preparation of N-(2-{3'-fluoro-4'-[2-(propane-2-sulfonylamino)-ethyl]-biphenyl-4-yl}-2-hydroxy-propyl)-2-propane sulfonamide.

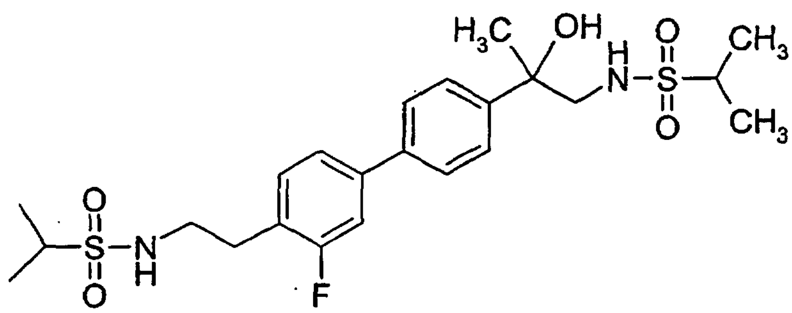

N-2-[4-(4'-Amino ethyl-3'-fluorophenyl)phenyl]2-hydroxy-propyl-2-propanesulfonamide (0.104 g, 0.26 mmol, prepared in example 42), $Et_3N$ (81 µL, 0.50 mmol) and isopropyl sulfonyl chloride (34 µL, 0.30 mmol) are combined with methylene chloride in a 12 mL vial. The reaction vial is stirred overnight at ambient temperature. The resulting mixture is diluted with $CH_2Cl_2$, washed successively with 1M HCl, saturated $NaHCO_3$, brine, dried ($MgSO_4$), filtered, and concentrated to provide the intermediate title compound.

Preparation of final title compound.

Scheme I, Step B: To a solution of DAST (26 µL, 0.20 mmol) in $CH_2Cl_2$ (0.3 mL) under $N_2$ is added dropwise N-(2-{3'-fluoro-4'-[2-(propane-2-sulfonylamino)-ethyl]-biphenyl}-2-hydroxy-propyl)-2-propanesulfonamide (0.100 g, 0.20 mmol) in $CH_2Cl_2$ (1.0 mL) at -78°C. Following addition the dry ice acetone-cooling bath is replaced with an ice water bath and the reaction mixture allowed to warm to ambient temperature. The reaction mixture is diluted with $CH_2Cl_2$, extracted with $H_2O$, dried ($Na_2SO_4$), filtered, and the filtrate is evaporated to dryness to give the final title compound.

Example 46

Preparation of N-{2-fluoro-2-[4(2-oxo-2,3-dihydro-1H-indolo-5 yl)-phenyl]-propyl}-2-propane sulfonamide.

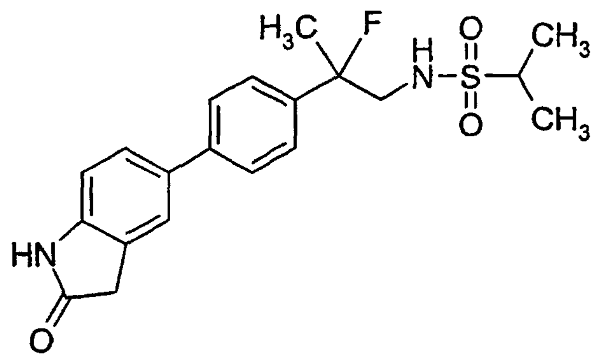

Preparation of 5-Bromo-oxindole.

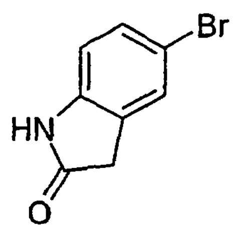

Oxindole (1.30 g, .010 mmol) was suspended in dry acetonitrile (22.0mL) under $N_2$ and cooled to -10° C. To this stirred mixture was added recrystallized NBS (2.00 g, 0.011 mol) portionwise. The resulting suspension was stirred at -10 to 10° C for 3 h. The precipitated solid was collected by filtration, washed with $H_2O$ and dried to give the intermediate title compound as an off white solid 1.75g (82%). Electrospray mass spectrum (M-1 )= 210, (M-1) = 211

Preparation of N-{2-Hydroxy-2-{4(2-oxo-2,3-dihydro-1H-indolo-5 yl)-phenyl]-propyl}-2-propane sulfonamide.

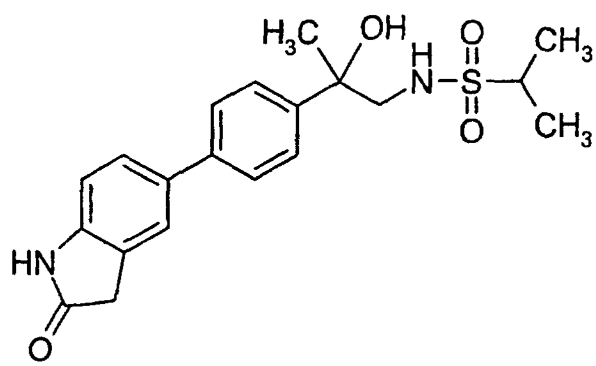

To a stirred mixture of N-{2-hydroxy-2-[4(4,4,5,5-tetramethyl-[1,3,2] dioxaboralan-2-yl)phenyl]propyl}-2-propanesulfonamide (0.383 g, 1.0 mmol, intermediate prepared in example 42), $PdCl_2$(dppf). $CH_2Cl_2$ (.033 g, .04 mmol) and 5-bromo-oxindole (0.276 g, 1.3 mmol) in DMF (20 mL) under $N_2$ is added 2M $Na_2CO_3$ (1.35 mL, 2.7 mmol) and the reaction mixture is heated and stirred under $N_2$ for 6 h. The reaction mixture is allowed to warm to ambient temperature and poured into EtOAc (100 mL). The organic layer is washed with $H_2O$ and brine, dried ($MgSO_4$) filtered and the filtrate evaporated *in vacuo.* Chromatography on silica gel with EtOAc/hexane gives the intermediate title compound.

Preparation of final title compound.

Scheme I, Step B: To a stirred solution of DAST (26 µL, 0.20 mmol) in (0.3 mL) of $CH_2Cl_2$ under $N_2$ at -78° C is added N-{2-hydroxy-2-[4(2-oxo-2,3-dihydro-1H-indolo-5 yl)-phenyl]-propyl}- 2- propane sulfonamide (0.077 g, 0.20 mmol). Following addition the dry ice acetone-cooling bath is replaced with an ice water bath and the reaction mixture allowed to warm to ambient temperature. The reaction mixture is diluted with $CH_2Cl_2$, extracted with $H_2O$, dried ($Na_2SO_4$), filtered, and the filtrate is evaporated to dryness to provide the final title compound.

Example 47

Preparation of {4'-[1-Fluoro-1-methyl-2-(propane-2-sulfonylamino)-ethyl]-3-nitrobiphenyl-4-yl}-acetic acid ethyl ester.

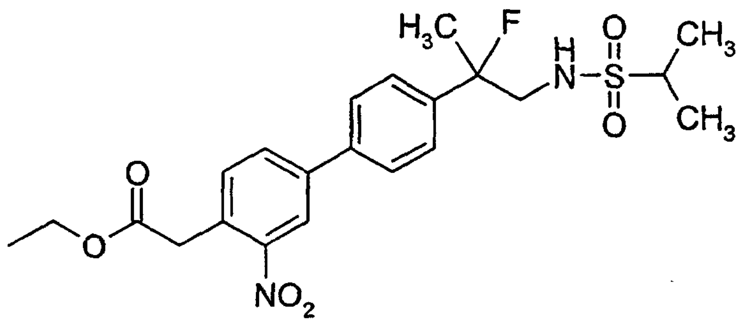

Preparation of 2-(4-Bromo-2-nitro-phenyl)-malonic acid diethyl ester.

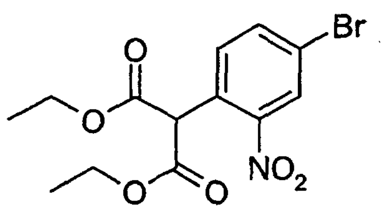

Diethyl malonate (3.34 mL, 22 mmol) was added cautiously to a stirred suspension of 60 %NaH (0.96 g, 24 mmol) in dry DMF (50 mL) at -10° C under $N_2$. The resulting mixture was stirred at -10 to 10° C for 1 h. To this mixture at 0° C was added 2,4-dibromo-nitrobenzene (3.34 mL, 0.022 mol) portionwise. The mixture was allowed to warm to ambient temperature and stirred overnight. Evaluation by TLC (EtOAc-hexane 4:1) showed only a trace of starting material. The reaction mixture was poured into a two-phase Diethyl ether-1M HCl mixture. The yellow Diethyl ether layer was dried ($MgSO_4$), filtered, and evaporated *in vacuo.* Chromatography on silica gel, eluting with EtOAc-hexane 5:95 gave a yellow liquid which rapidly solidified to give the intermediate title compound (2.50 g, 69%), mp 54-55° C.

Preparation of (4-Bromo-2-nitro-phenyl)-acetic acid ethyl ester.

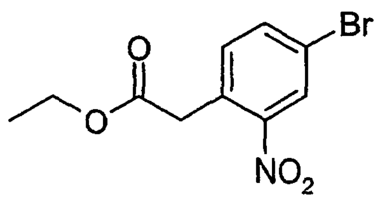

To a stirred solution of concentrated HCl (18.00 mL) diluted to 72.00 mL with 95% Ethanol was added 2-(4-bromo-2-nitro-phenyl)-malonic acid diethyl ester (2.50 g, 6.94 mmol). The resulting mixture was then heated at reflux under $N_2$ for 4 h. The reaction mixture was allowed to cool to ambient temperature and poured into $H_2O$ (250 mL). This aqueous mixture was then extracted with 2 x 150 mL of Diethyl ether. The $Et_2O$ layer was extracted with $H_2O$ and 5% $NaHCO_3$ washed with brine, dried ($MgSO_4$), and filtered. Evaporation of the filtrate *in vacuo* gave the intermediate title compound as a yellow liquid (1.52 g, 76%).

Preparation of {4'[1-Hydroxy-1-methyl-2-(propane-2-sulfonylamino)-ethyl]-3-nitro-biphenyl-4-yl}- acetic acid ethyl ester.

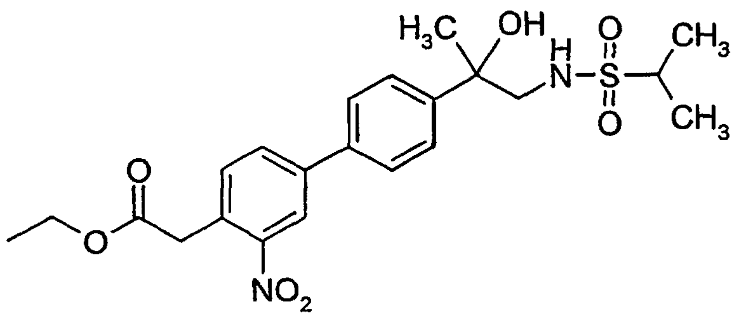

$PdCl_2$(dppf).$CH_2Cl_2$ (.077 g, 0.90 mmol) and N-{2-hydroxy-2-[4(4,4,5,5-tetramethyl-[1,3,2] dioxaboralan-2-yl) phenyl]propyl}-2-propanesulfonamide (0.383, 1.0 mmol, intermediate prepared in example 42) are mixed together in a dry flask under $N_2$ followed by (4-bromo-2-nitro-phenyl)-acetic acid ethyl ester (0.300 g, 1.04 mmol) , $CsCO_3$ (0.68 g, 2.09 mmol) and toluene(65 mL) respectively. The resulting mixture is heated and stirred at 96° C for 5h. The reaction mixture is allowed to cool to ambient temperature and is stirred overnight. The toluene is diluted with EtOAc (100mL) and filtered through celite. The resulting filtrate is extracted with 2x 200 mL of $H_2O$, washed with brine, dried ($MgSO_4$), and filtered. The filtrate is evaporated and chromatographed on silica gel, eluting with EtOAc- hexane to give the intermediate title compound.

Preparation of final title compound.

Scheme I, Step B: To a solution of DAST (118 µL, 0.998 mmol) in $CH_2Cl_2$ (0.8 mL) under $N_2$ at -78° C is added {4'-[1-hydroxy-1-methyl-2-(propane-2-sulfonylamino)-ethyl)-3-nitro-biphenyl-4-yl}-acetic acid ethyl ester (0.465 g, 1.00 mmol) in $CH_2Cl_2$ (3.0 mL). Following addition the dry ice acetone-cooling bath is replaced with an ice water bath and the reaction mixture allowed to warm to ambient temperature. The reaction mixture is diluted with $CH_2Cl_2$, extracted with $H_2O$, dried ($Na_2SO_4$), filtered, and the filtrate is evaporated to dryness to provide the final title compound.

Example 48

Preparation of {4'-[1-Fluoro-1-methyl-2-(propane-2-sulfonylamino)-ethyl]-3-nitrobiphenyl-4-yl}- acetic acid.

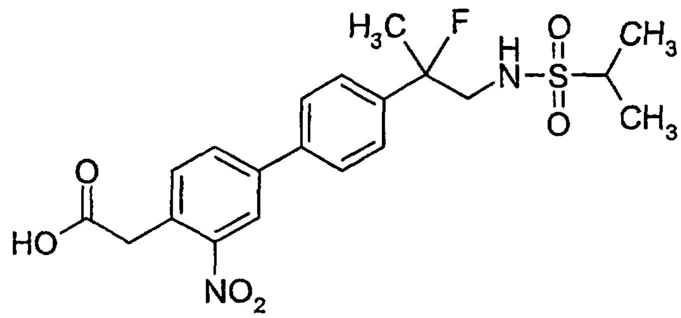

To a solution of {4'-[1-fluoro-1-methyl-2-(propane-2-sulfonylamino)-ethyl]-3-nitro-biphenyl-4-yl}- acetic acid ethyl ester. (0.341 g, 0.731 mmol, prepared in example 47) in Ethanol (5.0 mL) is added a solution of 6.7% NaOH (15.0 mL) and the resulting mixture is stirred under $N_2$ at ambient temperature for 72 h. The mixture is diluted with $H_2O$ (100mL) and acidified with 37% HCl. Extraction with EtOAc followed by drying ($MgSO_4$), filtration, and evaporation of the filtrate *in vacuo* gives the title compound.

Example 49

Preparation of N-{2-Fluoro-2-[4-(2-oxo-2,3-dihydro-1H-indol-6-yl)-phenyl]-propyl)-2-propanesulfonamide.

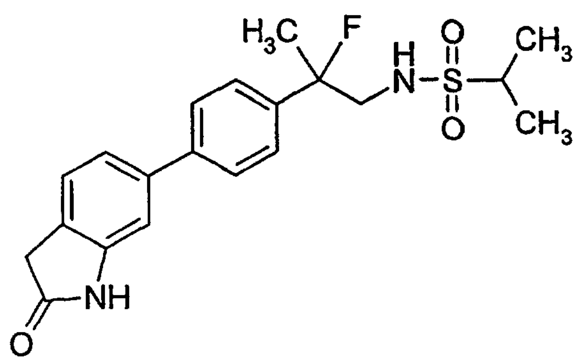

To a stirred solution of {4'-[1-fluoro-1-methyl-2-(propane-2-sulfonylamino)-ethyl]-3-nitro-biphenyl-4-yl}-acetic acid (0.311 g, 0.71 mmol, prepared in example 48) dissolved in Ethanol (4.0 mL)/50% $H_2SO_4$ (3.0 mL) at ambient temperature then heated to 90° C is added Zn ( 0.186 g, 2.84 mmol) in divided portions over 30 minutes. Heating and stirring is continued for 2 h after addition of the Zn. The mixture is allowed to cool to ambient temperature. It is then extracted (EtOAc) and the EtOAc is extracted with 5% $NaHCO_3$, washed with brine, dried ($MgSO_4$), filtered and the filtrate concentrated *in vacuo*. Chromatography of the residue (silica gel, EtOAc/hexane) provides the title compound.

Example 50

Preparation of Methyl 6-{N-[4-(1-fluoro-1-methyl-2-{[(methylethyl)sulfonyl]amino}ethyl)phenyl]carbamoyl}pyridine-3-carboxylate.

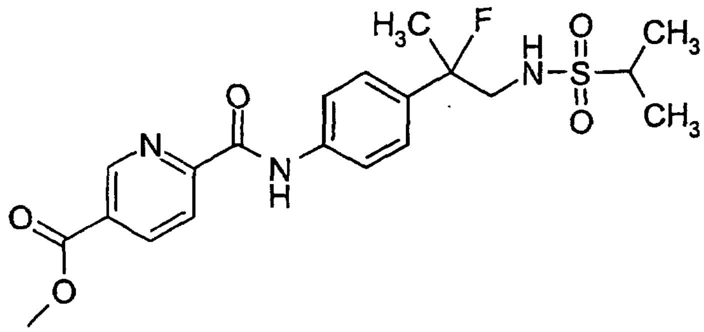

Preparation of Methyl 6-{N-[4-(1-hydroxy-1-methyl-2-{[(methylethyl)sulfonyl]amino}ethyl)phenyl]carbamoyl}pyridine-3-carboxylate.

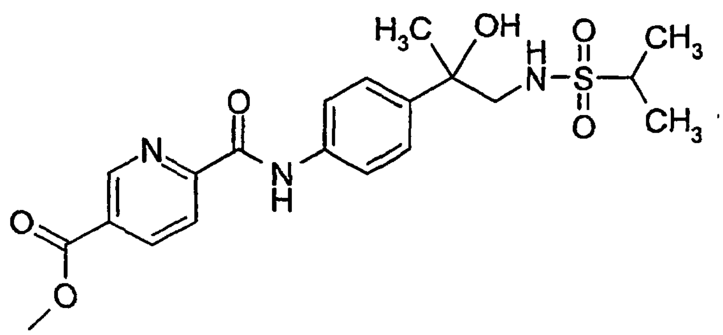

Scheme XII, Step A: Into a 50 mL single neck flask, 1 mL oxalyl chloride was added syringe wise to 5-(methoxycarbonyl)-pyridine-2-carboxylic acid (271 mg, 1.5 mmol) in methylene chloride (10 mL) while stirring under nitrogen at room temperature. Immediately, 1 drop of DMF was added by pipette initiating a foaming of the mixture. The reaction was stirred one hour at this temperature and then concentrated under reduced vacuum to yield a white semi-solid. This material was placed into THF (5 mL) and added dropwise to a stirring solution of [2-(4-aminophenyl)-2-hydroxypropyl][(methylethyl)sulfonyl]amine (400 mg, 1.5 mmol, intermediate prepared in example 19) and triethlyamine (152 mg) in THF (20 mL) at room temperature and the mixture was stirred overnight. In the morning, the solution was concentrated under reduced vacuum and the resulting oil was taken into methylene chloride and the organic layer was washed once with $H_2O$, dried over $K_2CO_3$, filtered, and concentrated under reduced vacuum to yield 244 mg as an orange solid. This material was purified via silica gel chromatography employing the chromatotron and using a 4000 micron rotor while eluting with a solvent of ethyl acetate to yield the intermediate title compound (165 mg, 25%) as a yellow solid. Ion spray M.S. 434.2 (M*-1).

| Calculated for $C_{20}H_{25}N_3O_6S$-$H_2O$: | | | |
|---|---|---|---|
| Theory | C 52.95, | H 6.00, | N 9.27. |
| Found | C 53.14, | H 5.67, | N 9.01. |

Preparation of final title compound.

Scheme XII, Step B: Into a 50 mL, 3 necked flask fitted with a stirrer and thermometer, 200 mg of methyl 6-{N-[4-(1-hydroxy-1-methyl-2-{[(methylethyl)sulfonyl]amino}ethyl)phenyl]carbamoyl}pyridine-3-carboxylate in methylene chloride (5 mL) is added dropwise to 0.06 mL DAST in methylene chloride (5 mL) while stirring at -78°C under a nitrogen atmosphere. Reaction is allowed to warm to room temperature and diluted with methylene chloride (25 mL). This organic layer is washed with $H_2O$, dried over $Na_2SO_4$, filtered, and concentrated under reduced vacuum to yield a slowly crystallizing yellow oil (80% yield). This material was used without further purification. Ion spray M.S. 436.3 (M*-1).

| Calculated for $C_{20}H_{24}N_3O_5SF$: | | | |
|---|---|---|---|
| Theory | C 54.91, | H 5.53, | N 9.60. |
| Found | C 54.10, | H 5.66, | N 9.12. |

Example 51

Preparation of 6-{N-[4-(1-Fluoro-1-methyl-2-{[(methylethyl)sulfonyl]amino}ethyl)phenyl]carbamoyl}pyridine-3-carboxylic acid.

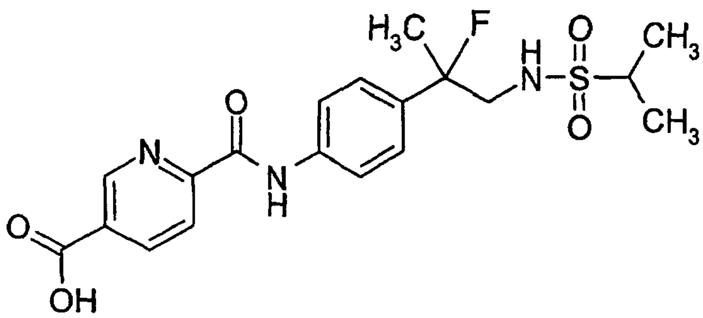

Methyl 6-{N-[4-(1-fluoro-1-methyl-2-{[(methylethyl)sulfonyl]amino}ethyl)phenyl]carbamoyl}pyridine-3-carboxylate (150 mg, 0.35 mmol, prepared in example 50), lithium hydroxide (52 mg, 1.24 mmol), tetrahydrofuran (6 mL), methanol (2 mL), and water (2 mL) were mixed together in a 25 mL 3 neck flask and stirred over night at room temperature. In the morning, the mixture was concentrated under reduced vacuum to yield a white solid. This material was taken into 1N HCl and the desired material was extracted into methylene chloride. The acidic solution was taken to pH 10 with 1N NaOH and the resulting precipitate was again extracted with methylene chloride. Both organic layers were combined and washed once with $H_2O$, dried over $MgSO_4$, filtered, and concentrated under reduced vacuum to yield the title compound (125 mg, 84%) as a tan solid. This material was used without further purification. Ion spray M.S. 403.9 (M* - 19 Fluorine).

Example 52

Preparation of N-[4-(1-Fluoro-1-methyl-2-{[(methylethyl)sulfonyl]amino}ethyl)phenyl][5-(N-methylcarbamoyl)(2-pyridyl)]carboxamide.

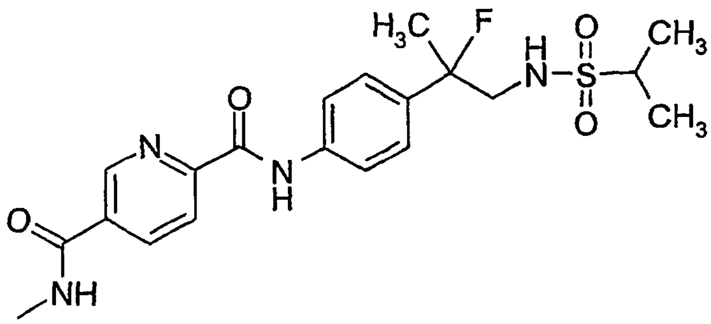

Into a 50 mL single neck flask, 1 mL oxalyl chloride was added syringe wise to 6-{N-[4-(1-fluoro-1-methyl-2-{[(methylethyl)sulfonyl]amino}ethyl)phenyl]carbamoyl}pyridine-3-carboxylic acid. (110 mg, 0.26 mmol, prepared in example 51) in methylene chloride (10 mL) while stirring under nitrogen at room temperature. Immediately, 1 drop of DMF was added by pipette initiating a foaming of the mixture. The reaction was stirred one hour at this temperature and then concentrated under reduced vacuum to yield a white semi-solid. This material was placed into dioxane (10 mL) and added dropwise to a stirring solution of 40% methylamine in water (3 mL, excess) at room temperature and the mixture was stirred overnight. In the morning, the solution was concentrated under reduced vacuum and the resulting oil was taken into methylene chloride and the organic layer was washed once with $H_2O$, dried over $K_2CO_3$, filtered, and concentrated under reduced vacuum to yield 78 mg as an oil. This material was purified via silica gel chromatography employing the chromatotron and using a 1000 micron rotor while eluting with a solvent of ethyl acetate to yield the title compound (28 mg, 25%) as a white solid. Ion spray M.S. 435.2 (M* - 1) and 417.1 (M* - 19 Fluorine).

| Calculated for $C_{20}H_{25}N_4O_4SF$: | | | |
|---|---|---|---|
| Theory | C 55.03, | H 5.77, | N 12.83. |
| Found | C 55.85, | H 6.09, | N 12.02. |

Example 53

Preparation of propane-2-sulfonic acid [2-fluoro-2-(2-fluoro-4-methoxy-phenyl)-propyl]-amide.

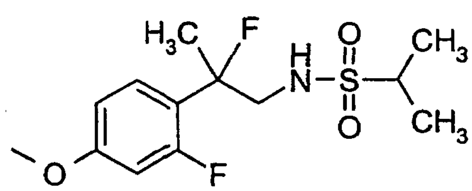

Preparation of 2-(2-Fluoro-4-methoxy-phenyl)-2-trimethylsilanyloxy-propionitrile.

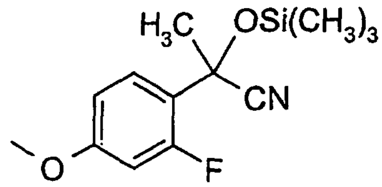

Scheme IV, Step A: 2'-Fluoro-4'-methoxy acetophenone (2.0 g, 11.89 mmol) was combined with zinc iodide (0.38 g, 1.19 mmol) in a 50 mL round bottom flask. Trimethylsilyl cyanide (4.8 mL, 35.67 mmol) was slowly added dropwise to the solid mixture, with the generation of heat. The resulting dark brown solution was stirred at room temperature under nitrogen overnight. The mixture was diluted with $CHCl_3$ and washed with $NaHCO_3$, water and brine, dried ($MgSO_4$), filtered, and concentrated to a yellow oil that was used in the next step without purification.

Preparation of 2-(2-Fluoro-4-methoxy-phenyl)-2-hydroxy-propylamine hydrochloride.

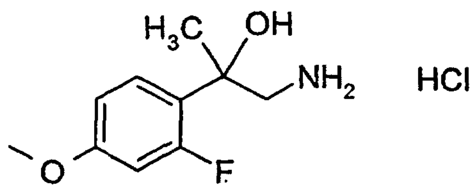

Scheme IV, Step B: 2-(2-Fluoro-4-methoxy-phenyl)-2-trimethylsilanyloxy-propionitrile (2.8 g, 10.47 mmol) was dissolved in dry THF (30 mL) and stirred at room temperature under nitrogen. A 2.0 M solution of borane-dimethylsulfide complex (15.7 mL, 31.41 mmol) was added dropwise and the reaction was heated at reflux for 3 hours then cooled to room temperature. Concentrated HCl was carefully added dropwise until the evolution of gas ceased. The reaction mixture was diluted with diethyl ether and a white precipitate formed. The solids were collected and washed with additional ether to give 2.5 g (100%) of 2-(2-fluoro-4-methoxy-phenyl)-2-hydroxy-propylamine hydrochloride. Electrospray mass spectrum (M-1)= 199.99 (free amine)

Preparation of Propane-2-sulfonic acid [2-(2-fluoro-4-methoxy-phenyl)-2-hydroxy-propyl]-amide.

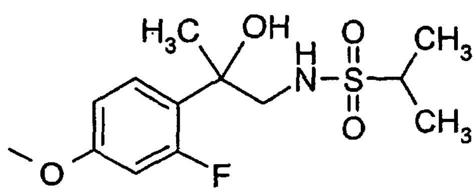

Scheme 1, Step A: 2-(2-Fluoro-4-methoxy-phenyl)-2-hydroxy-propylamine hydrochloride was converted to the free base by partitioning between ethyl acetate and 1 M NaOH. The amine (1.0 g, 5.02 mmol) was combined with propane-2-sulfonic acid benzotriazol-1-yl ester (1.3 g, 5.52 mmol) in DMF (25 mL) and heated at 120°C for 2 hr. then cooled to room temperature. The reaction mixture was diluted with ethyl acetate and extracted with 1 M HCl. The organic layer was separated and washed with water and saturated NaCl, dried ($MgSO_4$), filtered, and concentrated to a yellow oil which was used in the next step without purification,

Preparation of final title compound.

Scheme I, Step B: A dry $CH_2Cl_2$ solution (10 mL) of (diethylamino)sulfur trifluoride (DAST) (0.419 mL, 3.17 mmol) was cooled to -78°C while stirring under $N_2$. A $CH_2Cl_2$ solution (5 mL) of propane-2-sulfonic acid [2-(2-fluoro-4-methoxyphenyl)-2-hydroxy-propyl]-amide (0.79 g, 2.64 mmol) was added dropwise via syringe and the reaction was immediately brought to 0°C. The reaction was quenched with water and diluted with diethyl ether. The organic layer was separated and washed with water and saturated NaCl solution, dried ($MgSO_4$), filtered, and concentrated to a yellow oil. The crude residue was purified by silica gel chromatography, eluting with 30% ethyl acetate/hexanes, to provide the final propyl]-amide, (0.52 g, 64%) as a light yellow oil.

$^1$HNMR ($CDCl_3$) δ 1.20-1.22 (6H,d), 1.24-1.26 (6H,d), 1.68-1.73 (1H,d), 3.25 (1H, sept), 3.58-3.66 (2H, m), 3.80 (3H, s), 4.20 (1H, t), 6.60 (1H, m), 6.73 (1H, m), 7.36 (1H, t).

The following Table I specifically illustrates additional preferred substituents for $R^1$ Table I.

| $R^1$ |
|---|
| 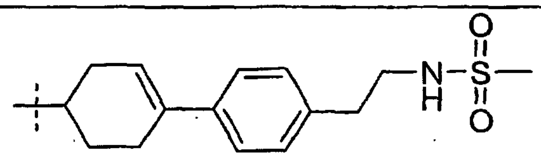 |

| $\underline{R}^1$ |
|---|
| 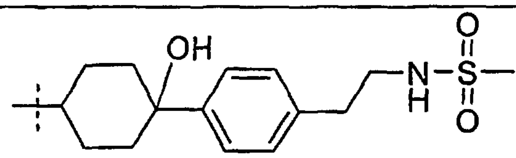 |
| 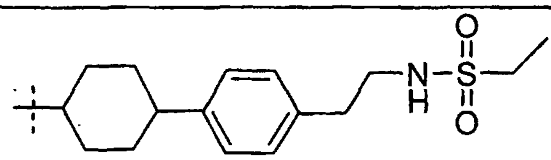 |
| 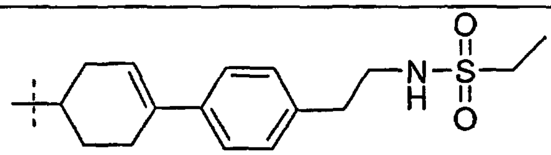 |
| 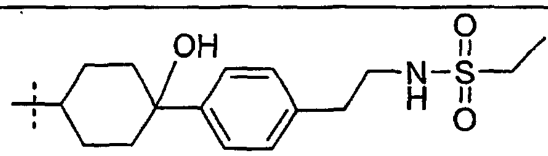 |
| 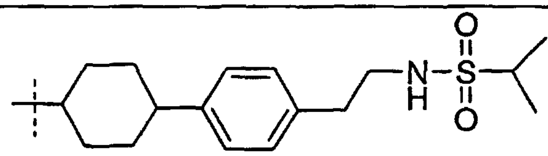 |
| 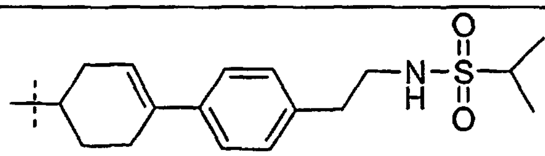 |
| 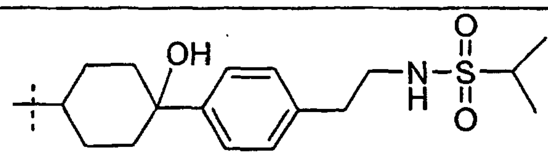 |
| 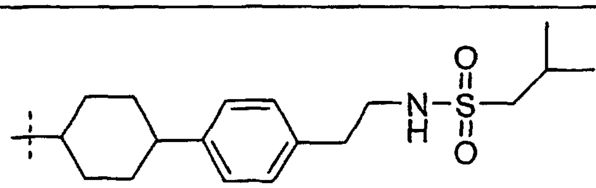 |
| 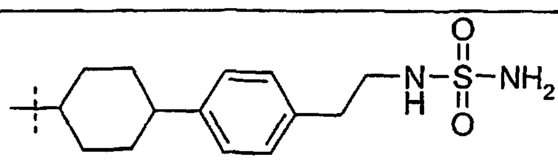 |

| $R^1$ |
|---|
| 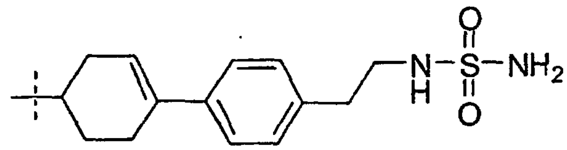 |
| 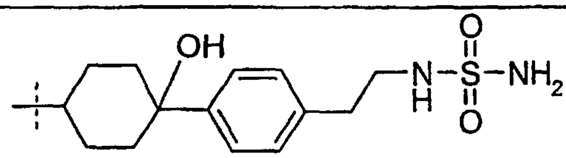 |
| 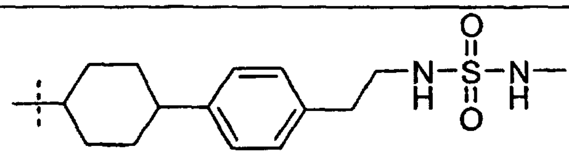 |
| 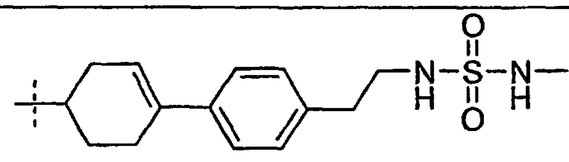 |
| 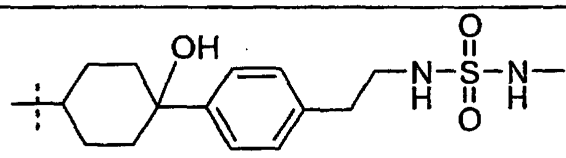 |
| 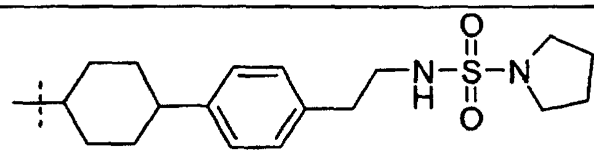 |
| 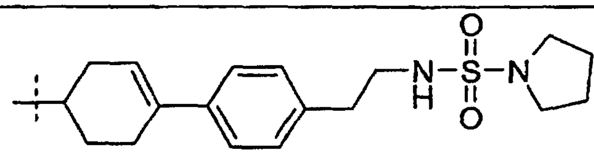 |
| 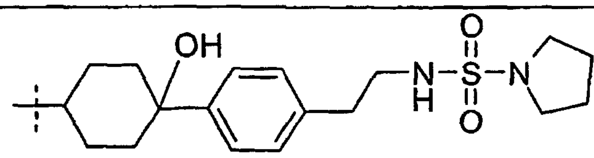 |
| 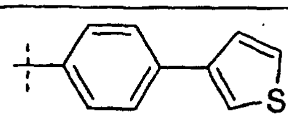 |

| $\underline{R^1}$ |
|---|
| 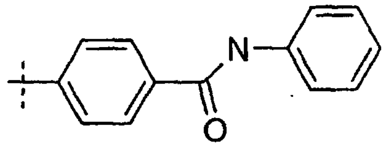 |
| 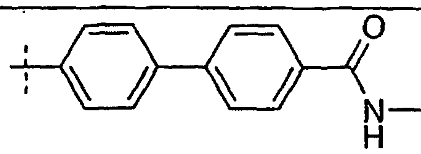 |
| 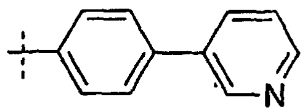 |
| 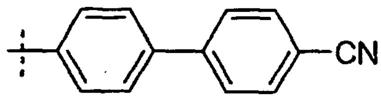 |
| 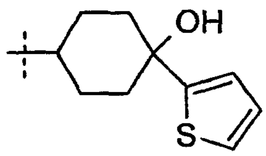 |
| 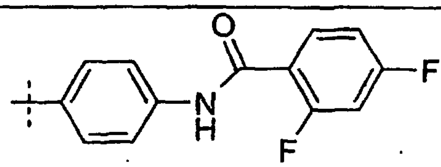 |
| 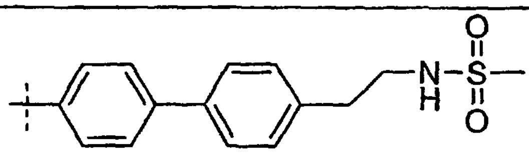 |
| 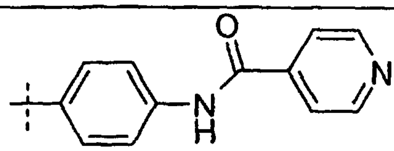 |
| 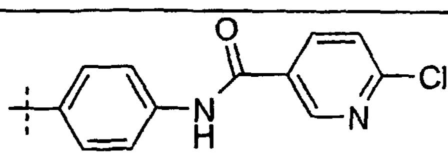 |

| $R^1$ |
|---|
| 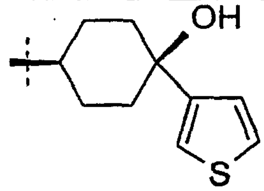 |
| 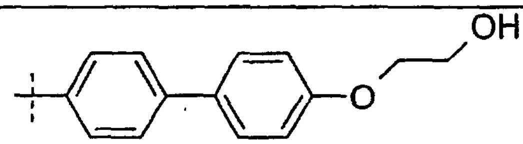 |

The following Table II illustrates additional compounds of the present invention. The following compounds can be prepared by one of ordinary skill in the art in a manner analogous to the techniques and procedures described hereinabove. The starting materials and reagents are readily available to one of ordinary skill in the art.

Table II

| Example | Compound |
|---|---|
| 54 | 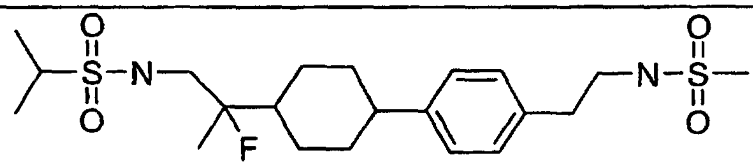 |
| 55 | 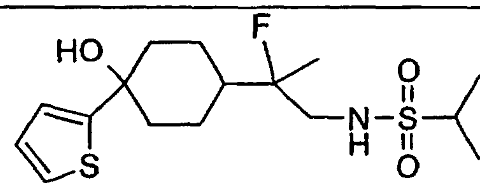 |
| 56 | 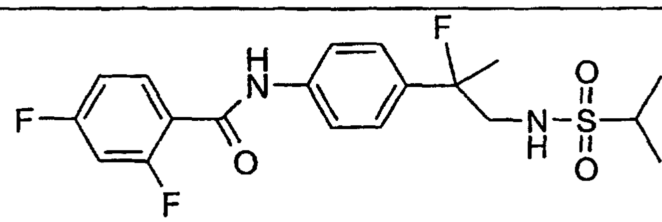 |
| 57 | 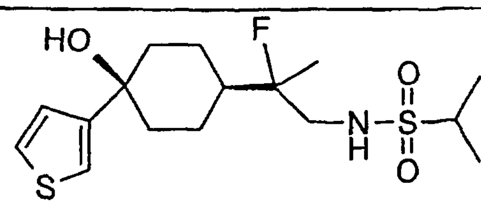 |

| Example | Compound |
|---|---|
| 58 | 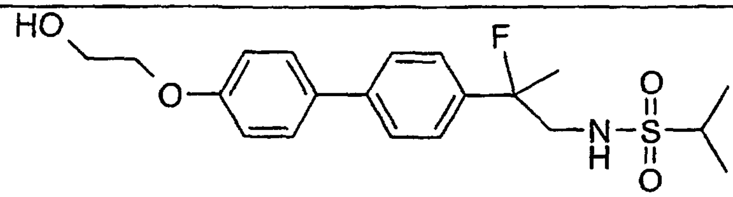 |

## Claims

1. Use of a compound of the formula:

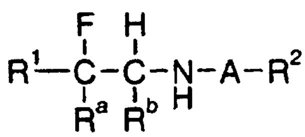

wherein:

A represents $SO_2$, $CO_2$, or CONH;
$R^a$ represents (1-6C)alkyl, (2-6C)alkenyl, -(1-4C)alkyl(3-8C)cycloalkyl, or -(1-4C)alkylaromatic;
$R^b$ represents H, (1-6C)alkyl, (2-6C)alkenyl, -(1-4C)alkyl(3-8C)cycloalkyl, or -(1-4C)alkylaromatic; or
$R^a$ and $R^b$ together with the carbon atoms to which they are attached form a (3-8C) saturated carbocyclic ring, a (3-8C) saturated carbocyclic ring containing a heteroatom selected from the group consisting of sulfur or oxygen, or a (5-8C) carbocyclic ring containing one double bond;
$R^1$ represents an unsubstituted or substituted aromatic group, an unsubstituted or substituted heteroaromatic group, or an unsubstituted or substituted (5-8C)cycloalkyl group;
$R^2$ represents (1-6C)alkyl, (3-6C)cycloalkyl, (1-6C)fluoroalkyl, (1-6C)chloroalkyl, (2-6C)alkenyl, (1-4C)alkoxy (1-4C)alkyl, phenyl which is unsubstituted or substituted by halogen, (1-4C)alkyl or (1-4C)alkoxy, or when A represents $SO_2$, a group of formula $R^3R^4N$ in which $R^3$ and $R^4$ each independently represents (1-4C)alkyl or, together with the nitrogen atom to which they are attached form an azetidinyl, pyrrolidinyl, piperidinyl, morpholino, piperazinyl, hexahydroazepinyl or octahydroazocinyl group;

or a pharmaceutically acceptable salt thereof, for the manufacture of a medicament for treating stroke.

2. The use according to claim 1 wherein A is $SO_2$.

3. The use according to claim 1 wherein A represents $CO_2$.

4. The use according to claim 1 wherein A represents CONH.

5. The use according to according to any one of claims 1 to 4 wherein $R^1$ represents a naphthyl group or a phenyl, furyl, thienyl or pyridyl group which is unsubstituted or substituted by one or two substituents selected independently from halogen; nitro; cyano; hydroxyimino; (1-10C)alkyl; (2-10C)alkenyl; (2-10C)alkynyl; (3-8C)cycloalkyl; hydroxy (3-8C)cycloalkyl; oxo(3-8C)cycloalkyl; halo(1-10C)alkyl; $(CH_2)_yX^1R^9$ in which y is 0 or an integer of from 1 to 4, $X^1$ represents O, S, $NR^{10}$, CO, COO, OCO, $CONR^{11}$, $NR^{12}CO$, $NR^{12}COCOO$ or $OCONR^{13}$, $R^9$ represents hydrogen, (1-10C)alkyl, (3-10C)alkenyl, (3-10C)alkynyl, pyrrolidinyl, tetrahydrofuryl, morpholino or (3-8C)cycloalkyl and $R^{10}$, $R^{11}$, $R^{12}$ and $R^{13}$ each independently represents hydrogen or (1-10C)alkyl, or $R^9$ and $R^{10}$, $R^{11}$, $R^{12}$ or $R^{13}$ together with the nitrogen atom to which they are attached form an azetidinyl, pyrrolidinyl, piperidinyl or morpholino group; N-(1-4C)alkylpiperazinyl; N-phenyl(1-4C)alkylpiperazinyl; thienyl; furyl; oxazolyl; isoxazolyl; pyrazolyl; imidazolyl; thiazolyl; pyridyl; pyridazinyl; pyrimidinyl; dihydrothienyl; dihydrofuryl; dihydrothiopyranyl; dihydropyranyl;

dihydrothiazolyl; (1-4C)alkoxycarbonyldihydrothiazolyl; (1-4C)alkoxycarbonyldimethyldihydrothiazolyl; tetrahydrothienyl; tetrahydrofuryl; tetrahydrothiopyranyl; tetrahydropyranyl; indolyl; benzofuryl; benzothienyl; benzimidazolyl; and a group of formula $R^{14}-(L^a)_n-X^2-(L^b)_m$ in which $X^2$ represents a bond, O, NH, S, SO, $SO_2$, CO, CH(OH), CONH, NHCO, NHCONH, NHCOO, COCONH, $OCH_2CONH$ or CH=CH, $L^a$ and $L^b$ each represent (1-4C)alkylene, one of n and m is 0 or 1 and the other is 0, and $R^{14}$ represents a phenyl or heteroaromatic group which is unsubstituted or substituted by one or two of halogen, nitro, cyano, hydroxyimino, (1-10C) alkyl, (2-10C)alkenyl, (2-10C)alkynyl, (3-8C)-cycloalkyl, 4-(1,1-dioxotetrahydro-1,2-thiazinyl), halo(1-10C)alkyl, cyano(2-10C)alkenyl, phenyl, and $(CH_2)_zX^3R^{15}$ in which z is 0 or an integer of from 1 to 4, $X^3$ represents O, S, $NR^{16}$, CO, CH(OH), COO, OCO, $CONR^{17}$, $NR^{18}CO$, $NHSO_2$, $SO_2NH$, $NHSO_2NR^{17}$, NHCONH, $OCONR^{19}$ or $NR^{19}COO$, $R^{15}$ represents hydrogen, (1-10C)alkyl, phenyl(1-4C)alkyl, halo(1-10C)alkyl, (1-4C)alkoxycarbonyl(1-4C)alkyl, (1-4C)alkylsulfonylamino (1-4C)alkyl, (N-(1-4C)alkoxycarbonyl)(1-4C)alkylsulfonylamino-(1-4C)alkyl, (3-10C)alkenyl, (3-10C)alkynyl, (3-8C)-cycloalkyl, camphoryl or an aromatic or heteroaromatic group which is unsubstituted or substituted by one or two of halogen, (1-4C)alkyl, halo(1-4C)alkyl, di(1-4C)alkylamino and (1-4C)alkoxy and $R^{16}$, $R^{17}$, $R^{18}$ and $R^{19}$ each independently represents hydrogen or (1-10C)alkyl, or $R^{15}$ and $R^{16}$, $R^{17}$, $R^{18}$ or $R^{19}$ together with the nitrogen atom to which they are attached form an azetidinyl, pyrrolidinyl, piperidinyl or morpholino group.

**6.** The use according to any one of claims 1 to 5 wherein $R^2$ represents (1-6C)alkyl, (3-6C)cycloalkyl, fluoro(1-6C) alkyl, chloro(1-6C)alkyl, (2-6C)alkenyl 1-4C)alkoxy(1-4C)alkyl, heteroaromatic, or phenyl which is unsubstituted or substituted by halogen, (1-4C)alkyl or (1-4C)alkoxy.

**7.** The use according to any one of claims 1 to 6 wherein $R^2$ represents isopropyl.

**8.** The use according to according to any one of claims 1 to 7 wherein $R^a$ represents (1-6C)alkyl or (2-6C)alkenyl.

**9.** The use according to any one of claims 1 to 8 wherein $R^a$ represents methyl and $R^b$ represents hydrogen.

**10.** The use according to claims 1 to 9 wherein $R^1$ represents a substituted aromatic group.

**11.** The use according to claim 10 wherein the substituted aromatic group is a substituted phenyl.

**12.** The use according to claim 11 wherein the substituted phenyl is substituted with halogen; nitro; cyano; hydroxyimino; (1-10C) alkyl; (2-10C)alkenyl; (2-10C)alkynyl; (3-8C)cycloalkyl; hydroxy(3-8C)cycloalkyl; oxo(3-8C)cycloalkyl; halo(1-10C)alkyl; $(CH_2)_yX^1R^9$ in which y is 0 or an integer of from 1 to 4, $X^1$ represents O, S, $NR^{10}$, CO, COO, OCO, $CONR^{11}$, $NR^{12}CO$, $NR^{12}COCOO$, $OCONR^{13}$, $R^9$ represents hydrogen, (1-10C) alkyl, (3-10C)alkenyl, (3-10C)alkynyl, pyrrolidinyl, tetrahydrofuryl, morpholino or (3-8C)cycloalkyl and $R^{10}$, $R^{11}$, $R^{12}$ and $R^{13}$ each independently represents hydrogen or (1-10C)alkyl, or $R^9$ and $R^{10}$, $R^{11}$, $R^{12}$ or $R^{13}$ together with the nitrogen atom to which they are attached form an azetidinyl, pyrrolidinyl, piperidinyl or morpholino group; N-(1-4C)alkylpiperazinyl; N-phenyl(1-4C)alkylpiperazinyl; thienyl; furyl; oxazolyl; isoxazolyl; pyrazolyl; imidazolyl; thiazolyl; pyridyl; pyridazinyl; pyrimidinyl; dihydrothienyl; dihydrofuryl; dihydrothiopyranyl; dihydropyranyl; dihydrothiazolyl; (1-4C)alkoxycarbonyl dihydrothiazolyl; (1-4C)alkoxycarbonyl dimethyl-dihydrothiazolyl; tetrahydrothienyl; tetrahydrofuryl; tetrahydrothiopyranyl; tetrahydropyranyl; indolyl; benzofuryl; benzothienyl; benzimidazolyl; and a group of formula $R^{14}-(L^a)_n-X^2-(L^b)_m$ in which $X^2$ represents a bond, O, NH, S, SO, $SO_2$, CO, CH(OH), CONH, NHCO, NHCONH, NHCOO, COCONH, $OCH_2CONH$, or CH=CH, $L^a$ and $L^b$ each represent (1-4C)alkylene, one of n and m is 0 or 1 and the other is 0, and $R^{14}$ represents a phenyl or heteroaromatic group which is unsubstituted or substituted by one or two of halogen; nitro; cyano; (1-10C) alkyl; (2-10C)alkenyl; (2-10C)alkynyl; (3-8C)cycloalkyl; 4-(1,1-dioxotetrahydro-1,2-thiazinyl); halo(1-10C)alkyl; cyano(2-10C)alkenyl; phenyl; and $(CH_2)_zX^3R^{15}$ in which z is 0 or an integer of from 1 to 4, $X^3$ represents O, S, $NR^{16}$, CO, CH(OH), COO, OCO, $CONR^{17}$, $NR^{18}CO$, $NHSO_2$, $SO_2NH$, $NHSO_2NR^{17}$, $OCONR^{19}$ or $NR^{19}COO$, $R^{15}$ represents hydrogen, (1-10C)alkyl, phenyl(1-4C)alkyl, halo(1-10C) alkyl, (1-4C)alkoxycarbonyl(1-4C)alkyl, (1-4C)alkylsulfonylamino(1-4C)alkyl, N-(1-4C)alkoxycarbonyl)(1-4C)alkylsulfonylamino(1-4C)alkyl, (3-10C)alkenyl, (3-10C)alkynyl, (3-8C)cycloalkyl, camphoryl, or an aromatic or heteroaromatic group which is unsubstituted or substituted by one or two of halogen, (1-4C)alkyl, halo(1-4C)alkyl, di (1-4C)alkylamino and (1-4C)alkoxy, and $R^{16}$, $R^{17}$, $R^{18}$ and $R^{19}$ each independently represents hydrogen or (1-10C) alkyl, or $R^{15}$ and $R^{16}$, $R^{17}$, $R^{18}$ or $R^{19}$ together with the nitrogen atom to which they are attached form an azetidinyl, pyrrolidinyl, piperidinyl or morpholino group.

**13.** The use according to claim 12 wherein the substituted phenyl is substituted with a group of formula $R^{14}-(L^a)_n-X^2-(L^b)_m$ in which $X^2$ represents a bond, O, NH, S, SO, $SO_2$, CO, CH(OH), CONH, NHCO, NHCONH, NHCOO, COCONH, $OCH_2CONH$, or CH=CH, $L^a$ and $L^b$ each represent (1-4C)alkylene, one of n and m is 0 or 1 and the

other is 0, and $R^{14}$ represents a phenyl or heteroaromatic group which is unsubstituted or substituted by one or two of halogen; nitro; cyano; (1-10C) alkyl; (2-10C)alkenyl; (2-10C)alkynyl; (3-8C)cycloalkyl; 4-(1,1-dioxotetrahydro-1,2-thiazinyl); halo(1-10C)alkyl; cyano(2-10C)alkenyl; phenyl; and $(CH_2)_zX^3R^{15}$ in which z is 0 or an integer of from 1 to 4, $X^3$ represents O, S, $NR^{16}$, CO, CH(OH), COO, OCO, $CONR^{17}$, $NR^{18}CO$, $NHSO_2$, $SO_2NH$, $NHSO_2NR^{17}$, $OCONR^{19}$ or $NR^{19}COO$, $R^{15}$ represents hydrogen, (1-10C)alkyl, phenyl(1-4C)alkyl, halo(1-10C) alkyl, (1-4C)alkoxycarbonyl(1-4C)alkyl, (1-4C)alkylsulfonylamino(1-4C)alkyl, N-(1-4C)alkoxycarbonyl)(1-4C)alkylsulfonylamino(1-4C)alkyl, (3-10C)alkenyl, (3-10C)alkynyl, (3-8C)cycloalkyl, camphoryl, or an aromatic or heteroaromatic group which is unsubstituted or substituted by one or two of halogen, (1-4C)alkyl, hato(1-4C)alkyl, di (1-4C)alkylamino and (1-4C)alkoxy, and $R^{16}$, $R^{17}$, $R^{18}$ and $R^{19}$ each independently represents hydrogen or (1-10C) alkyl, or $R^{15}$ and $R^{16}$, $R^{17}$, $R^{18}$ or $R^{19}$ together with the nitrogen atom to which they are attached form an azetidinyl, pyrrolidinyl, piperidinyl or morpholino group.

**14.** The use according to claim 13 wherein $(L^a)_n$-$X^2$-$(L^b)_m$ represents a bond, CONH, or $CH_2O$.

**15.** The use according to claim 14 wherein $R^{14}$ represents a phenyl which is unsubstituted or substituted by one or two of halogen; nitro; cyano; (1-10C) alkyl; (2-10C)alkenyl; (2-10C)alkynyl; (3-8C)cycloalkyl; 4-(1,1-dioxotetrahydro-1,2-thiazinyl); halo(1-10C)alkyl; cyano(2-10C)alkenyl; phenyl; and $(CH_2)_zX^3R^{15}$ in which z is 0 or an integer of from 1 to 4, $X^3$ represents O, S, $NR^{16}$, CO, CH(OH), COO, OCO, $CONR^{17}$, $NR^{18}CO$, $NHSO_2$, $SO_2NH$, $NHSO_2NR^{17}$, $OCONR^{19}$ or $NR^{19}COO$, $R^{15}$ represents hydrogen, (1-10C)alkyl, phenyl(1-4C)alkyl, halo(1-10C) alkyl, (1-4C)alkoxycarbonyl(1-4C)alkyl, (1-4C)alkylsulfonylamino(1-4C)alkyl, N-(1-4C)alkoxycarbonyl)(1-4C)alkylsulfonylamino(1-4C)alkyl, (3-10C)alkenyl, (3-10C)alkynyl, (3-8C)cycloalkyl, camphoryl, or an aromatic or heteroaromatic group which is unsubstituted or substituted by one or two of halogen, (1-4C)alkyl, halo(1-4C)alkyl, di (1-4C)alkylamino and (1-4C)alkoxy, and $R^{16}$, $R^{17}$, $R^{18}$ and $R^{19}$ each independently represent hydrogen or (1-10C) alkyl, or $R^{15}$ and $R^{16}$, $R^{17}$, R18 or $R^{19}$ together with the nitrogen atom to which they are attached form an azetidinyl, pyrrolidinyl, piperidinyl or morpholino group.

**16.** The use according to claim 15 wherein phenyl is substituted by one or two of halogen; nitro; cyano; (1-10C) alkyl; hato(1-10C)alkyl; and $(CH_2)_zX^3R^{15}$ in which z is 0, 1 or 2, $X^3$ represents O, $NR^{16}$, CO, COO, $CONR^{17}$, $NR^{18}CO$, $NHSO_2$, $SO_2NH$, $NHSO_2NR^{17}$, $OCONR^{19}$ or $NR^{19}COO$, $R^{15}$ represents hydrogen, (1-10C)alkyl, phenyl(1-4C) alkyl, halo(1-10C)alkyl, or (3-10C)alkenyl, and $R^{16}$, $R^{17}$, $R^{18}$ and $R^{19}$ each independently represent hydrogen or (1-10C)alkyl.

**17.** The use according to claim 16 wherein phenyl is substituted by one or two of fluoro; chloro, cyano; (1-4C)alkyl; trifluoromethyl; and $(CH_2)_zX^3R^{15}$ in which z is 0, or 2, $X^3$ represents $NR^{16}$, CO, COO, $CONR^{17}$, $NR^{18}CO$, $NHSO_2$, $SO_2NH$, $R^{15}$ represents hydrogen, (1-4C)alkyl, phenyl(1-4C)alkyl, or halo(1-4C)alkyl, and $R^{16}$, $R^{17}$, $R^{18}$ and $R^{19}$ each independently represent hydrogen or (1-4C)alkyl.

**18.** The use according to claim 17 wherein phenyl is substituted by one of fluoro; chloro, cyano; (1-4C)alkyl; and $(CH_2)_zX^3R^{15}$ in which z is 0, or 2, $X^3$ represents $CONR^{17}$, $NR^{18}CO$, $NHSO_2$, $SO_2NH$, $R^{15}$ represents hydrogen or (1-4C)alkyl, and $R^{16}$, $R^{17}$, $R^{18}$ and $R^{19}$ each independently represent hydrogen or (1-4C)alkyl.

**19.** Use of a compound of formula:

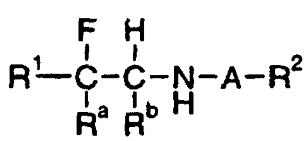

wherein:

A represents $SO_2$, $CO_2$, or CONH;
$R^a$ represents (1-6C)alkyl, (2-6C)alkenyl, -(1-4C)alkyl(3-8C)cycloalkyl, or -(1-4C)alkylaromatic;
$R^b$ represents H, (1-6C)alkyl, (2-6C)alkenyl, -(1-4C)alkyl(3-8C)cycloalkyl, or -(1-4C)alkylaromatic; or
$R^a$ and $R^b$ together with the carbon atoms to which they are attached form a (3-8C) saturated carbocyclic ring, a (3-8C) saturated carbocyclic ring containing a heteroatom selected from the group consisting of sulfur or oxygen, or a (5-8C) carbocyclic ring containing one double bond;
$R^1$ represents an unsubstituted or substituted aromatic group, an unsubstituted or substituted heteroaromatic

group, or an unsubstituted or substituted (5-8C)cycloalkyl group;
$R^2$ represents (1-6C)alkyl, (3-6C)cycloalkyl, (1-6C)fluoroalkyl, (1-6C)chloroalkyl, (2-6C)alkenyl, (1-4C)alkoxy (1-4C)alkyl, phenyl which is unsubstituted or substituted by halogen, (1-4C)alkyl or (1-4C)alkoxy, or when A represents $SO_2$, a group of formula $R^3R^4N$ in which $R^3$ and $R^4$ each independently represents (1-4C)alkyl or, together with the nitrogen atom to which they are attached form an azetidinyl, pyrrolidinyl, piperidinyl, morpholino, piperazinyl, hexahydroazepinyl or octahydroazocinyl group;

or a pharmaceutically acceptable salt thereof, for the manufacture of a medicament for treating traumatic brain injury, traumatic spinal cord injury, brain damage resulting from cerebral hypoperfusion, or other neurodegenerative conditions.

**20.** The use according to claim 19 for treating traumatic spinal cord injury.

## Patentansprüche

**1.** Verwendung einer Verbindung der Formel

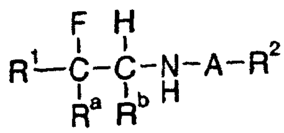

worin
A für $SO_2$, $CO_2$ oder CONH steht,
$R^a$ für $C_1$-$C_6$ Alkyl, $C_2$-$C_6$ Alkenyl, $C_1$-$C_4$ Alkyl-$C_3$-$C_8$-cycloalkyl oder einen $C_1$-$C_4$ Alkylaromaten steht,
$R^b$ für H, $C_1$-$C_6$ Alkyl, $C_2$-$C_6$ Alkenyl, $C_1$-$C_4$ Alkyl-$C_3$-$C_8$-cycloalkyl oder einen $C_1$-$C_4$ Alkylaromaten steht, oder
$R^a$ und $R^b$ zusammen mit den Kohlenstoffatomen, an die sie gebunden sind, einen gesättigten carbocydischen $C_3$-$C_8$ Ring, einen gesättigten carbocyclischen $C_3$-$C_8$ Ring, der ein Heteroatom enthält, das ausgewählt ist aus der Gruppe, die besteht aus Schwefel oder Sauerstoff oder einen carbocyclischen $C_5$-$C_8$ Ring, der eine Doppelbindung aufweist, bilden,
$R^1$ für eine unsubstituierte oder substituierte aromatische Gruppe, eine unsubstituierte oder substituierte heteroaromatische Gruppe oder eine unsubstituierte oder substituierte $C_5$-$C_8$ Cycloalkylgruppe steht,
$R^2$ für $C_1$-$C_6$ Alkyl, $C_3$-$C_6$ Cycloalkyl, $C_1$-$C_6$ Fluoralkyl, $C_1$-$C_6$ Chloralkyl, $C_2$-$C_6$ Alkenyl, $C_1$-$C_4$ Alkoxy-$C_1$-$C_4$-alkyl, Phenyl, das unsubstituiert oder substituiert ist mit Halogen, $C_1$-$C_4$ Alkyl oder $C_1$-$C_4$ Alkoxy steht oder wenn A für $SO_2$ steht, für eine Gruppe der Formel $R^3R^4N$ steht, worin $R^3$ und $R^4$ jeweils unabhängig für $C_1$-$C_4$ Alkyl stehen oder zusammen mit dem Stickstoffatom, an das sie gebunden sind, eine Azetidinyl-, Pyrrolidinyl-, Piperidinyl-, Morpholino-, Piperazinyl-, Hexahydroazepinyl- oder Octahydroazocinylgruppe bilden,
oder ein pharmazeutisch annehmbares Salz hiervon zur Herstellung eines Arzneimittels zur Behandlung von Schlaganfall.

**2.** Verwendung nach Anspruch 1, worin A für $SO_2$ steht.

**3.** Verwendung nach Anspruch 1, worin A für $CO_2$ steht.

**4.** Verwendung nach Anspruch 1, worin A für CONH steht.

**5.** Verwendung nach einem der Ansprüche 1 bis 4, worin $R^1$ für eine Naphthylgruppe oder eine Phenyl-, Furyl-, Thienyl- oder Pyridylgruppe steht, die unsubstituiert oder substituiert ist mit einem oder zwei Substituenten, die unabhängig ausgewählt sind aus Halogen, Nitro, Cyano, Hydroxyimino, $C_1$-$C_{10}$ Alkyl, $C_2$-$C_{10}$ Alkenyl, $C_2$-$C_{10}$ Alkinyl, $C_3$-$C_8$ Cycloalkyl, Nydroxy-$C_3$-$C_8$-cycloalkyl, Oxo-$C_3$-$C_8$-cycloalkyl, Halogen-$C_1$-$C_{10}$-alkyl, $(CH_2)_yX^1R^9$, worin Y für 0 oder eine ganze Zahl von 1 bis 4 steht, $X^1$ für O, S, $NR^{10}$, CO, COO, OCO, $CONR^{11}$, $NR^{12}CO$, $NR^{12}COCOO$ oder $OCONR^{13}$ steht, $R^9$ für Wasserstoff, $C_1$-$C_{10}$ Alkyl, $C_3$-$C_{10}$ Alkenyl, $C_3$-$C_{10}$ Alkinyl, Pyrrolidinyl, Tetrahydrofuryl, Morpholino oder $C_3$-$C_8$ Cycloalkyl steht und $R^{10}$, $R^{11}$, $R^{12}$ und $R^{13}$ jeweils unabhängig für Wasserstoff oder $C_1$-$C_{10}$ Alkyl stehen oder $R^9$ und $R^{10}$, $R^{11}$, $R^{12}$ oder $R^{13}$ zusammen mit dem Sticktoffatom, an das sie gebunden sind, eine Azetidinyl-, Pyrrolidinyl-, Piperidinyl- oder Morpholinogruppe bilden, aus N-$C_1$-$C_4$-Al-

kylpiperazinyl, N-Phenyl-$C_1$-$C_4$-alkylpiperazinyl, Thienyl, Furyl, Oxazolyl, Isoxazolyl, Pyrazolyl, Imidazolyl, Thiazolyl, Pyridyl, Pyridazinyl, Pyrimidinyl, Dihydrothienyl, Dihydrofuryl, Dihydrothiopyranyl, Dihydropyranyl, Dihydrothiazolyl, $C_1$-$C_4$ Alkoxycarbonyldihydrothiazolyl, $C_1$-$C_4$ Alkoxycarbonyldimethyldihydrothiazolyl, Tetrahydrothienyl, Tetrahydrofuryl, Tetrahydrothiopyranyl, Tetrahydropyranyl, Indolyl, Benzofuryl, Benzothienyl, Benzimidazolyl und einer Gruppe der Formel $R^{14}$-$(L^a)_nX^2$-$(L^b)_m$, worin $X^2$ für eine Bindung, O, NH, S, SO, $SO_2$, CO, CH(OH), CONH, NHCO, NHCONH, NHCOO, COCONH, $OCH_2CONH$ oder CH=CH steht, $L^a$ und $L^b$ jeweils für $C_1$-$C_4$ Alkylen stehen, eines aus n und m für 0 oder 1 steht und das andere für 0 steht und $R^{14}$ für eine Phenylgruppe oder heteroaromatische Gruppe steht, die unsubstituiert oder substituiert ist mit einem oder zwei aus Halogen, Nitro, Cyano, Hydroxyimino, $C_1$-$C_{10}$ Alkyl, $C_2$-$C_{10}$ Alkenyl, $C_2$-$C_{10}$ Alkinyl, $C_3$-$C_8$ Cycloalkyl, 4-(1,1-Dioxotetrahydro-1,2-thiazinyl), Halogen-$C_1$-$C_{10}$-alkyl, Cyano-$C_2$-$C_{10}$-alkenyl, Phenyl und $(CH_2)_zX^3R^{15}$, worin z für 0 oder eine ganze Zahl von 1 bis 4 steht, $X^3$ für O, S, $NR^{16}$, CO, CH(OH), COO, OCO, $CONR^{17}$, $NR^{18}CO$, $NHSO_2$, $SO_2NH$, $NHSO_2NR^{17}$, NHCONH, $OCONR^{19}$ oder $NR^{19}COO$ steht, $R^{15}$ steht für Wasserstoff, $C_1$-$C_{10}$ Alkyl, Phenyl-$C_1$-$C_4$-alkyl, Halogen-$C_1$-$C_{10}$-alkyl, $C_1$-$C_4$ Alkoxycarbonyl-$C_1$-$C_4$-alkyl, $C_1$-$C_4$ Alkylsulfonylamino-$C_1$-$C_4$-alkyl, N-$C_1$-$C_4$ Alkoxycarbonyl-$C_1$-$C_4$-alkylsulfonylamino-$C_1$-$C_4$alkyl, $C_3$-$C_{10}$ Alkenyl, $C_3$-$C_{10}$ Alkinyl, $C_3$-$C_8$ Cycloalkyl, Camphoryl oder eine aromatische oder heteroaromatische Gruppe, die unsubstituiert oder substituiert ist durch ein oder zwei aus Halogen, $C_1$-$C_4$ Alkyl, Halogen-$C_1$-$C_4$-alkyl, Di-$C_1$-$C_4$-alkylamino und $C_1$-$C_4$ Alkoxy und $R^{16}$, $R^{17}$, $R^{18}$ und $R^{19}$ jeweils unabhängig für Wasserstoff oder $C_1$-$C_{10}$ Alkyl stehen oder $R^{15}$ und $R^{16}$, $R^{17}$, $R^{18}$ oder $R^{19}$ zusammen mit dem Stickstoffatom, an das sie gebunden sind, eine Azetidinyl-, Pyrrolidinyl-, Piperidinyl- oder Morpholinogruppe bilden.

**6.** Verwendung nach einem der Ansprüche 1 bis 5, worin $R^2$ für $C_1$-$C_6$ Alkyl, $C_3$-$C_6$ Cycloalkyl, Fluor-$C_1$-$C_6$-alkyl, Chlor-$C_1$-$C_6$-alkyl, $C_2$-$C_6$ Alkenyl-$C_1$-$C_4$-alkoxy-$C_1$-$C_4$-alkyl, einen Heteroaromaten oder Phenyl steht, das unsubsituiert oder durch Halogen, $C_1$-$C_4$ Alkyl oder $C_1$-$C_4$ Alkoxy substituiert ist.

**7.** Verwendung nach einem der Ansprüche 1 bis 6, worin $R^2$ für Isopropyl steht.

**8.** Verwendung nach einem der Ansprüche 1 bis 7, worin $R^a$ für $C_1$-$C_6$ Alkyl oder $C_2$-$C_6$ Alkenyl steht.

**9.** Verwendung nach einem der Ansprüche 1 bis 8, worin $R^a$ für Methyl steht und $R^b$ für Wasserstoff steht.

**10.** Verwendung nach einem der Ansprüche 1 bis 9, worin $R^1$ für eine substituierte aromatische Gruppe steht.

**11.** Verwendung nach Anspruch 10, worin die substituierte aromatische Gruppe ein substituiertes Phenyl ist.

**12.** Verwendung nach Anspruch 11, worin das substituierte Phenyl substituiert ist mit Halogen, Nitro, Cyano, Hydroxyimino, $C_1$-$C_{10}$ Alkyl, $C_2$-$C_{10}$ Alkenyl, $C_2$-$C_{10}$ Alkinyl, $C_3$-$C_8$ Cycloalkyl, Hydroxy-$C_3$-$C_8$cycloalkyl, Oxo-$C_3$-$C_8$-cycloalkyl, Halogen-$C_1$-$C_{10}$-alkyl, $(CH_2)_yX^1R^9$, worin Y für 0 oder eine ganze Zahl von 1 bis 4 steht, $X^1$ für O, S, $NR^{10}$, CO, COO, OCO, $CONR^{11}$, $NR^{12}CO$, $NR^{12}COCOO$ oder $OCONR^{13}$ steht, $R^9$ für Wasserstoff, $C_1$-$C_{10}$ Alkyl, $C_3$-$C_{10}$ Alkenyl, $C_3$-$C_{10}$ Alkinyl, Pyrrolidinyl, Tetrahydrofuryl, Morpholino oder $C_3$-$C_8$ Cycloalkyl steht und $R^{10}$, $R^{11}$, $R^{12}$ und $R^{13}$ jeweils unabhängig für Wasserstoff oder $C_1$-$C_{10}$ Alkyl stehen oder $R^9$ und $R^{10}$, $R^{11}$, $R^{12}$ oder $R^{13}$ zusammen mit dem Sticktoffatom, an das sie gebunden sind, eine Azetidinyl-, Pyrrolidinyl-, Piperidinyl- oder Morpholinogruppe bilden, mit N-$C_1$-$C_4$-Alkylpiperazinyl, N-Phenyl-$C_1$-$C_4$-alkylpiperazinyl, Thienyl, Furyl, Oxazolyl, Isoxazolyl, Pyrazolyl, Imidazolyl, Thiazolyl, Pyridyl, Pyridazinyl, Pyrimidinyl, Dihydrothienyl, Dihydrofuryl, Dihydrothiopyranyl, Dihydropyranyl, Dihydrothiazolyl, $C_1$-$C_4$ Alkoxycarbonyldihydrothiazolyl, $C_1$-$C_4$ Alkoxycarbonyldimethyldihydrothiazolyl, Tetrahydrothienyl, Tetrahydrofuryl, Tetrahydtrothiopyranyl, Tetrahydropyranyl, Indolyl, Benzofuryl, Benzothienyl, Benzimidazolyl und eine Gruppe der Formel $R^{14}$-$(L^a)_n$-$X^2$-$(L^b)_m$, worin $X^2$ für eine Bindung, O, NH, S, SO, $SO_2$, CO, CH(OH), CONH, NHCO, NHCONH, NHCOO, COCONH, $OCH_2CONH$ oder CH=CH steht, $L^a$ und $L^b$ jeweils für $C_1$-$C_4$ Alkylen stehen, eines aus n und m für 0 oder 1 steht und das andere für 0 steht und $R^{14}$ für eine Phenylgruppe oder heteroaromatische Gruppe steht, die unsubstituiert oder substituiert ist mit einem oder zwei aus Halogen, Nitro, Cyano, $C_1$-$C_{10}$ Alkyl, $C_2$-$C_{10}$ Alkenyl, $C_2$-$C_{10}$ Alkinyl, $C_3$-$C_8$ Cycloalkyl, 4-(1,1-Dioxotetrahydro-1,2-thiazinyl), Halogen-$C_1$-$C_{10}$)-alkyl, Cyano-$C_2$-$C_{10}$-alkenyl, Phenyl und $(CH_2)_zX^3R^{15}$ , worin z für 0 oder eine ganze Zahl von 1 bis 4 steht, $X^3$ für O, S, $NR^{16}$, CO, CH(OH), COO, OCO, $CONR^{17}$, $NR^{18}CO$, $NHSO_2$, $SO_2NH$, $NHSO_2NR^{17}$, $OCONR^{19}$ oder $NR^{19}COO$ steht, $R^{15}$ steht für Wasserstoff, $C_1$-$C_{10}$ Alkyl, Phenyl-$C_1$-$C_4$-alkyl, Halogen-$C_1$-$C_{10}$)-alkyl, $C_1$-$C_4$ Alkoxycarbonyl-$C_1$-$C_4$-alkyl, $C_1$-$C_4$ Alkylsulfonylamino-$C_1$-$C_4$-alkyl, N-$C_1$-$C_4$ Alkoxycarbonyl-$C_1$-$C_4$alkylsulfonylamino-$C_1$-$C_4$-alkyl, $C_3$-$C_{10}$ Alkenyl, $C_3$-$C_{10}$ Alkinyl, $C_3$-$C_8$ Cycloalkyl, Camphoryl oder eine aromatische oder heteroaromatische Gruppe, die unsubstituiert oder substituiert ist durch ein oder zwei aus Halogen, $C_1$-$C_4$ Alkyl, Halogen-$C_1$-$C_4$-alkyl, Di-$C_1$-$C_4$-alkylamino und $C_1$-$C_4$ Alkoxy und $R^{16}$, $R^{17}$, $R^{18}$ und $R^{19}$ jeweils unabhängig für Wasserstoff oder $C_1$-$C_{10}$ Alkyl stehen oder $R^{15}$ und $R^{16}$, $R^{17}$, $R^{18}$ oder $R^{19}$ zusammen mit dem Stickstoffatom, an das sie gebunden sind, eine Azetidinyl- Pyrrolidinyl-, Piperidinyloder Morpholinogruppe bilden.

**13.** Verwendung nach Anspruch 12, worin das substituierte Phenyl mit einer Gruppe der Formel $R^{14}$-$(L^a)_n$-$X^2$-$(L^b)_m$ substituiert ist, worin $X^2$ für eine Bindung, O, NH, S, SO, $SO_2$, CO, CH(OH), CONH, NHCO, NHCONH, NHCOO, COCONH, $OCH_2CONH$ oder CH=CH steht, $L^a$ und $L^b$ jeweifs für $C_1$-$C_4$ Alkylen stehen, eines aus n und m für 0 oder 1 steht und das andere für 0 steht und $R^{14}$ für eine Phenylgruppe oder heteroaromatische Gruppe steht, die unsubstituiert oder substituiert ist mit einem oder zwei aus Halogen, Nitro, Cyano, $C_1$-$C_{10}$ Alkyl, $C_2$-$C_{10}$ Alkenyl, $C_2$-$C_{10}$ Alkinyl, $C_3$-$C_8$ Cycloalkyl, 4-(1,1-Dioxotetrahydro-1,2-thiazinyl), Halogen-$C_1$-$C_{10}$-alkyl, Cyano-$C_2$-$C_{10}$-alkenyl, Phenyl und $(CH_2)_zX^3R^{15}$, worin z für 0 oder eine ganze Zahl von 1 bis 4 steht, $X^3$ für O, S, $NR^{16}$, CO, CH (OH), COO, OCO, $CONR^{17}$, $NR^{18}CO$, $NHSO_2$, $SO_2NH$, $NHSO_2NR^{17}$, $OCONR^{19}$ oder $NR^{19}COO$ steht, $R^{15}$ steht für Wasserstoff, $C_1$-$C_{10}$ Alkyl, Phenyl-$C_1$-$C_4$-alkyl, Halogen-$C_1$-$C_{10}$-alkyl, $C_1$-$C_4$ Alkoxycarbonyl-$C_1$-$C_4$-alkyl, $C_1$-$C_4$ Alkylsulfonylamino-$C_1$-$C_4$alkyl, N-$C_1$-$C_4$ Alkoxycarbonyl-$C_1$-$C_4$-alkylsulfonylamino-$C_1$-$C_4$-alkyl, $C_3$-$C_{10}$ Alkenyl, $C_3$-$C_{10}$ Alkinyl, $C_3$-$C_8$ Cycloalkyl, Camphoryl oder eine aromatische oder heteroaromatische Gruppe, die unsubstituiert oder substituiert ist durch ein oder zwei aus Halogen, $C_1$-$C_4$ Alkyl, Halogen-$C_1$-$C_4$-alkyl, Di-$C_1$-$C_4$-alkylamino und $C_1$-$C_4$ Alkoxy und $R^{16}$, $R^{17}$, $R^{18}$ und $R^{19}$ jeweils unabhängig für Wasserstoff oder $C_1$-$C_{10}$ Alkyl stehen oder $R^{15}$ und $R^{16}$, $R^{17}$, $R^{18}$ oder $R^{19}$ zusammen mit dem Stickstoffatom, an das sie gebunden sind, eine Azetidinyl-, Pyrrolidinyl-, Piperidinyl- oder Morpholinogruppe bilden.

**14.** Verwendung nach Anspruch 13, worin $(L^a)_n$-$X^2$-$(L^b)_m$ für eine Bindung, CONH oder $CH_2O$ steht.

**15.** Verwendung nach Anspruch 14, worin $R^{14}$ für ein Phenyl steht, das unsubstituiert oder substituiert ist durch ein oder zwei aus Halogen, Nitro, Cyano, $C_1$-$C_{10}$ Alkyl, $C_2$-$C_{10}$ Alkenyl, $C_2$-$C_{10}$ Alkinyl, $C_3$-$C_8$ Cycloalkyl, 4-(1,1-Dioxotetrahydro-1,2-thiazinyl), Halogen-$C_1$-$C_{10}$-alkyl, Cyano-$C_2$-$C_{10}$-alkenyl, Phenyl und $(CH_2)_zX^3R^{15}$ , worin z für eine ganze Zahl von 1 bis 4 steht, $X^3$ für O, S, $NR^{16}$, CO, CH(OH), COO, OCO, $CONR^{17}$, $NR^{18}CO$, $NHSO_2$, $SO_2NH$, $NHSO_2NR^{17}$, $OCONR^{19}$ oder $NR^{19}COO$ steht, $R^{15}$ steht für Wasserstoff, $C_1$-$C_{10}$ Alkyl, Phenyl-$C_1$-$C_4$-alkyl, Halogen-$C_1$-$C_{10}$-alkyl, $C_1$-$C_4$ Alkoxycarbonyl-$C_1$-$C_4$-alkyl, $C_1$-$C_4$ Alkylsulfonylamino-$C_1$-$C_4$-alkyl, N-$C_1$-$C_4$ Alkoxycarbonyl-$C_1$-$C_4$-alkylsulfonylamino-$C_1$-$C_4$-alkyl, $C_3$-$C_{10}$ Alkenyl, $C_3$-$C_{10}$ Alkinyl, $C_3$-$C_8$ Cycloalkyl, Camphoryl oder eine aromatische oder heteroaromatische Gruppe, die unsubstituiert oder substituiert ist durch ein oder zwei aus Halogen, $C_1$-$C_4$ Alkyl, Halogen-$C_1$-$C_4$-alkyl, Di-$C_1$-$C_4$-alkylamino und $C_1$-$C_4$ Alkoxy und $R^{16}$, $R^{17}$, $R^{18}$ und $R^{19}$ jeweils unabhängig für Wasserstoff oder $C_1$-$C_{10}$ Alkyl stehen oder $R^{15}$ und $R^{16}$, $R^{17}$, $R^{18}$ oder $R^{19}$ zusammen mit dem Stickstoffatom, an das sie gebunden sind, eine Azetidinyl-, Pyrrolidinyl-, Piperidinyl- oder Morpholinogruppe bilden.

**16.** Verwendung nach Anspruch 15, worin Phenyl substituiert ist durch ein oder zwei aus Halogen, Nitro, Cyano, $C_1$-$C_{10}$ Alkyl, Halogen-$C_1$-$C_{10}$-alkyl und $(CH_2)_zX^3R^{15}$, worin z für 0, 1 oder 2 steht, $X^3$ für O, $NR^{16}$, CO, COO, $CONR^{17}$, $NR^{18}CO$, $NHSO_2$, $SO_2NH$, $NHSO_2NR^{17}$, $OCONR^{19}$ oder $NR^{19}COO$ steht, $R^{15}$ steht für Wasserstoff, $C_1$-$C_{10}$ Alkyl, Phenyl-$C_1$-$C_4$-alkyl, Halogen-$C_1$-$C_{10}$)-alkyl oder $C_3$-$C_{10}$ Alkenyl und $R^{16}$, $R^{17}$, $R^{18}$ und $R^{19}$ jeweils unabhängig für Wasserstoff oder $C_1$-$C_{10}$ Alkyl stehen.

**17.** Verwendung nach Anspruch 16, worin Phenyl substituiert ist durch ein oder zwei aus Fluor, Chlor, Cyano, $C_1$-$C_4$ Alkyl, Trifluormethyl und $(CH_2)_zX^3R^{15}$, worin z für 0 oder 2 steht, $X^3$ für $NR^{16}$, CO, COO, $CONR^{17}$, $NR^{18}CO$, $NHSO_2$ oder $SO_2NH$ steht, $R^{15}$ für Wasserstoff, $C_1$-$C_4$ Alkyl, Phenyl-$C_1$-$C_4$-alkyl oder Halogen-$C_1$-$C_4$-alkyl steht und $R^{16}$, $R^{17}$, $R^{18}$ und $R^{19}$ jeweils unabhängig für Wasserstoff oder $C_1$-$C_4$ Alkyl stehen.

**18.** Verwendung nach Anspruch 17, worin Phenyl substituiert ist durch eines aus Fluor, Chlor, Cyano, $C_1$-$C_4$ Alkyl und $(CH_2)_zX^3R^{15}$, worin z für 0 oder 2 steht, $X^3$ für $CONR^{17}$, $NR^{18}CO$, $NHSO_2$, und $SO_2NH$ steht, $R^{15}$ für Wasserstoff oder $C_1$-$C_4$ Alkyl steht und $R^{16}$, $R^{17}$, $R^{18}$ und $R^{19}$ jeweils unabhängig für Wasserstoff oder $C_1$-$C_4$ Alkyl stehen.

**19.** Verwendung einer Verbindung der Formel

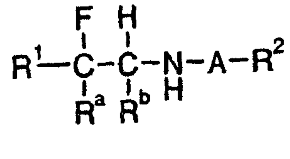

worin

A für $SO_2$, $CO_2$ oder CONH steht,
$R^a$ für $C_1$-$C_6$ Alkyl, $C_2$-$C_6$ Alkenyl, $C_1$-$C_4$ Alkyl-$C_3$-$C_8$-cycloalkyl oder einen $C_1$-$C_4$ Alkylaromaten steht,
$R^b$ für H, $C_1$-$C_6$ Alkyl, $C_2$-$C_6$ Alkenyl, $C_1$-$C_4$ Alkyl-$C_3$-$C_8$-cycloalkyl oder einen $C_1$-$C_4$ Alkylaromaten steht, oder
$R^a$ und $R^b$ zusammen mit den Kohlenstoffatomen, an die sie gebunden sind, einen gesättigten carbocyclischen $C_3$-$C_8$ Ring, einen gesättigten carbocyclischen $C_3$-$C_8$ Ring, der ein Heteroatom enthält, das ausgewählt ist aus der Gruppe, die besteht aus Schwefel oder Sauerstoff oder einen carbocyclischen $C_5$-$C_8$ Ring, der eine Doppelbindung aufweist, bilden,
$R^1$ für eine unsubstituierte oder substituierte aromatische Gruppe, eine unsubstituierte oder substituierte heteroaromatische Gruppe oder eine unsubstituierte oder substituierte $C_5$-$C_8$ Cycloalkylgruppe steht,
$R^2$ für $C_1$-$C_6$ Alkyl, $C_3$-$C_6$ Cycloalkyl, $C_1$-$C_6$ Fluoralkyl, $C_1$-$C_6$ Chloralkyl, $C_2$-$C_6$ Alkenyl, $C_1$-$C_4$ Alkoxy-$C_1$-$C_4$-alkyl, Phenyl, das unsubstituiert oder substituiert ist mit Halogen, $C_1$-$C_4$ Alkyl oder $C_1$-$C_4$ Alkoxy steht oder wenn A für $SO_2$ steht, für eine Gruppe der Formel $R^3R^4N$ steht, worin $R^3$ und $R^4$ jeweils unabhängig für $C_1$-$C_4$ Alkyl stehen oder zusammen mit dem Stickstoffatom, an das sie gebunden sind, eine Azetidinyl-, Pyrrolidinyl-, Piperidinyl-, Morpholino-, Piperazinyl-, Hexahydroazepinyl- oder Octahydroazocinylgruppe bilden,

oder eines pharmazeutisch annehmbaren Salzes hiervon zur Herstellung eines Arzneimittels zur Behandlung von traumatischer Hirnverletzung, traumatischer Spinalstrangverletzung, Hirnschädigung durch cerebrale Hypoperfusion oder anderen neurodegenerativen Zuständen.

**20.** Verwendung nach Anspruch 19 zur Behandlung einer traumatischen Spinalstrangverletzung.

## Revendications

**1.** Utilisation d'un composé répondant à la formule :

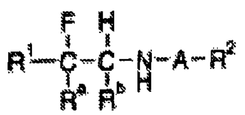

dans laquelle :

A représente $SO_2$, $CO_2$ ou CONH;
$R^a$ représente un groupe alkyle en $C_1$-$C_6$, un groupe alcényle en $C_2$-$C_6$, un groupe cycloalkyle en $C_3$-$C_8$ (alkyle en $C_1$-$C_4$) ou un groupe alkylaromatique en $C_1$-$C_4$ ;
$R^b$ représente H, un groupe alkyle en $C_1$-$C_6$, un groupe alcényle en $C_2$-$C_6$, un groupe cycloalkyle en $C_3$-$C_8$ (alkyle en $C_1$-$C_4$) ou un groupe alkylaromatique en $C_1$-$C_4$ ; ou
$R^a$ et $R^b$ ensemble avec les atomes de carbone auxquels ils sont liés forment un cycle carbocyclique saturé en $C_3$-$C_8$, un cycle carbocyclique saturé en $C_3$-$C_8$ comprenant un hétéroatome choisi dans le groupe constitué d'un atome de soufre ou d'oxygène, ou un cycle carbocyclique en $C_5$-$C_8$ comprenant une double liaison ;
$R^1$ représente un groupe aromatique non substitué ou substitué, un groupe hétéroaromatique non substitué ou substitué ou un groupe cycloalkyle en $C_5$-$C_8$ non substitué ou substitué ;
$R^2$ représente un groupe alkyle en $C_1$-$C_6$, un groupe cycloalkyle en $C_3$-$C_6$, un groupe fluoroalkyle en $C_1$-$C_6$, un groupe chloroalkyle en $C_1$-$C_6$, un groupe alcényle en $C_2$-$C_6$, un groupe alcoxy en $C_1$-$C_4$ (alkyle en $C_1$-$C_4$), un groupe phényle qui est non substitué ou substitué par un atome d'halogène, un groupe alkyle en $C_1$-$C_4$ ou un groupe alcoxy en $C_1$-$C_4$, ou lorsque A représente $SO_2$, un groupe répondant à la formule $R^3R^4N$, dans laquelle $R^3$ et $R^4$ représentent chacun indépendamment un groupe alkyle en $C_1$-$C_4$, ou ensemble avec l'atome d'azote auquel ils sont liés forment un groupe azétidinyle, pyrrolidinyle, pipéridinyle, morpholino, pipérazinyle, hexahydroazépinyle ou octahydroazocinyle ;

ou un sel pharmaceutiquement acceptable de celui-ci, pour la fabrication d'un médicament destiné à traiter une attaque.

**2.** Utilisation selon la revendication 1, dans laquelle A représente $SO_2$.

**3.** Utilisation selon la revendication 1, dans laquelle A représente $CO_2$.

4. Utilisation selon la revendication 1, dans laquelle A représente CONH.

5. Utilisation selon l'une quelconque des revendications 1 à 4, dans laquelle $R^1$ représente un groupe naphtyle ou un groupe phényle, furyle, thiényle ou pyridyle qui est non substitué ou substitué par un ou deux substituants choisis indépendamment parmi un atome d'halogène ; un groupe nitro ; un groupe cyano ; un groupe hydroxyimino ; un groupe alkyle en $C_1$-$C_{10}$ ; un groupe alcényle en $C_2$-$C_{10}$; un groupe alcynyle en $C_2$-$C_{10}$; un groupe cycloalkyle en $C_3$-$C_8$ ; un groupe hydroxy(cycloalkyle en $C_3$-$C_8$) ; un groupe oxo(cycloalkyle en $C_3$-$C_8$) un groupe halogénoalkyle en $C_1$-$C_{10}$; $(CH_2)_yX^1R^9$ dans lequel y vaut 0 ou un entier de 1 à 4, $X^1$ représente O, S, $NR^{10}$, CO, COO, OCO, $CONR^{11}$, $NR^{12}CO$, $NR^{12}COCOO$ ou $OCONR^{13}$, $R^9$ représente un atome d'hydrogène, un groupe alkyle en $C_1$-$C_{10}$, un groupe alcényle en $C_3$-$C_{10}$, un groupe alcynyle en $C_3$-$C_{10}$, un groupe pyrrolidinyle, un groupe tétrahydrofuryle, un groupe morpholino ou un groupe cycloalkyle en $C_3$-$C_8$ et $R^{10}$, $R^{11}$, $R^{12}$ et $R^{13}$ représentent chacun indépendamment un atome d'hydrogène ou un groupe alkyle en $C_1$-$C_{10}$, ou $R^9$ et $R^{10}$, $R^{11}$, $R^{12}$ ou $R^{13}$ ensemble avec l'atome d'azote auquel ils sont liés forment un groupe azétidinyle, pyrrolidinyle, pipéridinyle ou morpholino ; un groupe N-pipérazinyl(alkyle en $C_1$-$C_4$) ; un groupe N-pipérazinyl(alkyle en $C_1$-$C_4$) phényle ; un groupe thiényle ; un groupe furyle ; un groupe oxazolyle ; un groupe isoxazolyle ; un groupe pyrazolyle ; un groupe imidazolyle ; un groupe thiazolyle ; un groupe pyridyle ; un groupe pyridazinyle ; un groupe pyrimidinyle ; un groupe dihydrothiényle ; un groupe dihydrofuryle ; un groupe dihydrothiopyranyle ; un groupe dihydropyranyle ; un groupe dihydrothiazolyle ; un groupe carbonyldihydrothiazolyl(alcoxy en $C_1$-$C_4$) ; un groupe carbonyldiméthyldihydrothiazolyl(alcoxy en $C_1$-$C_4$) ; un groupe tétrahydrothiényle ; un groupe tétrahydrofuryle ; un groupe tétrahydrothiopyranyle ; un groupe tétrahydropyranyle ; un groupe indolyle ; un groupe benzofuryle ; un groupe benzothiényle ; un groupe benzimidazolyle ; et un groupe répondant à la formule $R^{14}$-$(L^a)_n$-$X^2$-$(L^b)_m$, dans laquelle $X^2$ représente une liaison, O, NH, S, SO, $SO_2$, CO, CH(OH), CONH, NHCO, NHCONH, NHCOO, COCONH, $OCH_2CONH$ ou CH=CH, $L^a$ et $L^b$ représentent chacun un groupe alkylène en $C_1$-$C_4$, un de n et m vaut 0 ou 1 et l'autre vaut 0, et $R^{14}$ représente un groupe phényle ou hétéroaromatique qui est non substitué ou substitué par un ou deux atomes d'halogène, groupes nitro, groupes cyano, groupes hydroxyimino, groupes alkyles en $C_1$-$C_{10}$, groupes alcényles en $C_2$-$C_{10}$, groupes alcynyles en $C_2$-$C_{10}$, groupes cycloalkyles en $C_3$-$C_8$, groupes 4-(1,1-dioxotétrahydro-1,2-thiazinyles), groupes halogénoalkyles en $C_1$-$C_{10}$, groupes cyanoalcényles en $C_2$-$C_{10}$, groupes phényles et $(CH_2)_zX^3R^{15}$ dans lequel z vaut 0 ou un nombre entier de 1 à 4, $X^3$ représente O, S, $NR^{16}$, CO, CH(OH), COO, OCO, $CONR^{17}$, $NR^{18}CO$, $NHSO_2$, $SO_2NH$, $NHSO_2NR^{17}$, NHCONH, $OCONR^{19}$ ou $NR^{19}COO$, $R^{15}$ représente un atome d'hydrogène, un groupe alkyle en $C_1$-$C_{10}$, un groupe phénylalkyle en $C_1$-$C_4$, un groupe halogénoalkyle en $C_1$-$C_{10}$, un groupe (alcoxy en $C_1$-$C_4$)carbonyl(alkyle en $C_1$-$C_4$), un groupe (alkyle en $C_1$-$C_4$) sulfonylamino(alkyle en $C_1$-$C_4$), un groupe (N-carbonylalcoxy en $C_1$-$C_4$)(alkyle en $C_1$-$C_4$)sulfonylamino(alkyle en $C_1$-$C_4$), un groupe alcényle en $C_3$-$C_{10}$, un groupe alcynyle en $C_3$-$C_{10}$, un groupe cycloalkyle en $C_3$-$C_8$, un groupe camphoryle ou un groupe aromatique ou hétéroaromatique qui est non substitué ou substitué par un ou deux atomes d'halogène, groupes alkyles en $C_1$-$C_4$, groupes halogénoalkyles en $C_1$-$C_4$, groupes di(alkyle en $C_1$-$C_4$) amino et groupes alcoxy en $C_1$-$C_4$ et $R^{16}$, $R^{17}$, $R^{18}$ et $R^{19}$ représentent chacun indépendamment un atome d'hydrogène ou un groupe alkyle en $C_1$-$C_{10}$, ou $R^{15}$ et $R^{16}$, $R^{17}$, $R^{18}$ ou $R^{19}$ ensemble avec l'atome d'azote auquel ils sont liés forment un groupe azétidinyle, pyrrolidinyle, pipéridinyle ou morpholino.

6. Utilisation selon l'une quelconque des revendications 1 à 5, dans laquelle $R^2$ représente un groupe alkyle en $C_1$-$C_6$, un groupe cycloalkyle en $C_3$-$C_6$, un groupe fluoroalkyle en $C_1$-$C_6$, un groupe chloroalkyle en $C_1$-$C_6$, un groupe alcényle en $C_2$-$C_6$, un groupe alcoxy en $C_1$-$C_4$ (alkyle en $C_1$-$C_4$), un groupe hétéroaromatique ou un groupe phényle qui est non substitué ou substitué par un atome d'halogène, un groupe alkyle en $C_1$-$C_4$ ou un groupe alcoxy en $C_1$-$C_4$.

7. Utilisation selon l'une quelconque des revendications 1 à 6, dans laquelle $R^2$ représente un groupe isopropyle.

8. Utilisation selon l'une quelconque des revendications 1 à 7, dans laquelle $R^a$ représente un groupe alkyle en $C_1$-$C_6$ ou un groupe alcényle en $C_2$-$C_6$.

9. Utilisation selon l'une quelconque des revendications 1 à 8, dans laquelle $R^a$ représente un groupe méthyle et $R^b$ représente un atome d'hydrogène.

10. Utilisation selon l'une quelconque des revendications 1 à 9, dans laquelle $R^1$ représente un groupe aromatique substitué

11. Utilisation selon la revendication 10, dans laquelle le groupe aromatique substitué est un groupe phényle substitué.

**12.** Utilisation selon la revendication 11, dans laquelle le groupe phényle substitué est substitué par un atome d'halogène ; un groupe nitro ; un groupe cyano ; un groupe hydroxyimino ; un groupe alkyle en $C_1$-$C_{10}$ ; un groupe alcényle en $C_2$-$C_{10}$ ; un groupe alcynyle en $C_2$-$C_{10}$ ; un groupe cycloalkyle en $C_3$-$C_8$ ; un groupe hydroxy(cycloalkyle en $C_3$-$C_8$) ; un groupe oxo(cycloalkyle en $C_3$-$C_8$) ; un groupe halogénoalkyle en $C_1$-$C_{10}$ ; $(CH_2)_yX^1R^9$ dans lequel y vaut 0 ou un entier de 1 à 4, $X^1$ représente O, S, $NR^{10}$, CO, COO, OCO, $CONR^{11}$, $NR^{12}CO$, $NR^{12}COCOO$ ou $OCONR^{13}$, $R^9$ représente un atome d'hydrogène, un groupe alkyle en $C_1$-$C_{10}$, un groupe alcényle en $C_3$-$C_{10}$, un groupe alcynyle en $C_3$-$C_{10}$, un groupe pyrrolidinyle, un groupe tétrahydrofuryle, un groupe morpholino ou un groupe cycloalkyle en $C_3$-$C_8$ et $R^{10}$, $R^{11}$, $R^{12}$ et $R^{13}$ représentent chacun indépendamment un atome d'hydrogène ou un groupe alkyle en $C_1$-$C_{10}$, ou $R^9$ et $R^{10}$, $R^{11}$, $R^{12}$ ou $R^{13}$ ensemble avec l'atome d'azote auquel ils sont liés forment un groupe azétidinyle, pyrrolidinyle, pipéridinyle ou morpholino ; un groupe N-pipérazinyl(alkyle en $C_1$-$C_4$) ; un groupe N-pipérazinyl(alkyle en $C_1$-$C_4$)phényle ; un groupe thiényle ; un groupe furyle ; un groupe oxazolyle ; un groupe isoxazolyle ; un groupe pyrazolyle ; un groupe imidazolyle ; un groupe thiazolyle ; un groupe pyridyle ; un groupe pyridazinyle ; un groupe pyrimidinyle ; un groupe dihydrothiényle ; un groupe dihydrofuryle ; un groupe dihydrothiopyranyle ; un groupe dihydropyranyle ; un groupe dihydrothiazolyle ; un groupe carbonyldihydrothiazolyl(alcoxy en $C_1$-$C_4$); un groupe carbonyldiméthyldihydrothiazolyl(alcoxy en $C_1$-$C_4$); un groupe tétrahydrothiényle ; un groupe tétrahydrofuryle ; un groupe tétrahydrothiopyranyle ; un groupe tétrahydropyranyle ; un groupe indolyle ; un groupe benzofuryle ; un groupe benzothiényle ; un groupe benzimidazolyle ; et un groupe répondant à la formule $R^{14}$-$(L^a)_n$-$X^2$-$(L^b)_m$, dans laquelle $X^2$ représente une liaison, O, NH, S, SO, $SO_2$, CO, CH(OH), CONH, NHCO, NHCONH, NHCOO, COCONH, $OCH_2CONH$ ou CH=CH, $L^a$ et $L^b$ représentent chacun un groupe alkylène en $C_1$-$C_4$, un de n et m vaut 0 ou 1 et l'autre vaut 0, et $R^{14}$ représente un groupe phényle ou hétéroaromatique qui est non substitué ou substitué par un ou deux atomes d'halogène, groupes nitro, groupes cyano, groupes alkyles en $C_1$-$C_{10}$, groupes alcényles en $C_2$-$C_{10}$, groupes alcynyles en $C_2$-$C_{10}$, groupes cycloalkyles en $C_3$-$C_8$, groupes 4-(1,1-dioxotétrahydro-1,2-thiazinyles), groupes halogénoalkyles en $C_1$-$C_{10}$, groupes cyanoalcényles en $C_2$-$C_{10}$, groupes phényles et $(CH_2)_zX^3R^{15}$ dans lequel z vaut 0 ou un nombre entier de 1 à 4, $X^3$ représente O, S, $NR^{16}$, CO, CH(OH), COO, OCO, $CONR^{17}$, $NR^{18}CO$, $NHSO_2$, $SO_2NH$, $NHSO_2NR^{17}$, $OCONR^{19}$ ou $NR^{19}COOO$, $R^{15}$ représente un atome d'hydrogène, un groupe alkyle en $C_1$-$C_{10}$, un groupe phénylalkyle en $C_1$-$C_4$, un groupe halogénoalkyle en $C_1$-$C_{10}$, un groupe (alcoxy en $C_1$-$C_4$)carbonyl(alkyle en $C_1$-$C_4$), un groupe (alkyle en $C_1$-$C_4$) sulfonylamino(alkyle en $C_1$-$C_4$), un groupe (N-carbonylalcoxy en $C_1$-$C_4$)(alkyle en $C_1$-$C_4$)sulfonylamino(alkyle en $C_1$-$C_4$), un groupe alcényle en $C_3$-$C_{10}$, un groupe alcynyle en $C_3$-$C_{10}$, un groupe cycloalkyle en $C_3$-$C_8$, un groupe camphoryle ou un groupe aromatique ou hétéroaromatique qui est non substitué ou substitué par un ou deux atomes d'halogène, groupes alkyles en $C_1$-$C_4$, groupes halogénoalkyles en $C_1$-$C_4$, groupes di(alkyle en $C_1$-$C_4$) amino et groupes alcoxy en $C_1$-$C_4$ et $R^{16}$, $R^{17}$, $R^{18}$ et $R^{19}$ représentent chacun indépendamment un atome d'hydrogène ou un groupe alkyle en $C_1$-$C_{10}$, ou $R^{15}$ et $R^{16}$, $R^{17}$, $R^{18}$ ou $R^{19}$ ensemble avec l'atome d'azote auquel ils sont liés forment un groupe azétidinyle, pyrrolidinyle, pipéridinyle ou morpholino.

**13.** Utilisation selon la revendication 12, dans laquelle le groupe phényle substitué est substitué par un groupe répondant à la formule $R^{14}$-$(L^a)_n$-$X^2$-$(L^b)_m$, dans laquelle $X^2$ représente une liaison, O, NH, S, SO, $SO_2$, CO, CH(OH), CONH, NHCO, NHCONH, NHCOO, COCONH, $OCH_2CONH$ ou CH=CH, $L^a$ et $L^b$ représentent chacun un groupe alkylène en $C_1$-$C_4$, un de n et m vaut 0 ou 1 et l'autre vaut 0, et $R^{14}$ représente un groupe phényle ou hétéroaromatique qui est non substitué ou substitué par un ou deux atomes d'halogène, groupes nitro, groupes cyano, groupes alkyles en $C_1$-$C_{10}$, groupes alcényles en $C_2$-$C_{10}$, groupes alcynyles en $C_2$-$C_{10}$, groupes cycloalkyles en $C_3$-$C_8$, groupes 4-(1,1-dioxotétrahydro-1,2-thiazinyles), groupes halogénoalkyles en $C_1$-$C_{10}$, groupes cyanoalcényles en $C_2$-$C_{10}$, groupes phényles et $(CH_2)_zX^3R^{15}$ dans lequel z vaut 0 ou un nombre entier de 1 à 4, $X^3$ représente O, S, $NR^{16}$, CO, CH(OH), COO, OCO, $CONR^{17}$, $NR^{18}CO$, $NHSO_2$, $SO_2NH$, $NHSO_2NR^{17}$, $OCONR^{19}$ ou $NR^{19}COO$, $R^{15}$ représente un atome d'hydrogène, un groupe alkyle en $C_1$-$C_{10}$, un groupe phénylalkyle en $C_1$-$C_4$, un groupe halogénoalkyle en $C_1$-$C_{10}$, un groupe (alcoxy en $C_1$-$C_4$)carbonyl(alkyle en $C_1$-$C_4$), un groupe (alkyle en $C_1$-$C_4$) sulfonylamino(alkyle en $C_1$-$C_4$), un groupe (N-carbonylalcoxy en $C_1$-$C_4$)(alkyle en $C_1$-$C_4$)sulfonylamino(alkyle en $C_1$-$C_4$), un groupe alcényle en $C_3$-$C_{10}$, un groupe alcynyle en $C_3$-$C_{10}$, un groupe cycloalkyle en $C_3$-$C_8$, un groupe camphoryle ou un groupe aromatique ou hétéroaromatique qui est non substitué ou substitué par un ou deux atomes d'halogène, groupes alkyles en $C_1$-$C_4$, groupes halogénoalkyles en $C_1$-$C_4$, groupes di(alkyle en $C_1$-$C_4$) amino et groupes alcoxy en $C_1$-$C_4$ et $R^{16}$, $R^{17}$, $R^{18}$ et $R^{19}$ représentent chacun indépendamment un atome d'hydrogène ou un groupe alkyle en $C_1$-$C_{10}$, ou $R^{15}$ et $R^{16}$, $R^{17}$, $R^{18}$ ou $R^{19}$ ensemble avec l'atome d'azote auquel ils sont liés forment un groupe azétidinyle, pyrrolidinyle, pipéridinyle ou morpholino.

**14.** Utilisation selon la revendication 13, dans laquelle $(L^a)_n$-$X^2$ -$(L^b)_m$ représente une liaison, CONH ou $CH_2O$.

**15.** Utilisation selon la revendication 14, dans laquelle $R^{14}$ représente un groupe phényle qui est non substitué ou substitué par un ou deux atomes d'halogène, groupes nitro, groupes cyano, groupes alkyles en $C_1$-$C_{10}$, groupes

alcényles en $C_2$-$C_{10}$, groupes alcynyles en $C_2$-$C_{10}$, groupes cycloalkyles en $C_3$-$C_8$, groupes 4-(1,1-dioxotétrahydro-1,2-thiazinyles), groupes halogénoalkyles en $C_1$-$C_{10}$, groupes cyanoalcényles en $C_2$-$C_{10}$, groupes phényles et $(CH_2)_zX^3R^{15}$ dans lequel z vaut 0 ou un nombre entier de 1 à 4, $X^3$ représente O, S, $NR^{16}$, CO, CH(OH), COO, OCO, $CONR^{17}$, $NR^{18}CO$, $NHSO_2$, $SO_2NH$, $NHSO_2NR^{17}$, $OCONR^{19}$ ou $NR^{19}COO$, $R^{15}$ représente un atome d'hydrogène, un groupe alkyle en $C_1$-$C_{10}$, un groupe phénylalkyle en $C_1$-$C_4$, un groupe halogénoalkyle en $C_1$-$C_{10}$, un groupe (alcoxy en $C_1$-$C_4$)carbonyl(alkyle en $C_1$-$C_4$), un groupe (alkyle en $C_1$-$C_4$)sulfonylamino(alkyle en $C_1$-$C_4$), un groupe (N-carbonylalcoxy en $C_1$-$C_4$)(alkyle en $C_1$-$C_4$)sulfonylamino(alkyle en $C_1$-$C_4$), un groupe alcényle en $C_3$-$C_{10}$, un groupe alcynyle en $C_3$-$C_{10}$, un groupe cycloalkyle en $C_3$-$C_8$, un groupe camphoryle ou un groupe aromatique ou hétéroaromatique qui est non substitué ou substitué par un ou deux atomes d'halogène, groupes alkyles en $C_1$-$C_4$, groupes halogénoalkyles en $C_1$-$C_4$, groupes di(alkyle en $C_1$-$C_4$)amino et groupes alcoxy en $C_1$-$C_4$ et $R^{16}$, $R^{17}$, $R^{18}$ et $R^{19}$ représentent chacun indépendamment un atome d'hydrogène ou un groupe alkyle en $C_1$-$C_{10}$, ou $R^{15}$ et $R^{16}$, $R^{17}$, $R^{18}$ ou $R^{19}$ ensemble avec l'atome d'azote auquel ils sont liés forment un groupe azétidinyle, pyrrolidinyle, pipéridinyle ou morpholino.

**16.** Utilisation selon la revendication 15, dans laquelle le groupe phényle est substitué par un ou deux atomes d'halogène, groupes nitro, groupes cyano, groupes alkyles en $C_1$-$C_{10}$, groupes halogénoalkyles en $C_1$-$C_{10}$ et $(CH_2)_zX^3R^{15}$ dans lequel z vaut 0, 1 ou 2, $X^3$ représente O, $NR^{16}$, CO, COO, $CONR^{17}$, $NR^{18}CO$, $NHSO_2$, $SO_2NH$, $NHSO_2NR^{17}$, $OCONR^{19}$ ou $NR^{19}COO$, $R^{15}$ représente un atome d'hydrogène, un groupe alkyle en $C_1$-$C_{10}$, un groupe phénylalkyle en $C_1$-$C_4$, un groupe halogénoalkyle en $C_1$-$C_{10}$ ou un groupe alcényle en $C_3$-$C_{10}$, et $R^{16}$, $R^{17}$, $R^{18}$ et $R^{19}$ représentent chacun indépendamment un atome d'hydrogène ou un groupe alkyle en $C_1$-$C_{10}$.

**17.** Utilisation selon la revendication 16, dans laquelle le groupe phényle est substitué par un ou deux atomes de fluor, atomes de chlore, groupes cyano, groupes alkyles en $C_1$-$C_4$, groupes trifluorométhyles et $(CH_2)_zX^3$ $R^{15}$ dans lequel z vaut 0 ou 2, $X^3$ représente $NR^{16}$, CO, COO, $CONR^{17}$, $NR^{18}CO$, $NHSO_2$ et $SO_2NH$, $R^{15}$ représente un atome d'hydrogène, un groupe alkyle en $C_1$-$C_4$, un groupe phénylalkyle en $C_1$-$C_4$ ou un groupe halogénoalkyle en $C_1$-$C_4$, et $R^{16}$, $R^{17}$, $R^{18}$ et $R^{19}$ représentent chacun indépendamment un atome d'hydrogène ou un groupe alkyle en $C_1$-$C_4$.

**18.** Utilisation selon la revendication 17, dans laquelle le groupe phényle est substitué par un ou deux atomes de fluor, atomes de chlore, groupes cyano, groupes alkyles en $C_1$-$C_4$, et $(CH_2)_zX^3R^{15}$ dans lequel z vaut 0 ou 2, $X^3$ représente $CONR^{17}$, $NR^{18}CO$, $NHSO_2$ et $SO_2NH$, $R^{15}$ représente un atome d'hydrogène ou un groupe alkyle en $C_1$-$C_4$, et $R^{16}$, $R^{17}$, $R^{18}$ et $R^{19}$ représentent chacun indépendamment un atome d'hydrogène ou un groupe alkyle en $C_1$-$C_4$.

**19.** Utilisation d'un composé répondant à la formule :

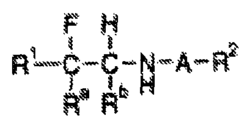

dans laquelle :

A représente $SO_2$, $CO_2$ ou CONH;
$R^a$ représente un groupe alkyle en $C_1$-$C_6$, un groupe alcényle en $C_2$-$C_6$, un groupe cycloalkyle en $C_3$-$C_8$ (alkyle en $C_1$-$C_4$) ou un groupe alkylaromatique en $C_1$-$C_4$ ;
$R^b$ représente H, un groupe alkyle en $C_1$-$C_6$, un groupe alcényle en $C_2$-$C_6$, un groupe cycloalkyle en $C_3$-$C_8$ (alkyle en $C_1$-$C_4$) ou un groupe alkylaromatique en $C_1$-$C_4$; ou
$R^a$ et $R^b$ ensemble avec les atomes de carbone auxquels ils sont liés forment un cycle carbocyclique saturé en $C_3$-$C_8$, un cycle carbocyclique saturé en $C_3$-$C_8$ comprenant un hétéroatome choisi dans le groupe constitué d'un atome de soufre ou d'oxygène, ou un cycle carbocyclique en $C_5$-$C_8$ comprenant une double liaison ;
$R^1$ représente un groupe aromatique non substitué ou substitué, un groupe hétéroaromatique non substitué ou substitué ou un groupe cycloalkyle en $C_5$-$C_8$ non substitué ou substitué ;
$R^2$ représente un groupe alkyle en $C_1$-$C_6$, un groupe cycloalkyle en $C_3$-$C_6$, un groupe fluoroalkyle en $C_1$-$C_6$, un groupe chloroalkyle en $C_1$-$C_6$, un groupe alcényle en $C_2$-$C_6$, un groupe alcoxy en $C_1$-$C_4$ (alkyle en $C_1$-$C_4$), un groupe phényle qui est non substitué ou substitué par un atome d'halogène, un groupe alkyle en $C_1$-$C_4$ ou un groupe alcoxy en $C_1$-$C_4$, ou lorsque A représente $SO_2$, un groupe répondant à la formule $R^3R^4N$, dans

laquelle $R^3$ et $R^4$ représentent chacun indépendamment un groupe alkyle en $C_1$-$C_4$, ou ensemble avec l'atome d'azote auquel ils sont liés forment un groupe azétidinyle, pyrrolidinyle, pipéridinyle, morpholino, pipérazinyle, hexahydroazépinyle ou octahydroazocinyle ;

ou un sel pharmaceutiquement acceptable de celui-ci, pour la fabrication d'un médicament destiné à traiter un traumatisme cérébral, un traumatisme de la moelle épinière, une lésion cérébrale due à une hypoperfusion cérébrale ou d'autres pathologies neurodégénératives.

**20.** Utilisation selon la revendication 19, pour le traitement d'un traumatisme de la moelle épinière.